# EUROPEAN PATENT APPLICATION

(11) **EP 3 970 739 A1**
(43) Date of publication of application: **23.03.2022**
(21) Application number: 20806866.8
(22) Date of filing: 12.05.2020
(51) Int. Cl.: A61K 39/00, A61P 35/00, A61P 37/04, C07K 14/705, C12Q 1/04, G01N 33/49, G01N 33/80

(54) **METHOD FOR DETERMINING ELIGIBILITY OF CANCER PATIENTS FOR PEPTIDE VACCINE THERAPY**

(30) Priority: 13.05.2019 JP 2019090530; 17.07.2019 JP 2019131830
(71) Applicant: Itoh, Kyogo, Miyaki-gun, Saga 841-0205 (JP)
(72) Inventor: NOGUCHI, Masanori, Kurume-shi, Fukuoka 830-0011 (JP); YUTANI, Shigeru, Kurume-shi, Fukuoka 830-0059 (JP); SHICHIJO, Shigeki, Kurume-shi, Fukuoka 830-0003 (JP); YAMADA, Akira, Kurume-shi, Fukuoka 830-0011 (JP); ITO, Kyogo, Miyaki-gun, Saga 841-0205 (JP)
(74) Representative: Vossius & Partner Patentanwälte Rechtsanwälte mbB
(86) International application number: PCT/JP2020/019002
(87) International publication number: WO 2020/230792

(57) **Abstract**

The present disclosure provides a method for determining whether a cancer patient is eligible for peptide vaccine therapy. The method comprises determining whether a cancer patient is eligible for peptide vaccine therapy, based on the neutrophil ratio and/or lymphocyte ratio in blood collected from the cancer patient approximately 7 to 35 days prior to the scheduled date of administration of a peptide vaccine agent.

## Description

### TECHNICAL FIELD

This application claims the benefit of priority of Japanese Patent Application Nos. 2019-090530 and 2019-131830, the entire contents of which are incorporated herein by reference.

The disclosure relates to a method of determining eligibility of a cancer patient for a peptide vaccine therapy.

### BACKGROUND

Personalized precision medicine has recently attracted attention, and development of new technologies for precision medicine is crucial for improving therapeutic effectiveness and suppressing medical cost.

It is known that effects of cancer vaccine therapies in which patients are vaccinated with a cancer antigen peptide vary among patients, and it is desired to select a treatment plan which can be expected to be effective for each patient. The present inventors have developed a tailor-made (made-to-order or personalized) peptide vaccine therapy in which a peptide for each patient is selected based on a secondary immune reaction (Non-Patent Literatures 1 to 3). Results of randomized phase II clinical study of the tailor-made peptide vaccine therapy have been partially reported (Non-Patent Literatures 4 to 9). In the clinical study, longer overall survival (OS) was observed in prostate cancer patients and colon cancer patients having received the tailor-made peptide vaccine therapy (Non-Patent Literatures 4 and 5). On the other hand, in randomized double-blind placebo-controlled phase III clinical study in recurrent glioblastoma patients, no significant difference was found between patients having received the tailor-made peptide vaccine therapy and patients in placebo group (Non-Patent Literature 10).

Accordingly, in the tailor-made peptide vaccine therapy it is desired to further select patients for whom the therapeutic effect is expected. For example, it is known that the effects of the tailor-made peptide vaccine therapy vary depending on gene expression profile of peripheral blood mononuclear cells of prostate cancer patients (Non-Patent Literature 11). Regarding a cancer vaccine therapy for brain tumor using a tailor-made peptide vaccine composition, a method of determining eligibility of patients is known (Patent Literature 1).

### REFERENCES

### PATENT LITERATURE

[Patent Literature 1] WO2018/212237

### NON-PATENT LITERATURE

[Non-Patent Literature 1] Noguchi M, Sasada T, Itoh K, Cancer Immunol Immunother. 2013 May;62(5):919-29
[Non-Patent Literature 2] Sasada T, Yamada A, Noguchi M, Itoh K, Current Medicinal Chemistry. 21: 2332-2345, 2014
[Non-Patent Literature 3] Kibe S, Yutani S, Motoyama S, Nomura T, et al., Cancer Immunology Research 2014;2(12):1154-62
[Non-Patent Literature 4] Noguchi M, Matsumoto K, Uemura H, et al., Clin Cancer Res. 22(1):54-60. 2016
[Non-Patent Literature 5] Yoshimura K, Minami T, Nozawa M, et al., Eur Urol. pii: S0302-2838(16)00008-7. doi: 10.1016/j.eururo.2015.12.050
[Non-Patent Literature 6] Shirahama T, Muroya D, Matsueda M, et al., Cancer Sci. 2017;108(5): 838-45
[Non-Patent Literature 7] Suekane S, Ueda K, Nishihara K, et al., Cancer Sci. 2017 Dec;108(12):2430-2437. doi: 10.1111/cas.13404.
[Non-Patent Literature 8] Takayama K, Sugawara S, Saijo Y, et al., Journal of Immunology Research 2016 Volume 2016, Article ID 1745108, 7 pages
[Non-Patent Literature 9] Noguchi M, Koga N, Moriya F. et al., Cancer Sci 2018; 109; 2660-2669
[Non-Patent Literature 10] Narita Y, Arakawa Y, Yamasaki F et al., Neuro Oncol. 2019 Feb;21(3) ; 348-359
[Non-Patent Literature 11] Komatsu N, et al., Cancer;118(12) :3208-21 ; 2012 Jun 15

### SUMMARY

An object of the disclosure is to determine eligibility of a cancer patient for a peptide vaccine therapy.

The inventors have found that levels of neutrophil percentage and/or levels of lymphocyte percentage of cancer patients correlate with their eligibility for peptide vaccine therapies.

Accordingly, an aspect of the disclosure provides a method comprising determining whether a cancer patient is eligible for a peptide vaccine therapy on the basis of a level of neutrophil percentage and/or a level of lymphocyte percentage in a blood sample obtained from the patient about 7 to 35 days before a scheduled administration date of a peptide vaccine composition.

An aspect of the disclosure provides a kit for determining whether a cancer patient is eligible for a peptide vaccine therapy, comprising a reagent for determining a level of neutrophil percentage and/or a level of lymphocyte percentage.

An aspect of the disclosure provides a peptide vaccine composition for treating a cancer in a patient, comprising at least one peptide antigen, wherein the patient has been determined eligible for a peptide vaccine therapy by the method described above.

An aspect of the disclosure provides a kit for preparing a peptide vaccine composition for treating a cancer in a patient, comprising at least one peptide antigen, wherein the patient has been determined eligible for a peptide vaccine therapy by the method described above.

An aspect of the disclosure provides a kit comprising WHSC2-103, CypB-129, UBE-43, HNRPL-140, SART3-109, Lck-208, PAP-213, Lck-486, and Lck-90.

An aspect of the disclosure provides a kit comprising one or plural polynucleotides encoding WHSC2-103, CypB-129, UBE-43, HNRPL-140, SART3-109, Lck-208, PAP-213, Lck-486, and Lck-90.

According to the disclosure, eligibility of a cancer patient for a peptide vaccine therapy can be determined and/or a cancer of a patient who has been determined eligible for a peptide vaccine therapy can be treated. Alternatively, the disclosure provides a kit for preparing a peptide vaccine composition comprising a new combination of peptide antigens or polynucleotides encoding them.

### BRIEF DESCRIPTION OF DRAWINGS

Fig. 1 shows a flowchart of Consolidated Standards of Reporting Trials (CONSORT) for Test Example 1.
Fig. 2 shows a Kaplan-Meier curve for overall survival time according to treatment in Test Example 1.
Fig. 3 shows the relationship among levels of neutrophil percentage (A) or levels of lymphocyte percentage (B), interaction p-values, and numbers of patients in Test Example 1.
Fig. 4 shows the relationship between levels of neutrophil percentage (A) or levels of lymphocyte percentage (B) and hazard ratios of median overall survival (OS) of the tailor-made peptide vaccine (PPV) arm and the placebo arm in Test Example 1. Each numerical value shown in the graph indicates the number of patients in the PPV arm who meet each cutoff values.
Fig. 5 shows median OS in the patients who met either or both the cutoffs of neutrophils ≥64% and lymphocytes <26% (A) and who met both the cutoffs of neutrophils <64% and lymphocytes ≥26% (B) in the PPV arm versus the placebo arm in Test Example 1, with the hazard ratio and p-value for the PPV arm versus the placebo arm.
Fig. 6 shows changes in IgG level (A) and CTL level (B) from baseline to 6 doses in the PPV arm according to the neutrophil percentage (<64% or ≥64%) and the lymphocyte percentage (≥26% or <26%) in Test Example 1.
Fig. 7 shows median OS of patients who meet the cutoff values of neutrophil percentage ≥64% or <64% with the hazard ratio and p-value in the ready-made peptide vaccine composition (KRM20) arm (A) and the placebo arm (B) of Test Example 3.
Fig. 8 shows median OS of patients who meet the cutoff values of lymphocyte percentage <26% or ≥26% with the hazard ratio and p-value in the ready-made peptide vaccine composition (KRM20) arm (A) and the placebo arm (B) of Test Example 3.
Fig. 9 shows median OS of patients who meet the cutoff values of either or both of neutrophil percentage ≥64% and lymphocyte percentage <26% (Others) or both of neutrophil percentage <64% and lymphocyte percentage ≥26% with the hazard ratio and p-value in the ready-made peptide vaccine composition (KRM20) arm (A) and the placebo arm (B) of Test Example 3.
Fig. 10 shows correlation between hazard ratios (HRs) of overall survival (OS) and levels of neutrophil percentage at enrollment for the clinical trial in the PPV therapy of Test Example 4.
Fig. 11 shows correlation between HRs of OS and levels of lymphocyte percentage at enrollment for the clinical trial in the PPV therapy of Test Example 4.
Fig. 12 shows correlation between HRs of OS and levels of neutrophil-lymphocyte ratio at enrollment for the clinical trial in the PPV therapy of Test Example 4.
Fig. 13 shows comparison of median OS between 1,522 cancer patients who meet either or both of neutrophil ≥64% and lymphocyte <26% and the other 1,066 cancer patients in 2,588 patients having received the PPV therapy of Test Example 4.
Fig. 14 shows comparison between median OS of 1,335 cancer patients who meet either or both of neutrophil ≥64% and lymphocyte <26% and median OS of the other 939 cancer patients in 2,274 patients having received the PPV therapy of Test Example 4 using the set of nine peptide antigens.

### DETAILED DESCRIPTION

Unless otherwise defined, the terms used herein are read as generally understood by a skilled person in the technical fields such as organic chemistry, medical sciences, pharmaceutical sciences, molecular biology, and microbiology. Several terms used herein are defined as described below. The definitions herein take precedence over the general understanding.

When a numerical value is accompanied with the term "about", the value is intended to represent any value in the range of -10% of the value to +10% of the value. For example, "about 20" means "a value from 18 to 22." A range defined with values of the lower and upper limits covers all values from the lower limit to the upper limit, including the values of the both limits. When a range is accompanied with the term "about", the both limits are read as accompanied with the term. For example, "about 20 to 30" is read as "18 to 33."

In the disclosure, the term "peptide vaccine therapy" refers to a method of treating cancer by administering a peptide vaccine composition. The peptide vaccine composition comprises at least one peptide antigen. In the disclosure, the term "peptide antigen" means a peptide for inducing a tumorspecific immune response which is derived from a tumor-associated antigen protein. The peptide antigens are roughly classified into short-chain peptide antigens and long-chain peptide antigens based on chain length. In the disclosure, examples of the peptide antigens include, but not limited to, peptide antigens synthesized chemically and peptide antigens isolated/purified from biological samples. Examples of the methods for chemically synthesizing a peptide include the Fmoc method and the azide method. Examples of the peptide antigens isolated/purified from biological samples include peptide antigens produced by peptide synthesis using genetically engineering technique. In one embodiment, the peptide antigen includes a chemically synthesized short-chain peptide antigen and/or long-chain peptide antigen. In one embodiment, the peptide antigen includes a chemically synthesized short-chain peptide.

In the disclosure, the term "short-chain peptide antigen" refers to an epitope peptide having a chain length sufficient to directly bind to MHC on the surface of an antigen presenting cell. In one embodiment, the short-chain peptide antigen consists of 8 to 17 amino acid residues, wherein a peptide antigen for inducing killer T cells may consist of 8 to 11 amino acid residues and a peptide antigen for inducing helper T cells may consist of 12 to 17 amino acid residues. In one embodiment, the short-chain peptide antigen consists of 8 to 10 or 9 to 10 amino acid residues.

In the disclosure, the term "long-chain peptide antigen" refers to a peptide of relatively long chain length having one or more epitopes. The long-chain peptide antigen usually cannot bind to MHC directly. It is incorporated into an antigen-presenting cell and processed into epitope peptides within the cell by the function of endosomal proteases or peptidases as well as cytoplasmic proteasomes. The long-chain peptide antigen may have a natural amino acid sequence of a tumor-associated antigen protein or an amino acid sequence prepared by artificially linking a plurality of epitope peptides.

In one embodiment, the peptide vaccine composition comprises at least one peptide antigen selected from the group of peptide antigens shown in Table 1 below. The peptide vaccine composition may comprise a peptide antigen not shown in Table 1.

**[Table 1]**

| peptide antigen | HLA type | tumor-associated antigen protein | position of amino acids | amino acid sequence | SEQ ID NO |
|---|---|---|---|---|---|
| CypB-129 | A2, A3sup | Cyclophi-lin B | 129-138 | KLKHYGPGWV | 1 |
| Lck-246 | A2 | p56 lck | 246-254 | KLVERLGAA | 2 |
| Lck-422 | A2, A3sup | p56 lck | 422-430 | DVWSFGILL | 3 |
| MAP-432 | A2, A26 | ppMAPkkk | 432-440 | DLLSHAFFA | 4 |
| WHSC2-103 | A2, A3sup, A26 | WHSC2 | 103-111 | ASLDSDPWV | 5 |
| HNRPL-501 | A2, A26 | HNRPL | 501-510 | NVLHFFNAPL | 6 |
| UBE-43 | A2 | UBE2V | 43-51 | RLQEWCSVI | 7 |
| UBE-85 | A2 | UBE2V | 85-93 | LIADFLSGL | 8 |
| WHSC2-141 | A2 | WHSC2 | 141-149 | ILGELREKV | 9 |
| HNRPL-140 | A2 | HNRPL | 140-148 | ALVEFEDVL | 10 |
| SART3-302 | A2 | SART3 | 302-310 | LLQAEAPRL | 11 |
| SART3-309 | A2 | SART3 | 309-317 | RLAEYQAYI | 12 |
| SART2-93 | A24 | SART2 | 93-101 | DYSARWNEI | 13 |
| SART3-109 | A24, A3sup, A26 | SART3 | 109-118 | VYDYNCHVDL | 14 |
| Lck-208 | A24 | p56 lck | 208-216 | HYTNASDGL | 15 |
| PAP-213 | A24 | PAP | 213-221 | LYCESVHNF | 16 |
| PSA-248 | A24 | PSA | 248-257 | HYRKWIKDTI | 17 |
| EGFR-800 | A24 | EGF-R | 800-809 | DYVREHKDNI | 18 |
| MRP3-503 | A24 | MRP3 | 503-511 | LYAWEPSFL | 19 |
| MRP3-1293 | A24 | MRP3 | 1293-1302 | NYSVRYRPGL | 20 |
| SART2-161 | A24 | SART2 | 161-169 | AYDFLYNYL | 21 |
| Lck-486 | A24 | p56 lck | 486-494 | TFDYLRSVL | 22 |
| Lck-488 | A24 | p56 lck | 488-497 | DYLRSVLEDF | 23 |
| PSMA-624 | A24 | PSMA | 624-632 | TYSVSFDSL | 24 |
| EZH2-735 | A24 | EZH2 | 735-743 | KYVGIEREM | 25 |
| PTHrP-102 | A24 | PTHrP | 102-111 | RYLTQETNKV | 26 |
| SART3-511 | A3sup | SART3 | 511-519 | WLEYYNLER | 27 |
| SART3-734 | A3sup | SART3 | 734-742 | QIRPIFSNR | 28 |
| Lck-90 | A3sup | p56 lck | 90-99 | ILEQSGEWWK | 29 |
| Lck-449 | A3sup | p56 lck | 449-458 | VIQNLERGYR | 30 |
| PAP-248 | A3sup | PAP | 248-257 | GIHKQKEKSR | 31 |

| | | | | | |
|---|---|---|---|---|---|
| *A3sup : A3, A11, A31, or A33 | | | | | |

The peptide vaccine therapy may be a "tailor-made peptide vaccine therapy". In the disclosure, the "tailor-made peptide vaccine therapy" is also referred to as a personalized peptide vaccine therapy or a made-to-order peptide vaccine therapy, and means a method of treating cancer by administering a tailor-made peptide vaccine composition. The tailor-made peptide vaccine composition refers to a peptide vaccine composition comprising a peptide antigen personally selected based on an analysis result of a patient, such as a tumor-associated antigen expressed in the patient or tumor specific immunoreactivity of the patient. The tailor-made peptide vaccine composition comprises 1 to 7, preferably 3 to 6, and more preferably 4 peptide antigens, although the number of the peptides is not limited. In one embodiment, the tailor-made peptide vaccine composition comprises at least one, preferably at least two, at least three, at least four, or at most four peptide antigens.

For example, the tailor-made peptide vaccine composition may include at least one peptide antigen which is determined by selecting a group of peptide antigens corresponding to the HLA type of a patient from groups of peptide antigens classified in accordance with HLA types, and then selecting at least one peptide antigen on the basis of immunoreactivities of the patient for each peptide antigen included in the selected group, for example, in the descending order of the immunoreactivities. In one embodiment, the tailor-made peptide vaccine composition comprises at least one peptide antigen selected on the basis of immunoreactivities of the patient for the peptide antigens corresponding to HLA-A24, - A2, -A3 supertype (A3 sup: -A3, -A11, -A31 or -A33), or -A26.

In the disclosure, the term "HLA" is an abbreviation of Human Leucocyte Antigen and refers to a human major histocompatibility complex (MHC). HLAs are roughly classified into class I antigens and class II antigens. The class I antigens are further classified into class Ia antigens (HLA-A, -B, and -C) and class Ib antigens (HLA-E, -F, and -G). The type of HLA can be determined in accordance with a conventional method such as serological typing, cytological typing, and DNA typing. In the disclosure, the type of "HLA" of a patient is usually determined before preparation of a peptide vaccine composition, but may be determined at any time. In one embodiment, the HLA type may be determined before, during, or after preparation of a peptide vaccine composition, or further determined before, during, or after an administration cycle.

The immunoreactivity of a patient for a peptide antigen can be determined, for example, by an antibody test using a sample obtained from the patient, such as body fluid or blood, e.g., whole blood, plasma, or serum. Examples of the antibody tests include methods using an enzyme-linked immunosorbent assay (ELISA), a flow cytometer or flowmetry, which is also referred to as a "bead-based multiplex assay" or "fluorescent bead array". A patient may be determined to have immunoreactivity for a peptide antigen, for example, when a determined value of immunoreactivity is sufficiently high as compared with a quantitative value determined in a negative control.

In one embodiment, a patient may be determined to have immunoreactivity for a peptide antigen when the determined value of immunoreactivity is higher than the sum of the quantitative value, i.e., the average value, determined with a placebo, which comprises an adjuvant and does not comprise the peptide antigen, and the value five times the standard deviation (S. D.), and is higher than the integer closest to the sum, i.e., the average value + 5 x S. D. In another embodiment, immunoreactivity of a patient may be measured by flow cytometry or flowmetry, wherein the patient may be evaluated not to have immunoreactivity for the peptide antigen or the immunoreactivity is not detected (ND) when the level of the signal, e.g., fluorescence intensity unit (FIU), for the peptide antigen is less than 10 FIU, and evaluated to have the immunoreactivity when the level is 10 FIU or more.

The immunoreactivity of a patient for a peptide antigen can be determined, for example, by using a substrate, e.g., a microtiter plate or a bead, onto which each peptide antigen is immobilized. Such substrate is set so that a predetermined level of a signal, e.g., 1000 FIU, is generated in response to a predetermined positive specimen, e.g., a blood sample of which the reactivity is known. In this embodiment, the immunoreactivity can be determined by allowing the substrate to contact with a blood sample from the patient, washing the substrate, allowing the substrate to contact with an anti-human antibody labeled with a marker substance such as a fluorescence substance, i.e., a labeled secondary antibody, washing the substrate, and then detecting a signal derived from the marker substance on the substrate, e.g., fluorescence. The patient here may be evaluated not to have immunoreactivity for the peptide antigen or the immunoreactivity is not detected (ND) when the level of the signal, e.g., FIU, on the substrate is less than 10 FIU, and evaluated to have the immunoreactivity when the level is 10 FIU or more.

In one embodiment, the immunoreactivity is determined before preparing a peptide vaccine composition used in a first administration protocol. In another embodiment, the immunoreactivity is further determined after the first administration protocol, and based on the result, a peptide antigen comprised in a peptide vaccine composition used in the next administration protocol may be selected.

In one embodiment, the tailor-made peptide vaccine composition comprises at least one peptide antigen selected from the peptide antigens shown in Table 1 above. The tailor-made peptide vaccine composition may comprise a peptide antigen not shown in Table 1.

In one embodiment, the tailor-made peptide vaccine composition comprises at least one peptide antigen selected from SART3-109, Lck-208, PAP-213, PSA-248, EGFR-800, MRP3-1293, Lck-486, Lck-488, PSMA-624, PTHrP-102, CypB-129, Lck-246, WHSC2-103, UBE-43, WHSC2-141, HNRPL-140, SART3-302, SART3-734, Lck-90, and Lck-449.

In one embodiment, the tailor-made peptide vaccine composition comprises at least one peptide antigen selected from SART2-93, SART3-109, PAP-213, PSA-248, EGFR-800, MRP 3-503, MRP3-1293, SART2-161, Lck-486, Lck-488, PSMA-624, and PTHrP-102.

In one embodiment, the tailor-made peptide vaccine composition comprises at least one peptide selected from WHSC2-103, CypB-129, UBE-43, HNRPL-140, SART3-109, Lck-208, PAP-213, Lck-486, and Lck-90.

In one embodiment, the tailor-made peptide vaccine composition may comprise a peptide antigen corresponding to the HLA type of a patient, e.g., HLA-A24, -A2, -A3sup, or - A26. For example, the patient is HLA-A24 positive and the peptide antigen is selected from the group consisting of SART2-93, SART3-109, Lck-208, PAP-213, PSA-248, EGFR-800, MRP3-503, MRP3-1293, SART2-161, Lck-486, Lck-488, PSMA-624, EZH2-735, and PTHrP-102, preferably from the group consisting of SART3-109, Lck-208, PAP-213, and Lck-486; the patient is HLA-A2 positive and the peptide antigen is selected from the group consisting of CypB-129, Lck-246, Lck-422, MAP-432, WHSC2-103, HNRPL-501, UBE-43, UBE-85, WHSC2-141, HNRPL-140, SART3-302, and SART3-309, preferably from the group consisting of WHSC2-103, CypB-129, UBE-43, and HNRPL-140; the patient is HLA-A3, -A11, -A31, or -A33 positive, and the peptide antigen is selected from the group consisting of CypB-129, Lck-422, WHSC2-103, SART3-109, SART3-511, SART3-734, Lck-90, Lck-449, and PAP-248, preferably from the group consisting of WHSC2-103, CypB-129, SART3-109, and Lck-90; or the patient is HLA-A26 positive and the peptide antigen is selected from the group consisting of MAP-432, WHSC2-103, HNRPL-501, and SART3-109, preferably from the group consisting of WHSC2-103 and SART3-109.

The peptide vaccine therapy may be a "ready-made peptide vaccine therapy". In the disclosure, the term "ready-made peptide vaccine therapy" refers to a method of treating cancer by administering a ready-made peptide vaccine composition. The ready-made peptide vaccine composition does not comprise a peptide antigen personally selected based on the immunoreactivity of patients for peptide antigens but comprises a predetermined peptide antigen. In one embodiment, the ready-made peptide vaccine composition comprises at least one of the peptide antigens shown in Table 1. The ready-made peptide vaccine composition may comprise any combination of or all of the peptide antigens shown in Table 1.

In one embodiment, the ready-made peptide vaccine composition may comprise any combination of or all of peptide antigens selected from the group consisting of SART3-109, Lck-208, PAP-213, PSA-248, EGFR-800, MRP3-1293, Lck-486, Lck-488, PSMA-624, PTHrP-102, CypB-129, Lck-246, WHSC2-103, UBE-43, WHSC2-141, HNRPL-140, SART3-302, SART3-734, Lck-90, and Lck-449.

In one embodiment, the ready-made peptide vaccine composition may comprise any combination of or all of peptide antigens selected from SART2-93, SART3-109, PAP-213, PSA-248, EGFR-800, MRP3-503, MRP3-1293, SART2-161, Lck-486, Lck-488, PSMA-624, and PTHrP-102.

In one embodiment, the ready-made peptide vaccine composition may comprise a peptide antigen corresponding to the HLA type of a patient, e.g., HLA-A24, A2, A3 supertype, or A26. The ready-made peptide vaccine composition used for a patient of each HLA type may comprise any combination of or all of peptide antigens corresponding to each HLA types shown in Table 1. For example, a patient is HLA-A24 positive and the peptide antigen is selected from the group consisting of SART2-93, SART3-109, Lck-208, PAP-213, PSA-248, EGFR-800, MRP3-503, MRP3-1293, SART2-161, Lck-486, Lck-488, PSMA-624, EZH2-735, and PTHrP-102; a patient is HLA-A2 positive and the peptide antigen is selected from the group consisting of CypB-129, Lck-246, Lck-422, MAP-432, WHSC2-103, HNRPL-501, UBE-43, UBE-85, WHSC2-141, HNRPL-140, SART3-302, and SART3-309; a patient is HLA-A3, -A11, -A31, or -A33 positive, and the peptide antigen is selected from the group consisting of CypB-129, Lck-422, WHSC2-103, SART3-109, SART3-511, SART3-734, Lck-90, Lck-449, and PAP-248; or a patient is HLA-A26 positive and the peptide antibody is selected from the group consisting of MAP-432, WHSC2-103, HNRPL-501, and SART3-109.

The peptide vaccine composition is prepared in accordance with a conventional method. The peptide vaccine composition may be prepared by mixing a peptide antigen in the form of powder or liquid with a pharmaceutically acceptable carrier, but the preparation method is not limited thereto. In one embodiment, the peptide vaccine composition may further contain an adjuvant for enhancing a specific immune response induced by the peptide antigen.

In the disclosure, the term "adjuvant" refers to a substance or an auxiliary agent that is added to, mixed with, or simultaneously administered with the peptide vaccine composition, thereby enhancing a specific immune response to the peptide antigen comprised in the peptide vaccine composition. In general, adjuvants are roughly classified into two types, i.e., those activating an innate immune receptor and those providing a delivery system. Examples of the former include substances derived from components of microbes such as bacteria, viruses and fungi, and derivatives thereof. Examples of the latter include mineral salts such as aluminum salt, oil-in-water emulsions and liposomes. The peptide vaccine composition may contain either of the former and the latter, or a combined adjuvant obtained by combining them, but the adjuvant is not limited thereto.

The peptide vaccine composition may be administered by a method such as subcutaneous administration, intradermal administration, intramuscular administration, intravenous administration, nasal administration, or oral administration. In one embodiment, the peptide vaccine composition is administered to a patient by subcutaneous injection. A peptide vaccine composition in the dosage form of an injection can be prepared in accordance with a conventional method, for example, by dissolving a peptide antigen in the form of powder or liquid in a pharmaceutically acceptable injection solvent.

An administration schedule and a dose of the peptide vaccine composition can be appropriately set by those skilled in the art depending on factors such as the health condition, the age and the weight of a patient, the route of administration, and the dosage form, so that an effective dose of the composition can be administered to the patient. For example, the peptide vaccine composition can be administered once every week, every 2 weeks, every 3 weeks, every 4 weeks, every 5 weeks, or every 6 weeks. The administration schedule may include, for example, several cycles, e.g., 2, 3, 4, 5, 6, 7, 8 or more cycles, each including several, e.g., 2, 3, 4, 5, 6, 7, 8, 9, 10 or more, times of administration of the peptide vaccine composition. An administration interval and/or the number of times of administration of the peptide vaccine composition in each cycle may be the same or different among the cycles. A washout period of, e.g., 1, 2, 3, 4, 5, 6 or more weeks may be provided between cycles.

As an example of the administration schedule, each cycle includes 6 times of the administration, with the peptide vaccine composition administered at a frequency of once a week in the first cycle, at a frequency of once every two weeks in the second cycle, and at a frequency of once every three weeks or more in the third and following cycles. In one embodiment, each cycle includes 6 times of the administration, with the peptide vaccine composition administered at a frequency of once a week in the first cycle, and at a frequency of once every two weeks in the second and following cycles. In one embodiment, each cycle includes 6 times of the administration, with the peptide vaccine composition administered at a frequency of once a week in the first cycle, at a frequency of once every two weeks in the second cycle, at a frequency of once every four weeks in the third cycle, and at a frequency of once every four to twelve weeks in the fourth and following cycles. In one embodiment, the peptide vaccine composition is administered 4 times at a frequency of once a week in the first cycle, 8 times at a frequency of once every two weeks in the second cycle, 8 times at a frequency of once every four weeks in the third cycle, and at a frequency of once every four to twelve weeks in the fourth and following cycles. In one embodiment, each cycle includes 4 times of the administration, with the peptide vaccine composition administered at a frequency of once a week in the first cycle, at a frequency of once every four to eight weeks in the second cycle, and at a frequency of once every four to twelve weeks in the third and following cycles. In one embodiment, the peptide vaccine composition is administered 6 times at a frequency of once a week in the first cycle, and at a frequency of once every three weeks in the second and following cycles.

In one embodiment, each cycle includes 8 times of the administration, with the peptide vaccine composition administered 4 times at a frequency of once a week and then 4 times at a frequency of once every two weeks in the first cycle, and 4 times at a frequency of once every two weeks and then 4 times at a frequency of once every four weeks in the second cycle. In one embodiment, each cycle includes 4 times of the administration, with the peptide vaccine composition administered at a frequency of once every four weeks in the first and second cycles. In these embodiments, the administration may be continued at a frequency of once every two to twelve weeks after the second cycle.

Each dose of the peptide vaccine composition for an adult may be, for example, about 0.01 to 100 mg, about 0.1 to 50 mg, about 1 to 10 mg, about 2 to 8 mg, or about 3 to 6 mg, e.g., about 3 mg or about 6 mg, of each peptide antigen.

When a plurality of, e.g., two, three, or four peptide antigens are used, peptide vaccine compositions each comprising one or more peptide antigens may be used in combination, or a single composition comprising all the peptide antigens may be used. When a combination of peptide vaccine compositions is used, each composition may be administered to different sites.

In the disclosure, the term "cancer patient" means a human suffering from cancer. In one embodiment, the patient is HLA-A24, -A2, -A3sup, or -A26 positive. Examples of the cancers include, but are not limited to, lung cancer, prostate cancer, colon cancer, pancreatic cancer, breast cancer, urinary tract cancer, biliary tract cancer, ovarian cancer, uterine cancer, hepatocellular cancer (liver cancer), head and neck cancer, sarcoma, esophageal cancer, renal cancer, and gastric cancer.

In one embodiment, the cancer is prostate cancer, lung cancer, breast cancer, colon cancer, gastric cancer, hepatocellular cancer, urinary tract cancer, pancreatic cancer, biliary tract cancer, uterine cancer, or ovarian cancer. In one embodiment, the cancer is prostate cancer, lung cancer, breast cancer, colon cancer, urinary tract cancer, pancreatic cancer, biliary tract cancer, uterine cancer, or ovarian cancer. In one embodiment, the cancer is prostate cancer, particularly castration-resistant prostate cancer, metastatic castration-resistant prostate cancer, or docetaxel-resistant prostate cancer. In one embodiment, the cancer is prostate cancer, lung cancer, breast cancer, colon cancer, gastric cancer, urinary tract cancer, pancreatic cancer, biliary tract cancer, uterine cancer, or ovarian cancer, and determination is made based on the level of neutrophil percentage. In one embodiment, the cancer is prostate cancer, lung cancer, breast cancer, colon cancer, hepatocellular cancer, urinary tract cancer, pancreatic cancer, biliary tract cancer, uterine cancer, or ovarian cancer, and determination is made based on the level of lymphocyte percentage. In one embodiment, the cancer is prostate cancer, lung cancer, breast cancer, colon cancer, hepatocellular cancer, urinary tract cancer, pancreatic cancer, biliary tract cancer, uterine cancer, or ovarian cancer, and determination is made based on the level of neutrophil percentage and the level of lymphocyte percentage.

In one embodiment, the cancer is lung cancer, prostate cancer, colon cancer, pancreatic cancer, breast cancer, urinary tract cancer, biliary tract cancer, ovarian cancer, uterine cancer, head and neck cancer, sarcoma, esophageal cancer, or renal cancer. In one embodiment, the cancer is lung cancer, prostate cancer, colon cancer, pancreatic cancer, breast cancer, urinary tract cancer, biliary tract cancer, ovarian cancer, uterine cancer, head and neck cancer, sarcoma, esophageal cancer, renal cancer, or gastric cancer, and determination is made based on the level of neutrophil percentage. In one embodiment, the cancer is lung cancer, prostate cancer, colon cancer, pancreatic cancer, breast cancer, urinary tract cancer, biliary tract cancer, ovarian cancer, uterine cancer, hepatocellular cancer, head and neck cancer, sarcoma, esophageal cancer, or renal cancer, and determination is made based on the level of lymphocyte percentage. In one embodiment, the cancer is lung cancer, prostate cancer, colon cancer, pancreatic cancer, breast cancer, urinary tract cancer, biliary tract cancer, ovarian cancer, uterine cancer, hepatocellular cancer, head and neck cancer, sarcoma, esophageal cancer, or renal cancer, and determination is made based on the level of neutrophil percentage and the level of lymphocyte percentage.

In the disclosure, a blood sample used for determining the level of neutrophil percentage and/or the level of lymphocyte percentage is obtained from a cancer patient before the administration of the peptide vaccine composition. For example, the blood sample is obtained from a cancer patient about 7 to 35 days, about 10 to 28 days, about 14 to 28 days, or about 14 to 21 days before a scheduled administration date of the peptide vaccine composition. Alternatively, the blood sample is obtained from a cancer patient about 7 to 21 days, about 10 to 18 days, about 11 to 17 days, about 12 to 16 days, about 13 to 15 days, or about 14 days before. The blood sample can be obtained, for example, from the vein by a conventional method.

In the disclosure, the term "level of neutrophil percentage" refers to the number of neutrophils relative to the number of leukocytes, which is the total number of lymphocytes, neutrophils, eosinophils, basophils, and monocytes, expressed in percentage. In the disclosure, the term "level of lymphocyte percentage" refers to the number of lymphocytes relative to the number of leukocytes expressed in percentage. The level of neutrophil percentage and the level of lymphocyte percentage are usually evaluated in blood tests, and can be determined by a conventional method, for example, by using an automatic blood cell analyzer, or by visual inspection, ELISA, flowcytometry, or flowmetry. Leukocytes, neutrophils, and lymphocytes may be counted and the level of neutrophil percentage and the level of lymphocyte percentage may be calculated by any method known to those skilled in the art.

The level of neutrophil percentage and the level of lymphocyte percentage can be determined using neutrophil and lymphocyte markers, respectively. Examples of the neutrophil markers include CD14, CD32, CD64, CD11b, CD16, CD18, CD44, and CD55. Examples of the lymphocyte markers include CD3 (T cell), CD19 (B cell), CD20 (B cell), CD56 (NK cell), and CD7 (T cell, NK cell). Alternatively, the level of neutrophil percentage and the level of lymphocyte percentage may be determined using reagents staining neutrophils and lymphocytes, respectively. Examples of the reagents for staining neutrophils include May-Grunwald stain solution plus Giemsa stain solution (May Giemsa stain). Examples of the reagents for staining lymphocytes include Wright's stain solution plus Giemsa stain solution (Wright's Giemsa stain).

When the level of neutrophil percentage is low and/or the level of lymphocyte percentage is high, the patient may be determined eligible for the peptide vaccine therapy. In one embodiment, in order to determine whether a patient is eligible for the peptide vaccine therapy, the level of neutrophil percentage or the level of lymphocyte percentage of the patient is compared with a previously set cutoff level of neutrophil percentage or lymphocyte percentage. For example, when the level of neutrophil percentage of a patient is lower than the cutoff level of neutrophil percentage and/or the level of lymphocyte percentage of the patient is higher than the cutoff level of lymphocyte percentage, the patient may be determined eligible for the peptide vaccine therapy. For example, when the level of neutrophil percentage of a patient is equal to or lower than the cutoff level of neutrophil percentage and/or the level of lymphocyte percentage of the patient is equal to or higher than the cutoff level of lymphocyte percentage, the patient may be determined eligible for the peptide vaccine therapy.

The cutoff levels may be set by a known method employing any of various statistical analysis methods. In general, the cutoff levels may be set by statistically analyzing the levels of neutrophil percentage or the levels of lymphocyte percentage of patients having shown therapeutic effectiveness (i.e., effective group) and patients not having shown therapeutic effectiveness (i.e., ineffective group) among patients to whom the peptide vaccine compositions have been administered (i.e., drug group). A plurality of cutoff levels may be set for each of the level of neutrophil percentage and the level of lymphocyte percentage. Cutoff levels may be set for each subgroup of patients classified in accordance with characteristics, for example, type of cancer, sex, age, and race. Statistical analysis software such as SAS Ver. 9.4 may be used for setting cutoff levels.

In one embodiment, the cutoff level of neutrophil percentage or lymphocyte percentage is a value with which patients of a drug group can be classified with a statistically significant difference into an effective group and an ineffective group. The statistically significant difference may be analyzed by a known test method, such as log-rank test.

The cutoff level may be set on the basis of sensitivity and/or specificity. Preferably, the cutoff level indicates both high sensitivity and high specificity. Here, the term "sensitivity" means a true positive rate, and the term "specificity" means a true negative rate. For example, a level of neutrophil percentage or a level of lymphocyte percentage at which an effective group shows a high positive rate and an ineffective group shows a high negative rate can be set as the cutoff level.

For example, the cutoff value may be set by receiver operating characteristic analysis (ROC analysis), which is conventionally used for evaluating effectiveness of diagnosing methods. The ROC analysis includes creating an ROC curve by plotting sensitivities and false positive rates (1 - specificity) at each cutoff level on the ordinate and abscissa, respectively. When a diagnosing method is not effective at all, the ROC curve is a diagonal straight line. As the effectiveness gets higher, the ROC curve becomes round and gets closer to the upper left corner. Accordingly, a cutoff level which corresponds to a point closest to the upper left corner on the ROC curve provides good sensitivity and specificity. The cutoff level also may be set based on Youden index. Specifically, the values of sensitivity and specificity are obtained based on the levels of neutrophil percentage or the levels of lymphocyte percentage in an effective group and an ineffective group and then an ROC curve is created with the obtained values by commercially available analysis software. The level that provides sensitivity and specificity as close as possible to 100% may be employed as the cutoff level.

Alternatively, for example, a level of neutrophil percentage or a level of lymphocyte percentage at which the highest diagnostic efficiency is achieved may be set as the cutoff level. The diagnostic efficiency herein is a ratio of cases in which effectively treated patients and ineffectively treated patients are correctly diagnosed as "effective" and "ineffective", respectively, to the total number of cases.

The cutoff level of neutrophil percentage is in a range of, for example, about 55% to 75%, about 60% to 70%, or about 63% to 65%, for example, about 64%. The cutoff level of lymphocyte percentage is in a range of, for example, about 15% to 35%, about 20% to 30%, or about 25% to 27%, for example, about 26%. The cutoff levels are not limited to these values, and may be appropriately set in accordance with factors such as purpose of diagnosis, a characteristic of a patient, type of disease, and type of peptide vaccine composition.

Expression of the therapeutic effectiveness is not limited to "eligible" and "not eligible". For example, "eligible" may be expressed as "high possibility of therapeutic success", "responsive to treatment", or "therapeutic success is expected", and "not eligible" may be expressed as "low possibility of therapeutic success", "not responsive to treatment", or "therapeutic success is not expected". For example, the therapeutic success may be extension of overall survival or progression-free survival, e.g., relative to median overall survival or progression-free survival of a placebo group or an untreated group.

An aspect of the disclosure provides a kit for determining whether a cancer patient is eligible for a peptide vaccine therapy, comprising a reagent for determining a level of neutrophil percentage and/or a level of lymphocyte percentage. In one embodiment, the kit is used in an automatic blood cell analyzer, a visual test, ELISA, flowcytometry, or flowmetry. The reagent includes, but is not limited to, a reagent capable of specifically binding to a neutrophil marker and/or a lymphocyte marker, such as an antibody. The kit can be appropriately produced by a conventional method.

An aspect of the disclosure provides a method of determining whether a cancer patient is eligible for a peptide vaccine therapy, comprising
(1) obtaining a blood sample from the patient 7 to 35 days before a scheduled administration date of a peptide vaccine composition,
(2) determining a level of neutrophil percentage and/or a level of lymphocyte percentage in the blood sample, and
(3) determining whether the patient is eligible for the peptide vaccine therapy on the basis of the determined level of neutrophil percentage and/or the determined level of lymphocyte percentage.

An aspect of the disclosure provides a reagent for determining a level of neutrophil percentage and/or a level of lymphocyte percentage for use in a method of determining whether a cancer patient is eligible for a peptide vaccine therapy.

An aspect of the disclosure provides use of a reagent for determining a level of neutrophil percentage and/or a level of lymphocyte percentage in a method of determining whether a cancer patient is eligible for a peptide vaccine therapy.

An aspect of the disclosure provides use of a reagent for determining a level of neutrophil percentage and/or a level of lymphocyte percentage for producing a diagnostic drug for determining whether a cancer patient is eligible for a peptide vaccine therapy.

When a patient is determined eligible for a peptide vaccine therapy by the method of the disclosure, the patient is recommended to receive the peptide vaccine therapy and the cancer may be treated by administering a peptide vaccine composition. Accordingly, an aspect of the disclosure provides a method of treating cancer, comprising administering an effective dose of a peptide vaccine composition comprising at least one peptide antigen to a patient who has been determined eligible for the peptide vaccine therapy by the method described above. The features described above with respect to the determination method, such as the peptide antigen, the peptide vaccine composition, the determination process, the cancer, the patient, the administration method, and the cutoff level are also applied to the method of treating cancer. The term "treatment" herein means reducing or removing a cause of a cancer, delaying or stopping progression, recurrence or metastasis of a cancer, and/or reducing, alleviating, improving, or removing symptoms of a cancer in a cancer patient.

An aspect of the disclosure provides a method of treating a patient suffering from cancer, comprising (1) determining whether the patient is eligible for a peptide vaccine therapy by the method described above; and (2) administering an effective dose of a peptide vaccine composition comprising at least one peptide antigen to the patient on the basis of the result of step (1).

An aspect of the disclosure provides a method of treating cancer, comprising
(1) obtaining a blood sample from a patient 7 to 35 days before a scheduled administration date of a peptide vaccine composition,
(2) determining a level of neutrophil percentage and/or a level of lymphocyte percentage in the blood sample,
(3) determining whether the patient is eligible for a peptide vaccine therapy on the basis of the determined level of neutrophil percentage and/or the determined level of lymphocyte percentage, and
(4) administering an effective dose of the peptide vaccine composition comprising at least one peptide antigen to the patient who has been determined eligible for the peptide vaccine therapy in step (3).

An aspect of the disclosure provides a peptide vaccine composition for treating cancer of a patient who has been determined eligible for a peptide vaccine therapy by the method described above, comprising at least one peptide antigen. The peptide vaccine composition may be the tailor-made peptide vaccine composition or the ready-made peptide vaccine composition described above.

An aspect of the disclosure provides a method of treating cancer comprising administering a peptide antigen to a patient who has been determined eligible for a peptide vaccine therapy by the method of determination disclosed herein.

An aspect of the disclosure provides a peptide antigen for use in treating cancer of a patient who has been determined eligible for a peptide vaccine therapy by the method described above.

An aspect of the disclosure provides use of a peptide antigen in treatment of cancer of a patient who has been determined eligible for a peptide vaccine therapy by the method described above.

An aspect of the disclosure provides use of a peptide antigen for producing a peptide vaccine composition for treating cancer of a patient who has been determined eligible for a peptide vaccine therapy by the method described above.

An aspect of the disclosure provides a treatment kit for preparing a tailor-made peptide vaccine composition. The treatment kit comprises at least one peptide antigen selected from the 31 peptide antigens shown in Table 1, and a tailor-made peptide vaccine composition can be prepared by selecting, for example, at least one, preferably at least two, at least three, at least four, or at most four peptide antigens from them. The tailor-made peptide vaccine composition prepared using the treatment kit is administered to a patient who has been determined eligible by the method described above.

The treatment kit comprises at least one peptide antigen in the form of powder or liquid. The treatment kit may further comprise, for example, a pharmaceutically acceptable carrier and an adjuvant enhancing a specific immune response induced by the peptide antigen. The treatment kit can be appropriately produced by a conventional method. The features of the peptide vaccine composition and the adjuvant described in the context of the method of determination are also applied to the treatment kit.

As proved in Test Example 4 described below, the effect of a tailor-made peptide vaccine composition comprising a peptide antigen selected from the following nine peptide antigens: WHSC2-103, CypB-129, UBE-43, HNRPL-140, SART3-109, Lck-208, PAP-213, Lck-486, and Lck-90, is not inferior to the effect of a tailor-made peptide vaccine composition comprising a peptide antigen selected from the 31 peptide antigens shown in Table 1. A smaller number of warehouse peptide antigens are preferable, for example, in view of cost and handleability. Accordingly, the disclosure provides a kit comprising these nine peptide antigens. This kit can be used for preparing a tailor-made peptide vaccine composition for treating cancer. The features of the tailor-made peptide vaccine composition and the kit described herein in the context of the method of determination are also applied to this kit.

An aspect of the disclosure provides a kit comprising one or plural polynucleotides encoding WHSC2-103, CypB-129, UBE-43, HNRPL-140, SART3-109, Lck-208, PAP-213, Lck-48, and Lck-90. This kit can be used for preparing a tailor-made peptide vaccine composition for treating cancer. The polynucleotide may be in the form of any nucleic acid such as a DNA or an RNA. Such a polynucleotide can be easily produced on the basis of the amino acid sequences of the peptides and the sequences of the DNAs encoding them by a conventional method such as DNA synthesis or PCR amplification. The kit comprises one or plural polynucleotides, and each polynucleotide may encode one or plural peptide antigens.

A recombinant expression vector for expressing a peptide antigen can be produced by incorporating such a polynucleotide into an expression vector. Accordingly, an aspect of the disclosure provides a kit comprising one or plural expression vectors encoding WHSC2-103, CypB-129, UBE-43, HNRPL-140, SART3-109, Lck-208, PAP-213, Lck-486, and Lck-90. This kit can be used for preparing a tailor-made peptide vaccine composition for treating cancer. The kit comprises one or plural vectors, and each vector may encode one or plural peptide antigens.

Any expression vector which is appropriately selected in view of, e.g., host or purpose may be used herein. Examples of the expression vectors include plasmids, phage vectors, and virus vectors. For example, when the host is *E.coli,* the vector may be a plasmid vector such as pUC118, pUC119, pBR322, or pCR3, or a phage vector such as λZAPII or λgt11. When the host is yeast, the vector may be, e.g., pYES2 or pYEUra3. When the host is an insect cell, the vector can be, e.g., pAcSGHisNT-A. When the host is an animal cell, the vector may be a plasmid vector such as pKCR, pCDM8, pGL2, pcDNA31, pRc/RSV, or pRc/CMV, or a virus vector such as a retrovirus vector, an adenovirus vector, or an adenoassociated virus vector.

The vector may appropriately have an element, such as an inducible promoter, a gene encoding a signal sequence, a gene of selection marker, and a terminator.

The vector may have a sequence to generate a fusion protein with a tag such as thioredoxin, His tag, or GST (glutathione S transferase) for facilitating isolation and purification. For this purpose, an expression vector having an appropriate promoter capable of functioning in a host cell, such as lac, tac, trc, trp, CMV, or SV40 initial promoter, may be used, including a GST fusion protein vector, e.g., pGEX4T, a vector having a tag sequence such as Myc or His, e.g., pcDNA3.1/Myc-His, or a vector for expressing a fusion protein with thioredoxin and His tag, e.g., pET32a.

A transformed cell including the expression vector can be produced by transforming a host cell with the expression vector produced as described above. Examples of the hosts used herein include *E.coli,* yeast, insect cells, and animal cells. Examples of *E.coli* include HB101, C600, JM109, DH5α, and AD494 (DE3) of *E-coli* K-12 strain. Examples of the yeast include *Saccharomyces cerevisiae.* Examples of the animal cells include L929 cell, BALB/c3T3 cell, C127 cell, CHO cell, COS cell, Vero cell, and HeLa cell. Examples of the insect cells include sf9.

An expression vector may be introduced into a host cell by a conventional method suitable for the host cell, such as a calcium phosphate method, a DEAE-dextran method, an electroporation method, or a method using a gene transfer lipid (Lipofectamine, Lipofectin; Gibco-BRL). Then the transformed cells having the introduced expression vector may be selected by culturing the host cells in a conventional medium containing a selection marker.

For example, the disclosure provides the following embodiments.
[A-1] A method comprising determining whether a cancer patient is eligible for a peptide vaccine therapy on the basis of a level of neutrophil percentage and/or a level of lymphocyte percentage in a blood sample obtained from the patient about 7 to 35 days before a scheduled administration date of a peptide vaccine composition.
[A-2] The method according to item A-1, comprising determining whether the patient is eligible for the peptide vaccine therapy by comparing the level of neutrophil percentage and/or the level of lymphocyte percentage determined for the patient with a cutoff level of neutrophil percentage and/or a cutoff level of lymphocyte percentage.
[A-3] The method according to item A-2, comprising determining the patient is eligible for the peptide vaccine therapy when the level of neutrophil percentage determined for the patient is less than the cutoff level of neutrophil percentage.
[A-4] The method according to item A-3, wherein the cutoff level of neutrophil percentage is in the range of about 55% to 75%.
[A-5] The method according to item A-3 or A-4, wherein the cutoff level of neutrophil percentage is in the range of about 60% to 70%.
[A-6] The method according to any one of items A-3 to A-5, wherein the cutoff level of neutrophil percentage is in the range of about 63% to 65%.
[A-7] The method according to any one of items A-3 to A-6, wherein the cutoff level of neutrophil percentage is about 64%.
[A-8] The method according to item A-2, comprising determining the patient is eligible for the peptide vaccine therapy when the level of lymphocyte percentage determined for the patient is not less than the cutoff level of lymphocyte percentage.
[A-9] The method according to item A-8, wherein the cutoff level of lymphocyte percentage is in the range of about 15% to 35%.
[A-10] The method according to item A-8 or A-9, wherein the cutoff level of lymphocyte percentage is in the range of about 20% to 30%.
[A-11] The method according to any one of items A-8 to A-10, wherein the cutoff level of lymphocyte percentage is in the range of about 25% to 27%.
[A-12] The method according to any one of items A-8 to A-11, wherein the cutoff level of lymphocyte percentage is about 26%.
[A-13] The method according to any one of items A-2 to A-12, comprising determining the patient is eligible for the peptide vaccine therapy when the level of neutrophil percentage determined for the patient is less than the cutoff level of neutrophil percentage and the level of lymphocyte percentage determined for the patient is not less than the cutoff level of lymphocyte percentage.
[A-14] The method according to item A-13, comprising determining the patient is eligible for the peptide vaccine therapy when the level of neutrophil percentage determined for the patient is less than about 64% and the level of lymphocyte percentage determined for the patient is not less than about 26%.
[A-15] The method according to any one of items A-1 to A-14, wherein the blood sample is obtained about 7 to 21 days before the scheduled administration date of the peptide vaccine composition.
[A-16] The method according to any one of items A-1 to A-15, wherein the blood sample is obtained about 11 to 17 days before the scheduled administration date of the peptide vaccine composition.
[A-17] The method according to any one of items A-1 to A-16, wherein the blood sample is obtained about 13 to 15 days before the scheduled administration date of the peptide vaccine composition.
[A-18] The method according to any one of items A-1 to A-14, wherein the blood sample is obtained about 14 to 28 days before the scheduled administration date of the peptide vaccine composition.
[A-19] The method according to any one of items A-1 to A-18, wherein the blood sample is obtained about 14 days before the scheduled administration date of the peptide vaccine composition.
[A-20] The method according to any one of items 1 to A-19, wherein the patient is positive for HLA-A24, HLA-A2, HLA-A3, HLA-A11, HLA-A31, HLA-A33, or HLA-A26.
[A-21] The method according to any one of items A-1 to A-20, wherein the patient is positive for HLA-A24.
[A-22] The method according to any one of items A-1 to A-21, wherein the peptide vaccine therapy is a tailor-made peptide vaccine therapy.
[A-23] The method according to item A-22, wherein the tailor-made peptide vaccine therapy uses a peptide vaccine composition comprising at least one peptide antigen selected from the group consisting of CypB-129, Lck-246, Lck-422, MAP-432, WHSC2-103, HNRPL-501, UBE-43, UBE-85, WHSC2-141, HNRPL-140, SART3-302, SART3-309, SART2-93, SART3-109, Lck-208, PAP-213, PSA-248, EGFR-800, MRP3-503, MRP3-1293, SART2-161, Lck-486, Lck-488, PSMA-624, EZH2-735, PTHrP-102, SART3-511, SART3-734, Lck-90, Lck-449, and PAP-248, wherein the at least one peptide antigen is selected on the basis of immunoreactivity of the patient for each peptide antigen.
[A-24] The method according to item A-22 or A-23, wherein the tailor-made peptide vaccine therapy uses a peptide vaccine composition comprising at least one peptide antigen selected from the group consisting of SART3-109, Lck-208, PAP-213, PSA-248, EGFR-800, MRP3-1293, Lck-486, Lck-488, PSMA-624, PTHrP-102, CypB-129, Lck-246, WHSC2-103, UBE-43, WHSC2-141, HNRPL-140, SART3-302, SART3-734, Lck-90, and Lck-449, wherein the at least one peptide antigen is selected on the basis of immunoreactivity of the patient for each peptide antigen.
[A-25] The method according to any one of items A-22 to A-24, wherein the tailor-made peptide vaccine therapy uses a peptide vaccine composition comprising at least one peptide antigen selected from the group consisting of SART2-93, SART3-109, PAP-213, PSA-248, EGFR-800, MRP3-503, MRP3-1293, SART2-161, Lck-486, Lck-488, PSMA-624, and PTHrP-102, wherein the at least one peptide antigen is selected on the basis of immunoreactivity of the patient for each peptide antigen.
[A-26] The method according to item A-22 or A-23, wherein the patient is positive for HLA-A24 and the tailor-made peptide vaccine therapy uses a peptide vaccine composition comprising at least one peptide antigen selected from the group consisting of SART2-93, SART3-109, Lck-208, PAP-213, PSA-248, EGFR-800, MRP3-503, MRP3-1293, SART2-161, Lck-486, Lck-488, PSMA-624, EZH2-735, and PTHrP-102, wherein the at least one peptide antigen is selected on the basis of immunoreactivity of the patient for each peptide antigen.
[A-27] The method according to item A-22 or A-23, wherein the patient is positive for HLA-A2 and the tailor-made peptide vaccine therapy uses a peptide vaccine composition comprising at least one peptide antigen selected from the group consisting of CypB-129, Lck-246, Lck-422, MAP-432, WHSC2-103, HNRPL-501, UBE-43, UBE-85, WHSC2-141, HNRPL-140, SART3-302, and SART3-309, wherein the at least one peptide antigen is selected on the basis of immunoreactivity of the patient for each peptide antigen.
[A-28] The method according to item A-22 or A-23, wherein the patient is positive for HLA-A3, HLA-A11, HLA-A31, or HLA-A33 and the tailor-made peptide vaccine therapy uses a peptide vaccine composition comprising at least one peptide antigen selected from the group consisting of CypB-129, Lck-422, WHSC2-103, SART3-109, SART3-511, SART3-734, Lck-90, Lck-449, and PAP-248, wherein the at least one peptide antigen is selected on the basis of immunoreactivity of the patient for each peptide antigen.
[A-29] The method according to item A-22 or A-23, wherein the patient is positive for HLA-A26 and the tailor-made peptide vaccine therapy uses a peptide vaccine composition comprising at least one peptide antigen selected from the group consisting of MAP-432, WHSC2-103, HNRPL-501, and SART3-109, wherein the at least one peptide antigen is selected on the basis of immunoreactivity of the patient for each peptide antigen.
[A-30] The method according to any one of items A-22 to A-29, wherein the peptide vaccine composition comprises at least two peptide antigens.
[A-31] The method according to any one of items A-22 to A-30, wherein the peptide vaccine composition comprises at most four peptide antigens.
[A-32] The method according to any one of items A-23 to A-31, wherein the peptide antigen is selected in the descending order of the immunoreactivities of the patient.
[A-33] The method according to any one of items A-1 to A-21, wherein the peptide vaccine therapy is a ready-made peptide vaccine therapy.
[A-34] The method according to item A-33, wherein the ready-made peptide vaccine therapy uses a peptide vaccine composition comprising at least one peptide antigen selected from the group consisting of CypB-129, Lck-246, Lck-422, MAP-432, WHSC2-103, HNRPL-501, UBE-43, UBE-85, WHSC2-141, HNRPL-140, SART3-302, SART3-309, SART2-93, SART3-109, Lck-208, PAP-213, PSA-248, EGFR-800, MRP3-503, MRP3-1293, SART2-161, Lck-486, Lck-488, PSMA-624, EZH2-735, PTHrP-102, SART3-511, SART3-734, Lck-90, Lck-449, and PAP-248.
[A-35] The method according to item A-33 or A-34, wherein the ready-made peptide vaccine therapy uses a peptide vaccine composition comprising at least one peptide antigen selected from the group consisting of SART3-109, Lck-208, PAP-213, PSA-248, EGFR-800, MRP3-1293, Lck-486, Lck-488, PSMA-624, PTHrP-102, CypB-129, Lck-246, WHSC2-103, UBE-43, WHSC2-141, HNRPL-140, SART3-302, SART3-734, Lck-90, and Lck-449.
[A-36] The method according to any one of items A-33 to A-35, wherein the ready-made peptide vaccine therapy uses a peptide vaccine composition comprising at least one peptide antigen selected from the group consisting of SART2-93, SART3-109, PAP-213, PSA-248, EGFR-800, MRP3-503, MRP3-1293, SART2-161, Lck-486, Lck-488, PSMA-624, and PTHrP-102.
[A-37] The method according to item A-33 or A-34, wherein the patient is positive for HLA-A24 and the ready-made peptide vaccine therapy uses a peptide vaccine composition comprising at least one peptide antigen selected from the group consisting of SART2-93, SART3-109, Lck-208, PAP-213, PSA-248, EGFR-800, MRP3-503, MRP3-1293, SART2-161, Lck-486, Lck-488, PSMA-624, EZH2-735, and PTHrP-102.
[A-38] The method according to item A-33 or A-34, wherein the patient is positive for HLA-A2 and the ready-made peptide vaccine therapy uses a peptide vaccine composition comprising at least one peptide antigen selected from the group consisting of CypB-129, Lck-246, Lck-422, MAP-432, WHSC2-103, HNRPL-501, UBE-43, UBE-85, WHSC2-141, HNRPL-140, SART3-302, and SART3-309.
[A-39] The method according to item A-33 or A-34, wherein the patient is positive for HLA-A3, HLA-A11, HLA-A31, or HLA-A33 and the ready-made peptide vaccine therapy uses a peptide vaccine composition comprising at least one peptide antigen selected from the group consisting of CypB-129, Lck-422, WHSC2-103, SART3-109, SART3-511, SART3-734, Lck-90, Lck-449, and PAP-248.
[A-40] The method according to item A-33 or A-34, wherein the patient is positive for HLA-A26 and the ready-made peptide vaccine therapy uses a peptide vaccine composition comprising at least one peptide antigen selected from the group consisting of MAP-432, WHSC2-103, HNRPL-501, and SART3-109.
[A-41] The method according to any one of items A-34 to A-40, wherein the ready-made peptide vaccine therapy uses a peptide vaccine composition comprising all peptide antigens recited in the item.
[A-42] The method according to any one of items A-1 to A-41, wherein the cancer is prostate cancer, lung cancer, breast cancer, colon cancer, gastric cancer, hepatocellular cancer, urinary tract cancer, pancreatic cancer, biliary tract cancer, uterine cancer, or ovarian cancer.
[A-43] The method according to any one of items A-1 to A-42, wherein the cancer is prostate cancer, lung cancer, breast cancer, colon cancer, urinary tract cancer, pancreatic cancer, biliary tract cancer, uterine cancer, or ovarian cancer.
[A-44] The method according to any one of items A-1 to A-43, wherein the cancer is prostate cancer.
[A-45] The method according to any one of items A-1 to A-44, wherein the cancer is castration-resistant prostate cancer.
[A-46] The method according to any one of items A-1 to A-45, wherein the cancer is metastatic castration-resistant prostate cancer.
[A-47] The method according to any one of items A-1 to A-46, wherein the cancer is docetaxel-resistant prostate cancer.
[A-48] The method according to any one of items A-1 to A-42, wherein the cancer is prostate cancer, lung cancer, breast cancer, colon cancer, gastric cancer, urinary tract cancer, pancreatic cancer, biliary tract cancer, uterine cancer, or ovarian cancer, and whether the patient is eligible for the peptide vaccine therapy is determined on the basis of the level of neutrophil percentage.
[A-49] The method according to any one of items A-1 to A-42, wherein the cancer is prostate cancer, lung cancer, breast cancer, colon cancer, hepatocellular cancer, urinary tract cancer, pancreatic cancer, biliary tract cancer, uterine cancer, or ovarian cancer, and whether the patient is eligible for the peptide vaccine therapy is determined on the basis of the level of lymphocyte percentage.
[A-50] The method according to any one of items A-1 to A-42, wherein the cancer is prostate cancer, lung cancer, breast cancer, colon cancer, hepatocellular cancer, urinary tract cancer, pancreatic cancer, biliary tract cancer, uterine cancer, or ovarian cancer, and whether the patient is eligible for the peptide vaccine therapy is determined on the basis of the level of neutrophil percentage and the level of lymphocyte percentage.
[A-51] The method according to any one of items A-1 to A-50, comprising determining the level of neutrophil percentage and/or the level of lymphocyte percentage in the blood sample obtained from the cancer patient.
[A-52] A kit for determining whether a cancer patient is eligible for a peptide vaccine therapy, comprising a reagent for determining a level of neutrophil percentage and/or a level of lymphocyte percentage.
[A-53] The kit according to item A-52, for use in the method according to any one of items A-1 to A-51.
[A-54] The kit according to item A-52 or A-53, wherein the reagent is an antibody capable of specifically binding to a neutrophil marker and/or a lymphocyte marker.
[A-55] A peptide vaccine composition for treating a cancer in a patient, comprising at least one peptide antigen, wherein the patient has been determined eligible for a peptide vaccine therapy by the method according to any one of items A-1 to A-51.
[A-56] The peptide vaccine composition according to item A-55, which is the peptide vaccine composition recited in any one of items A-23 to A-32 and A-34 to A-41.
[A-57] A kit for preparing a peptide vaccine composition for treating a cancer in a patient, comprising at least one peptide antigen, wherein the patient has been determined eligible for a peptide vaccine therapy by the method according to any one of items A-1 to A-51.
[A-58] The kit according to item A-57, comprising at least one peptide antigen selected from the group consisting of CypB-129, Lck-246, Lck-422, MAP-432, WHSC2-103, HNRPL-501, UBE-43, UBE-85, WHSC2-141, HNRPL-140, SART3-302, SART3-309, SART2-93, SART3-109, Lck-208, PAP-213, PSA-248, EGFR-800, MRP3-503, MRP3-1293, SART2-161, Lck-486, Lck-488, PSMA-624, EZH2-735, PTHrP-102, SART3-511, SART3-734, Lck-90, Lck-449, and PAP-248.
[A-59] The kit according to item A-57 or A-58, comprising at least one peptide antigen selected from the group consisting of SART3-109, Lck-208, PAP-213, PSA-248, EGFR-800, MRP3-1293, Lck-486, Lck-488, PSMA-624, PTHrP-102, CypB-129, Lck-246, WHSC2-103, UBE-43, WHSC2-141, HNRPL-140, SART3-302, SART3-734, Lck-90, and Lck-449.
[A-60] The kit according to any one of items A-57 to A-59, comprising at least one peptide antigen selected from the group consisting of SART2-93, SART3-109, PAP-213, PSA-248, EGFR-800, MRP3-503, MRP3-1293, SART2-161, Lck-486, Lck-488, PSMA-624, and PTHrP-102.
[A-61] The kit according to any one of items A-57 to A-60, comprising at least two peptide antigens.
[A-62] The peptide vaccine composition or kit according to any one of items A-55 to A-61, wherein the peptide vaccine composition is for being administered to a patient who is positive for HLA-A24, HLA-A2, HLA-A3, HLA-A11, HLA-A31, HLA-A33, or HLA-A26.
[A-63] The peptide vaccine composition or kit according to any one of items A-55 to A-62, wherein the peptide vaccine composition is for being administered to a patient who is positive for HLA-A24.
[A-64] The peptide vaccine composition or kit according to any one of items A-55 to A-63, wherein the cancer is a cancer recited in any one of items A-42 to A-47.
[A-65] The peptide vaccine composition or kit according to any one of items A-55 to A-64, wherein the cancer is prostate cancer, lung cancer, breast cancer, colon cancer, gastric cancer, urinary tract cancer, pancreatic cancer, biliary tract cancer, uterine cancer, or ovarian cancer, and the peptide vaccine composition is for being administered to the patient who has been determined eligible for the peptide vaccine therapy on the basis of the level of neutrophil percentage.
[A-66] The peptide vaccine composition or kit according to any one of items A-55 to A-64, wherein the cancer is prostate cancer, lung cancer, breast cancer, colon cancer, hepatocellular cancer, urinary tract cancer, pancreatic cancer, biliary tract cancer, uterine cancer, or ovarian cancer, and the peptide vaccine composition is for being administered to the patient who has been determined eligible for the peptide vaccine therapy on the basis of the level of lymphocyte percentage.
[A-67] The peptide vaccine composition or kit according to any one of items A-55 to A-64, wherein the cancer is prostate cancer, lung cancer, breast cancer, colon cancer, hepatocellular cancer, urinary tract cancer, pancreatic cancer, biliary tract cancer, uterine cancer, or ovarian cancer, and the peptide vaccine composition is for being administered to the patient who has been determined eligible for the peptide vaccine therapy on the basis of the level of neutrophil percentage and the level of lymphocyte percentage.
[B-1] A method comprising determining whether a cancer patient is eligible for a tailor-made peptide vaccine therapy on the basis of a level of neutrophil percentage and/or a level of lymphocyte percentage in a blood sample obtained from the patient about 7 to 35 days before a scheduled administration date of a tailor-made peptide vaccine composition.
[B-2] The method according to item B-1, comprising determining the level of neutrophil percentage and/or the level of lymphocyte percentage in the blood sample obtained from the cancer patient.
[B-3] The method according to item B-1 or B-2, comprising determining whether the patient is eligible for the tailor-made peptide vaccine therapy by comparing the level of neutrophil percentage of the patient and/or the level of lymphocyte percentage of the patient with a cutoff level of neutrophil percentage and/or a cutoff level of lymphocyte percentage.
[B-4] The method according to item B-3, comprising determining the patient is eligible for the tailor-made peptide vaccine therapy when the level of neutrophil percentage of the patient is less than the cutoff level of neutrophil percentage.
[B-5] The method according to item B-4, wherein the cutoff level of neutrophil percentage is in the range of about 55% to 75%.
[B-6] The method according to item B-4 or B-5, wherein the cutoff level of neutrophil percentage is in the range of about 60% to 70%.
[B-7] The method according to any one of items B-4 to B-6, wherein the cutoff level of neutrophil percentage is in the range of about 63% to 65%.
[B-8] The method according to any one of items B-4 to B-7, wherein the cutoff level of neutrophil percentage is about 64%.
[B-9] The method according to item B-3, comprising determining the patient is eligible for the tailor-made peptide vaccine therapy when the level of lymphocyte percentage of the patient is not less than the cutoff level of lymphocyte percentage.
[B-10] The method according to item B-9, wherein the cutoff level of lymphocyte percentage is in the range of about 15% to 35%.
[B-11] The method according to item B-9 or B-10, wherein the cutoff level of lymphocyte percentage is in the range of about 20% to 30%.
[B-12] The method according to any one of items B-9 to B-11, wherein the cutoff level of lymphocyte percentage is in the range of about 25% to 27%.
[B-13] The method according to any one of items B-9 to B-12, wherein the cutoff level of lymphocyte percentage is about 26%.
[B-14] The method according to item B-3, comprising determining the patient is eligible for the tailor-made peptide vaccine therapy when the level of neutrophil percentage of the patient is less than the cutoff level of neutrophil percentage and the level of lymphocyte percentage of the patient is not less than the cutoff level of lymphocyte percentage.
[B-15] The method according to item B-14, wherein the cutoff level of neutrophil percentage is in the range of about 55% to 75% and the cutoff level of lymphocyte percentage is in the range of about 15% to 35%.
[B-16] The method according to item B-14 or B-15, wherein the cutoff level of neutrophil percentage is in the range of about 60% to 70% and the cutoff level of lymphocyte percentage is in the range of about 20% to 30%.
[B-17] The method according to any one of items B-14 to B-16, wherein the cutoff level of neutrophil percentage is in the range of about 63% to 65% and the cutoff level of lymphocyte percentage is in the range of about 25% to 27%.
[B-18] The method according to any one of items B-14 to B-17, wherein the cutoff level of neutrophil percentage is about 64% and the cutoff level of lymphocyte percentage is about 26%.
[B-19] The method according to any one of items B-1 to B-18, wherein the blood sample is obtained about 7 to 21 days before the scheduled administration date of the tailor-made peptide vaccine composition.
[B-20] The method according to any one of items B-1 to B-19, wherein the blood sample is obtained about 11 to 17 days before the scheduled administration date of the tailor-made peptide vaccine composition.
[B-21] The method according to any one of items B-1 to B-20, wherein the blood sample is obtained about 13 to 15 days before the scheduled administration date of the tailor-made peptide vaccine composition.
[B-22] The method according to any one of items B-1 to B-21, wherein the blood sample is obtained about 14 days before the scheduled administration date of the tailor-made peptide vaccine composition.
[B-23] The method according to any one of items B-1 to 22, wherein the patient is positive for HLA-A24, HLA-A2, HLA-A3, HLA-A11, HLA-A31, HLA-A33, or HLA-A26.
[B-24] The method according to any one of items B-1 to B-23, wherein the tailor-made peptide vaccine therapy uses a tailor-made peptide vaccine composition comprising at least one peptide antigen selected from the group consisting of CypB-129, Lck-246, Lck-422, MAP-432, WHSC2-103, HNRPL-501, UBE-43, UBE-85, WHSC2-141, HNRPL-140, SART3-302, SART3-309, SART2-93, SART3-109, Lck-208, PAP-213, PSA-248, EGFR-800, MRP3-503, MRP3-1293, SART2-161, Lck-486, Lck-488, PSMA-624, EZH2-735, PTHrP-102, SART3-511, SART3-734, Lck-90, Lck-449, and PAP-248, wherein the at least one peptide antigen is selected on the basis of immunoreactivity of the patient for each peptide antigen.
[B-25] The method according to any one of items B-1 to B-24, wherein the tailor-made peptide vaccine therapy uses a tailor-made peptide vaccine composition comprising at least one peptide antigen selected from the group consisting of WHSC2-103, CypB-129, UBE-43, HNRPL-140, SART3-109, Lck-208, PAP-213, Lck-486, and Lck-90, wherein the at least one peptide antigen is selected on the basis of immunoreactivity of the patient for each peptide antigen.
[B-26] The method according to any one of items B-1 to B-23, wherein the patient is positive for HLA-A24 and the tailor-made peptide vaccine therapy uses a tailor-made peptide vaccine composition comprising at least one peptide antigen selected from the group consisting of SART2-93, SART3-109, Lck-208, PAP-213, PSA-248, EGFR-800, MRP3-503, MRP3-1293, SART2-161, Lck-486, Lck-488, PSMA-624, EZH2-735, and PTHrP-102, wherein the at least one peptide antigen is selected on the basis of immunoreactivity of the patient for each peptide antigen.
[B-27] The method according to any one of items B-1 to B-23, wherein the patient is positive for HLA-A24 and the tailor-made peptide vaccine therapy uses a tailor-made peptide vaccine composition comprising at least one peptide antigen selected from the group consisting of SART3-109, Lck-208, PAP-213, and Lck-486, wherein the at least one peptide antigen is selected on the basis of immunoreactivity of the patient for each peptide antigen.
[B-28] The method according to any one of items B-1 to B-23, wherein the patient is positive for HLA-A2 and the tailor-made peptide vaccine therapy uses a tailor-made peptide vaccine composition comprising at least one peptide antigen selected from the group consisting of CypB-129, Lck-246, Lck-422, MAP-432, WHSC2-103, HNRPL-501, UBE-43, UBE-85, WHSC2-141, HNRPL-140, SART3-302, and SART3-309, wherein the at least one peptide antigen is selected on the basis of immunoreactivity of the patient for each peptide antigen.
[B-29] The method according to any one of items B-1 to B-23, wherein the patient is positive for HLA-A2 and the tailor-made peptide vaccine therapy uses a tailor-made peptide vaccine composition comprising at least one peptide antigen selected from the group consisting of WHSC2-103, CypB-129, UBE-43, and HNRPL-140, wherein the at least one peptide antigen is selected on the basis of immunoreactivity of the patient for each peptide antigen.
[B-30] The method according to any one of items B-1 to B-23, wherein the patient is positive for HLA-A3, HLA-A11, HLA-A31, or HLA-A33 and the tailor-made peptide vaccine therapy uses a tailor-made peptide vaccine composition comprising at least one peptide antigen selected from the group consisting of CypB-129, Lck-422, WHSC2-103, SART3-109, SART3-511, SART3-734, Lck-90, Lck-449, and PAP-248, wherein the at least one peptide antigen is selected on the basis of immunoreactivity of the patient for each peptide antigen.
[B-31] The method according to any one of items B-1 to B-23, wherein the patient is positive for HLA-A3, HLA-A11, HLA-A31, or HLA-A33 and the tailor-made peptide vaccine therapy uses a tailor-made peptide vaccine composition comprising at least one peptide antigen selected from the group consisting of WHSC2-103, CypB-129, SART3-109, and Lck-90, wherein the at least one peptide antigen is selected on the basis of immunoreactivity of the patient for each peptide antigen.
[B-32] The method according to any one of items B-1 to B-23, wherein the patient is positive for HLA-A26 and the tailor-made peptide vaccine therapy uses a tailor-made peptide vaccine composition comprising at least one peptide antigen selected from the group consisting of MAP-432, WHSC2-103, HNRPL-501, and SART3-109, wherein the at least one peptide antigen is selected on the basis of immunoreactivity of the patient for each peptide antigen.
[B-33] The method according to any one of items B-1 to B-23, wherein the patient is positive for HLA-A26 and the tailor-made peptide vaccine therapy uses a tailor-made peptide vaccine composition comprising at least one peptide antigen selected from the group consisting of WHSC2-103 and SART3-109, wherein the at least one peptide antigen is selected on the basis of immunoreactivity of the patient for each peptide antigen.
[B-34] The method according to any one of items B-1 to B-33, wherein the tailor-made peptide vaccine composition comprises at least two peptide antigens.
[B-35] The method according to any one of items B-1 to B-34, wherein the tailor-made peptide vaccine composition comprises at most four peptide antigens.
[B-36] The method according to any one of items B-1 to B-35, wherein the peptide antigen is selected in the descending order of the immunoreactivities of the patient.
[B-37] The method according to any one of items B-1 to B-36, wherein the cancer is lung cancer, prostate cancer, colon cancer, pancreatic cancer, breast cancer, urinary tract cancer, biliary tract cancer, ovarian cancer, uterine cancer, hepatocellular cancer, head and neck cancer, sarcoma, esophageal cancer, renal cancer, or gastric cancer.
[B-38] The method according to any one of items B-1 to B-37, wherein the cancer is lung cancer, prostate cancer, colon cancer, pancreatic cancer, breast cancer, urinary tract cancer, biliary tract cancer, ovarian cancer, uterine cancer, hepatocellular cancer, head and neck cancer, sarcoma, esophageal cancer, or renal cancer.
[B-39] The method according to any one of items B-1 to B-38, wherein the cancer is lung cancer, prostate cancer, colon cancer, pancreatic cancer, breast cancer, urinary tract cancer, biliary tract cancer, ovarian cancer, uterine cancer, head and neck cancer, sarcoma, esophageal cancer, or renal cancer.
[B-40] The method according to any one of items B-1 to B-37, wherein the cancer is lung cancer, prostate cancer, colon cancer, pancreatic cancer, breast cancer, urinary tract cancer, biliary tract cancer, ovarian cancer, uterine cancer, head and neck cancer, sarcoma, esophageal cancer, renal cancer, or gastric cancer, and whether the patient is eligible for the peptide vaccine therapy is determined on the basis of the level of neutrophil percentage.
[B-41] The method according to any one of items B-1 to B-37, wherein the cancer is lung cancer, prostate cancer, colon cancer, pancreatic cancer, breast cancer, urinary tract cancer, biliary tract cancer, ovarian cancer, uterine cancer, hepatocellular cancer, head and neck cancer, sarcoma, esophageal cancer, or renal cancer, and whether the patient is eligible for the peptide vaccine therapy is determined on the basis of the level of lymphocyte percentage.
[B-42] The method according to any one of items B-1 to B-37, wherein the cancer is lung cancer, prostate cancer, colon cancer, pancreatic cancer, breast cancer, urinary tract cancer, biliary tract cancer, ovarian cancer, uterine cancer, hepatocellular cancer, head and neck cancer, sarcoma, esophageal cancer, or renal cancer, and whether the patient is eligible for the peptide vaccine therapy is determined on the basis of the level of neutrophil percentage and the level of lymphocyte percentage.
[B-43] A kit for determining whether a cancer patient is eligible for a tailor-made peptide vaccine therapy, comprising a reagent for determining a level of neutrophil percentage and/or a level of lymphocyte percentage.
[B-44] The kit according to item B-43, for use in the method according to any one of items B-1 to B-42.
[B-45] The kit according to item B-43 or B-44, wherein the reagent is an antibody capable of specifically binding to a neutrophil marker and/or a lymphocyte marker.
[B-46] A tailor-made peptide vaccine composition for treating a cancer in a patient, comprising at least one peptide antigen, wherein the patient has been determined eligible for a tailor-made peptide vaccine therapy by the method according to any one of items B-1 to B-42.
[B-47] A tailor-made peptide vaccine composition for treating a cancer in a patient, comprising at least one peptide antigen selected from the group consisting of WHSC2-103, CypB-129, UBE-43, HNRPL-140, SART3-109, Lck-208, PAP-213, Lck-486, and Lck-90, wherein the patient has been determined eligible for a tailor-made peptide vaccine therapy by a method comprising determining whether the cancer patient is eligible for the tailor-made peptide vaccine therapy on the basis of a level of neutrophil percentage and/or a level of lymphocyte percentage in a blood sample obtained from the patient about 7 to 35 days before a scheduled administration date of the tailor-made peptide vaccine composition.
[B-48] The tailor-made peptide vaccine composition according to item B-46 or B-47, which is the tailor-made peptide vaccine composition recited in any one of items B-24 to B-36.
[B-49] A kit for preparing a tailor-made peptide vaccine composition for treating a cancer in a patient, comprising at least one peptide antigen, wherein the patient has been determined eligible for a tailor-made peptide vaccine therapy by the method according to any one of items B-1 to B-42.
[B-50] The kit according to item B-49, comprising at least one peptide antigen selected from the group consisting of CypB-129, Lck-246, Lck-422, MAP-432, WHSC2-103, HNRPL-501, UBE-43, UBE-85, WHSC2-141, HNRPL-140, SART3-302, SART3-309, SART2-93, SART3-109, Lck-208, PAP-213, PSA-248, EGFR-800, MRP3-503, MRP3-1293, SART2-161, Lck-486, Lck-488, PSMA-624, EZH2-735, PTHrP-102, SART3-511, SART3-734, Lck-90, Lck-449, and PAP-248.
[B-51] The kit according to item B-49 or B-50, comprising at least one peptide antigen selected from the group consisting of WHSC2-103, CypB-129, UBE-43, HNRPL-140, SART3-109, Lck-208, PAP-213, Lck-486, and Lck-90.
[B-52] A kit for preparing a tailor-made peptide vaccine composition, the kit comprising at least one peptide antigen selected from the group consisting of WHSC2-103, CypB-129, UBE-43, HNRPL-140, SART3-109, Lck-208, PAP-213, Lck-486, and Lck-90, wherein the tailor-made peptide vaccine composition is for treating a cancer in a patient who has been determined eligible for a tailor-made peptide vaccine therapy by a method comprising determining whether the cancer patient is eligible for the tailor-made peptide vaccine therapy on the basis of a level of neutrophil percentage and/or a level of lymphocyte percentage in a blood sample obtained from the patient about 7 to 35 days before a scheduled administration date of the tailor-made peptide vaccine composition.
[B-53] The kit according to any one of items B-49 to B-52, comprising at least two peptide antigens.
[B-54] The kit according to any one of items B-49 to B-53, comprising WHSC2-103, CypB-129, UBE-43, HNRPL-140, SART3-109, Lck-208, PAP-213, Lck-486, and Lck-90.
[B-55] The kit according to any one of items B-49 to B-54, for preparing the tailor-made peptide vaccine composition according to any one of items B-24 to B-36.
[B-56] The tailor-made peptide vaccine composition or kit according to any one of items B-46 to B-55, wherein the tailor-made peptide vaccine composition is for being administered to a patient who is positive for HLA-A24, HLA-A2, HLA-A3, HLA-A11, HLA-A31, HLA-A33, or HLA-A26.
[B-57] The tailor-made peptide vaccine composition or kit according to any one of items B-46 to B-56, wherein the cancer is a cancer recited in any one of items B-37 to B-39.
[B-58] The tailor-made peptide vaccine composition or kit according to any one of items B-46 to B-57, wherein the cancer is lung cancer, prostate cancer, colon cancer, pancreatic cancer, breast cancer, urinary tract cancer, biliary tract cancer, ovarian cancer, uterine cancer, head and neck cancer, sarcoma, esophageal cancer, renal cancer, or gastric cancer, and the tailor-made peptide vaccine composition is for being administered to the patient who has been determined eligible for the tailor-made peptide vaccine therapy on the basis of the level of neutrophil percentage.
[B-59] The tailor-made peptide vaccine composition or kit according to any one of items B-46 to B-57, wherein the cancer is lung cancer, prostate cancer, colon cancer, pancreatic cancer, breast cancer, urinary tract cancer, biliary tract cancer, ovarian cancer, uterine cancer, hepatocellular cancer, head and neck cancer, sarcoma, esophageal cancer, or renal cancer, and the tailor-made peptide vaccine composition is for being administered to the patient who has been determined eligible for the tailor-made peptide vaccine therapy on the basis of the level of lymphocyte percentage.
[B-60] The tailor-made peptide vaccine composition or kit according to any one of items B-46 to B-57, wherein the cancer is lung cancer, prostate cancer, colon cancer, pancreatic cancer, breast cancer, urinary tract cancer, biliary tract cancer, ovarian cancer, uterine cancer, hepatocellular cancer, head and neck cancer, sarcoma, esophageal cancer, or renal cancer, and the peptide vaccine composition is for being administered to the patient who has been determined eligible for the peptide vaccine therapy on the basis of the level of neutrophil percentage and the level of lymphocyte percentage.
[B-61] A kit for preparing a tailor-made peptide vaccine composition for treating a cancer, comprising WHSC2-103, CypB-129, UBE-43, HNRPL-140, SART3-109, Lck-208, PAP-213, Lck-486, and Lck-90.
[B-62] A kit for preparing a tailor-made peptide vaccine composition for treating a cancer, the kit comprising one or plural polynucleotides encoding WHSC2-103, CypB-129, UBE-43, HNRPL-140, SART3-109, Lck-208, PAP-213, Lck-486, and Lck-90.
[B-63] A kit for preparing a tailor-made peptide vaccine composition for treating a cancer, the kit comprising one or plural expression vectors encoding WHSC2-103, CypB-129, UBE-43, HNRPL-140, SART3-109, Lck-208, PAP-213, Lck-486, and Lck-90.
[B-64] A kit for preparing a tailor-made peptide vaccine composition for treating a cancer, the kit comprising one or plural transformed cells, wherein the one or plural transformed cells comprise one or plural polynucleotides or one or plural expression vectors encoding WHSC2-103, CypB-129, UBE-43, HNRPL-140, SART3-109, Lck-208,PAP-213, Lck-486, and Lck-90.
[B-65] The kit according to any one of items B-61 to B-64, wherein the cancer is lung cancer, prostate cancer, colon cancer, pancreatic cancer, breast cancer, urinary tract cancer, biliary tract cancer, ovarian cancer, uterine cancer, hepatocellular cancer, head and neck cancer, sarcoma, esophageal cancer, renal cancer, or gastric cancer.
[B-66] The kit according to any one of items B-61 to B-64, wherein the cancer is lung cancer, prostate cancer, colon cancer, pancreatic cancer, breast cancer, urinary tract cancer, biliary tract cancer, ovarian cancer, uterine cancer, hepatocellular cancer, head and neck cancer, sarcoma, esophageal cancer, or renal cancer.
[B-67] The kit according to any one of items B-61 to B-64, wherein the cancer is lung cancer, prostate cancer, colon cancer, pancreatic cancer, breast cancer, urinary tract cancer, biliary tract cancer, ovarian cancer, uterine cancer, head and neck cancer, sarcoma, esophageal cancer, renal cancer, or gastric cancer.
[B-68] A kit comprising WHSC2-103, CypB-129, UBE-43, HNRPL-140, SART3-109, Lck-208, PAP-213, Lck-486, and Lck-90.
[B-69] A kit comprising one or plural polynucleotides encoding WHSC2-103, CypB-129, UBE-43, HNRPL-140, SART3-109, Lck-208, PAP-213, Lck-486, and Lck-90.
[B-70] A kit comprising one or plural expression vectors encoding WHSC2-103, CypB-129, UBE-43, HNRPL-140, SART3-109, Lck-208, PAP-213, Lck-486, and Lck-90.
[B-71] A kit comprising one or plural transformed cells, wherein the one or plural transformed cells comprise one or plural polynucleotides or one or plural expression vectors encoding WHSC2-103, CypB-129, UBE-43, HNRPL-140, SART3-109, Lck-208, PAP-213, Lck-486, and Lck-90.

The entire contents of the documents cited herein are incorporated herein by reference.

The embodiments described above are non-limiting and may be modified without deviating from the scope of the invention as defined by the appended claims. The following example is non-limiting and provided only for describing the invention.

### EXAMPLES

### [Test Example 1]

### Trial oversight

This trial was a randomized, double-blind, placebocontrolled phase III study conducted by the Fujifilm Corporation (Tokyo) with support from the Japan Agency for Medical Research and Development (Project no. 18im0110802h0008). The protocol was approved by the institutional review board or ethical committee at all of the participating institutions, and was registered in the UMIN Clinical Trials Registry (UMIN0000113088). All patients provided their written informed consent before enrollment and after receiving a full explanation of the protocol. The trial was performed in accordance with the principles of the Declaration of Helsinki.

### Patients

HLA-A24-positive patients with castration-resistant prostate cancer (CRPC) that was progressing within 12 months after docetaxel chemotherapy were enrolled from 68 medical centers in Japan. Eligible patients were age ≥20 years with histologically confirmed adenocarcinoma of the prostate. The other inclusion criteria were as follows: positive immunoglobulin G (IgG) responses to at least 2 of 12 warehouse peptides listed in Table 2 at the screening test; an Eastern Cooperative Oncology Group (ECOG) performance status (PS) of 0 or 1; life expectancy of not less than 12 weeks; serum testosterone level not more than 50 ng/dl; and adequate bone marrow function, hepatic function, and renal function. The treatment of patients without a previous bilateral orchiectomy continued with luteinizing hormone-releasing agonists. The exclusion criteria included acute infection; history of severe allergic reactions; pulmonary, cardiac, or other systemic diseases; and other inappropriate conditions for enrollment as judged by clinicians. Prior enzalutamide or abiraterone was permitted.

**[Table 2]**

| Peptide name | HLA type | Original protein | Position of peptide | Amino acid sequence |
|---|---|---|---|---|
| SART2-93 | A24 | SART2 | 93-101 | DYSARWNEI |
| SART3-109 | A24, A3 family^{a}, A26 | SART3 | 109-118 | VYDYNCHVDL |
| PAP-213 | A24 | PAP | 213-221 | LYCESVHNF |
| PSA-248 | A24 | PSA | 248-257 | HYRKWIKDTI |
| EGFR-800 | A24 | EGF-R | 800-809 | DYVREHKDNI |
| MRP3-503 | A24 | MRP3 | 503-511 | LYAWEPSFL |
| MRP3-1293 | A24 | MRP3 | 1293-1302 | NYSVRYRPGL |
| SART2-161 | A24 | SART2 | 161-169 | AYDFLYNYL |
| Lck-486 | A24 | p56 Ick | 486-494 | TFDYLRSVL |
| Lck-488 | A24 | p56 Ick | 488-497 | DYLRSVLEDF |
| PSMA-624 | A24 | PSMA | 624-632 | TYSVSFDSL |
| PTHrP-102 | A24 | PTHrP | 102-111 | RYLTQETNKV |

| | | | | |
|---|---|---|---|---|
| ^{a}A3 family; HLA-A3, -A11, -A31 or -A33. Abbreviations: EGFR, epidermal growth factor-receptor; HLA, human leukocyte antigen; Lck, p56Ick; MRP, multidrug resistanceassociated protein; PAP, prostatic acid phosphatase PSA, prostate-specific antigen; PSMA, prostate-specific membrane antigen; PTHrP, parathyroid hormone-related peptide; SART, squamous cell carcinoma antigens. Note: The safety and immunological effects of these 12 peptides had been confirmed in previous clinical trials and all peptides were prepared under conditions of GMP using a Multiple Peptide System (San Diego, CA). | | | | |

### Treatments

We randomly assigned the patients in a 2:1 ratio to receive a tailor-made peptide vaccine (PPV) or a placebo, using the minimization technique with the following stratification factors: age (younger than 75 years old, or 75 years old or older, PS (0 or 1), and use of enzalutamide or abiraterone (with or without) at each participating institution. This study was double-blinded, and all physicians, patients, and investigators giving the interventions, assessing outcomes, and analyzing data were masked to the patients' treatment assignment. Up to 4 of 12 warehouse peptides selected based on the individual patient's pre-existing peptide-specific IgG level or the corresponding placebo were emulsified with Montanide ISA 51 incomplete Freund's adjuvant (Seppic, Paris, France). Each selected study drug in 1.5 ml of emulsion (3 mg/peptide or saline solution) was subcutaneously injected once per week for 6 weeks, followed by once every 2 weeks for a maximum of 30 weeks until disease progression. The first vaccination was performed after 14 to 28 days from blood sampling for determining the eligibility of patients for the clinical trial.

### Outcomes

The study's primary endpoint was overall survival (OS), defined as the length of time from the patient's random assignment to his death by any cause. The secondary endpoints were progression-free survival (PFS), the 1-year survival rate, the patients' immune responses, and safety. PFS was defined as the length of time from the patient's random assignment until objective disease progression was detected based on the PSA Working Group Consensus Criteria 2, the Response Evaluation Criteria in Solid Tumors 1.1 criteria, or death.

The patients' immune responses were assessed based on their IgG titers measured by a Luminex system (Komatsu N, Shichijo S, Nakagawa M, et al. Scand J Clin Lab Invest 2004;64:535-45) and their cytotoxic T lymphocyte (CTL) activity measured by an interferon-gamma (IFN-γ) release assay (Hida N, Maeda Y, Katagiri K, et al. Cancer Immunol Immunother 2002;51:219-28) using blood samples at pre-treatment and every six treatments. Safety was assessed on the basis of a physical examination, vital signs measurements, clinical laboratory analyses, and adverse events (AEs) graded using the Common Terminology Criteria for Adverse Events, ver. 4.0.

### Statistical analyses

All efficacy analyses were based on the full analysis set (FAS), defined as the patients who had received at least one dose of the treatment. The planned sample size of 300 patients (200 in the PPV arm and 100 in the placebo arm) provided 90% power at a two-sided significance level of 0.05 to detect a hazard ratio (HR) of 0.6 for the primary endpoint of OS, corresponding to an increase in the median OS from 11 to 17 months. All analyses were performed after 23 months from the last patient in or when 80% of events for the primary endpoint of OS was reached. The follow-up ended in October 2017, and the final analyses were performed on a database frozen in May 2018.

Survival curves were obtained by the Kaplan-Meier method, and 95% confidence intervals (CIs) were calculated. The comparison of OS values was performed by the Harrington-Fleming test. A stratified log-rank test by factors at random assignment was used to compare the OS for the PPV versus placebo arms. The effects of treatments are reported as HRs with 95%CIs and the interaction P values for subgroup categories are shown in a Forest plot. Mean changes in immune responses from baseline at each time point were compared between the two arms in a linear regression model. All toxicity grades and severe (grade 3 or more) toxicities were compared between the two arms (Fisher's exact test). All results of the statistical tests with a p-value less than 5% were accepted as significant. All analyses were performed with JMP ver. 13 software (SAS, Cary, NC).

### Patient characteristics

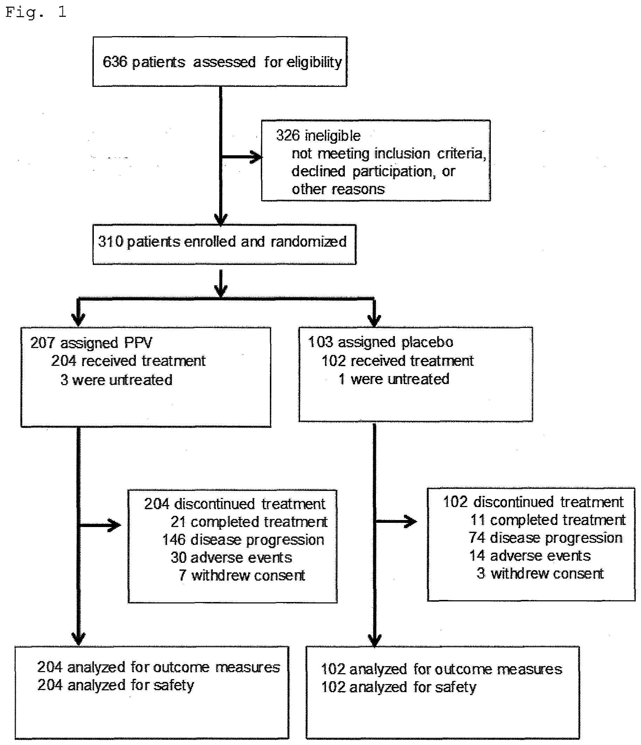

Between August 2013 and April 2016, 636 patients were screened for eligibility for this study, and we randomly assigned the 310 eligible patients to the PPV (n=207) or placebo arm (n=103). The most common reason for ineligibility was HLA mismatch. Three patients in the PPV arm and one patient in the placebo arm did not receive treatment, resulting in a total of 306 patients analyzed as the full analysis set (PPV, n=204; placebo, n=102). Fig. 1 provides the patient flowchart. No imbalances existed between the two randomization arms except for a lower prostate-specific antigen (PSA) level in the PPV arm (Table 3).

**[Table 3]**

| **Patients' Baseline Characteristics by Treatment Arm** | | | | | | |
|---|---|---|---|---|---|---|
| | | | | | | |
| | | PPV | | | Placebo | |
| | | (n = 204) | | | (n = 102) | |

| | Characteristics | No. - | % | | No. | % |
|---|---|---|---|---|---|---|
| Median age, years | | 71.0 | | | 72.0 | |
| (range) | | (53-84) | | | (56--82) | |
| Age group (years) | | | | | i | |
| | ≤65 | 37 | 18.1 | | 19 | 18.6 |
| | 66-74 | 104 | 51.0 | | 52 | 51.0 |
| | ≥75 | 63 | 30.9 | | 31 | 30.4 |
| ECOG Peformance status | | | | | | |
| | 0 | 162 | 79.4 | | 82 | 80.4 |
| | 1 | 42 | 20.6 | | 20 | 19.6 |
| Prior use of enzartamide | | | | | | |
| or abilaterone | | | | | | |
| | yes | 66 | 32.4 | | 35 | 34.3 |
| | no | 44 | 21.6 | | 20 | 19.6 |
| | unknown | 94 | 46.1 | | 47 | 46.1 |
| Serum PSA level, ng/ml | | | | | | |
| | <50 | 109 | 53.4 | | 50 | 49.0 |
| | 50-499 | 83 | 40.7 | | 37 | 36.3 |
| | ≥500 | 12 | 5.9 | | 15 | 14.7 |
| Gleason score at diagnosis | | | | | | |
| | ≤ 6 | 4 | 2.4 | | 3 | 2.9 |
| | 7 | 30 | 14.6 | | 20 | 19.6 |
| | ≥ 8 | 166 | 80.6 | | 75 | 73.5 |
| | unknown | 4 | 2.4 | | 4 | 4.0 |
| No. of metastatic sites | | | | | | |
| | 0 | 15 | 7.4 | | 7 | 6.9 |
| | 1 | 115 | 56.4 | | 61 | 59.8 |
| | ≥ 2 | 74 | 36.3 | | 34 | 33.3 |
| Median proportion of white | | | | | | |
| blood cell type, % (range) | | | | | | |
| | neutrophil | 70.0 (35-94.1) | | | 70.6 39-91) | |
| | lymphocyte | 21.7 (3.6-50,5) | | | 21.4 (6 -47.1) | |
| | eosinophil | 0.2 (0-2) | | | 0.30 (0-1.5) | |
| | basophil | 1 (0-13.8) | | | 1 11.6) | |
| | monocyte | 6.1 (1.7-15) | | | 6 (2-17) | |
| neutrophil to lymphocyte ratio | | 3.2 (0.7-26.1) | | | 3.3 9-15.2) | |
| | | | | | | |
| Abbreviations; ECOG, Eastern Cooperative Oncology Group; PPV, | | | | | | |
| personalized peptide vaccination; PS, peformance status; PSA, prostate- | | | | | | |
| specific antigen. | | | | | | |

### Efficacy

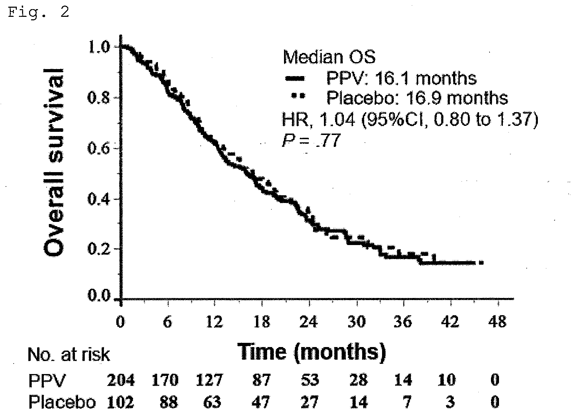

At the final analysis (cutoff date: October 1, 2017), the median length of follow-up for the censored cases was 29.8 months in the PPV arm and 27.4 months in the placebo arm, and 89.5% of all patients had discontinued treatment. The median total number of doses of treatment drugs was 12 (interquartile range [IQR] 8-19) in the PPV arm and 14 (IQR 10-21) in the placebo arm. The reasons for discontinuation (PPV vs. placebo) were disease progression (71.6% vs. 72.5%), adverse effects (AEs) (14.7% vs. 13.7%), and withdrawal consent (3.4% vs. 2.9%). One-hundred-sixty deaths (78.4%) had occurred in the PPV arm and 77 deaths (75.5%) had occurred in the placebo arm. The PPV did not improve the patients' OS compared to the placebo; the median OS was 16.1 months (95%CI: 13-18.2 months) with the PPV and 16.9 months (95%CI: 13.1-20.4 months) with the placebo: HR 1.04, 95%CI: 0.80-1.37, p=0.77 (Fig. 2). The estimated median PFS was 4.2 months (95%CI: 4.0-5.6 months) with the PPV and 5.8 months (95%CI: 4.1-7.3 months) with the placebo (HR 1.09, 95%CI: 0.85-1.40, p=0.32) (data not shown). The median 1-year survival rates were also similar between the PPV and placebo arms: 62.3% versus 62.4%.

The treatment arm effects were evaluated among various patient subgroups based on the patients' baseline characteristics. The analysis of subgroups based on the percentages of neutrophils and lymphocytes in circulation prior to the study entry revealed higher HR values for OS in un-favor of the PPV arm in (1) the patients whose neutrophil % values were greater than or equal to the median and (2) the patients whose lymphocyte % values were less than the median (HR 1.32, 95%CI: 0.91-1.95 and HR 1.35, 95%CI: 0.93-2.00), with a significant interaction (p=0.04 and p=0.05, respectively; Table 4).

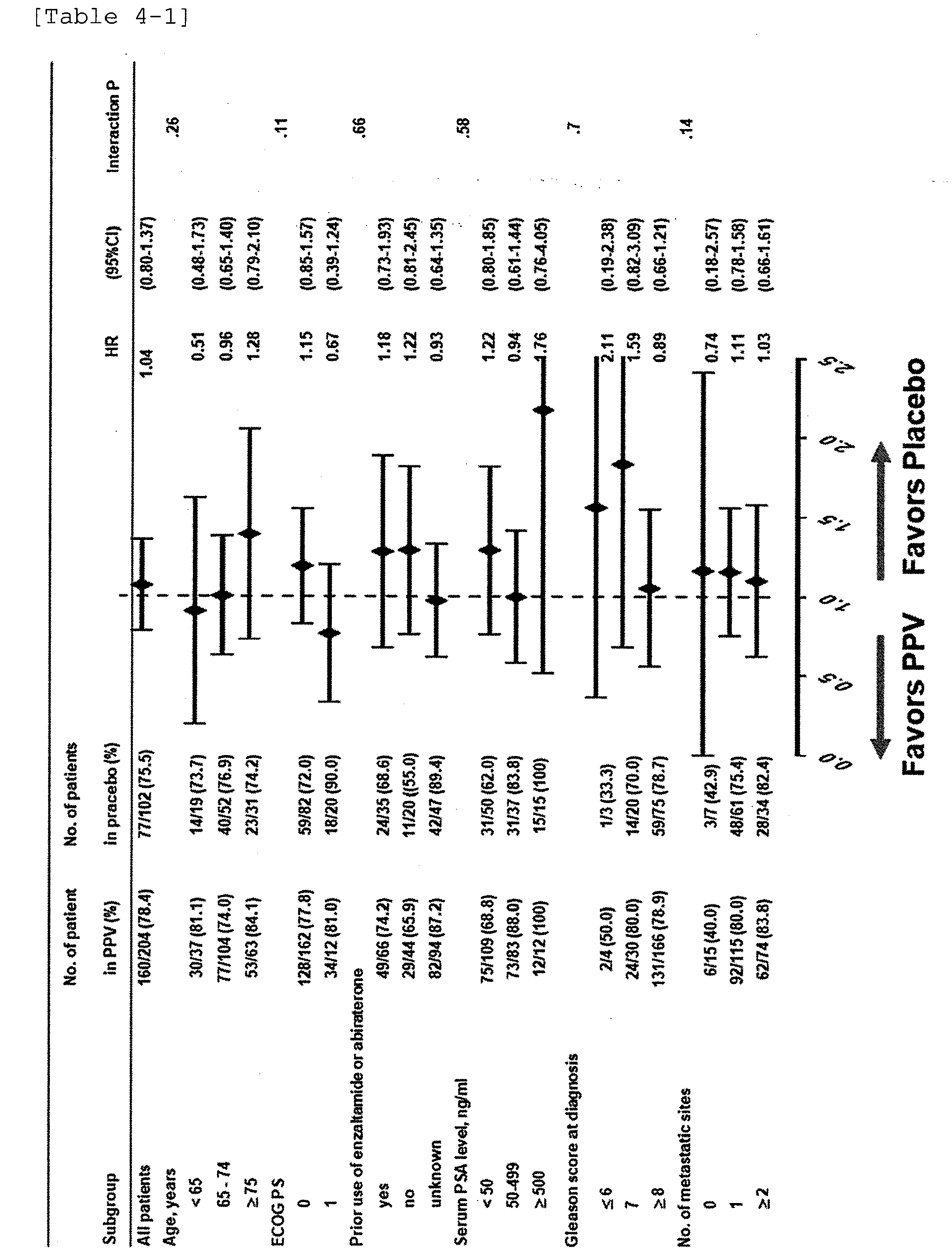

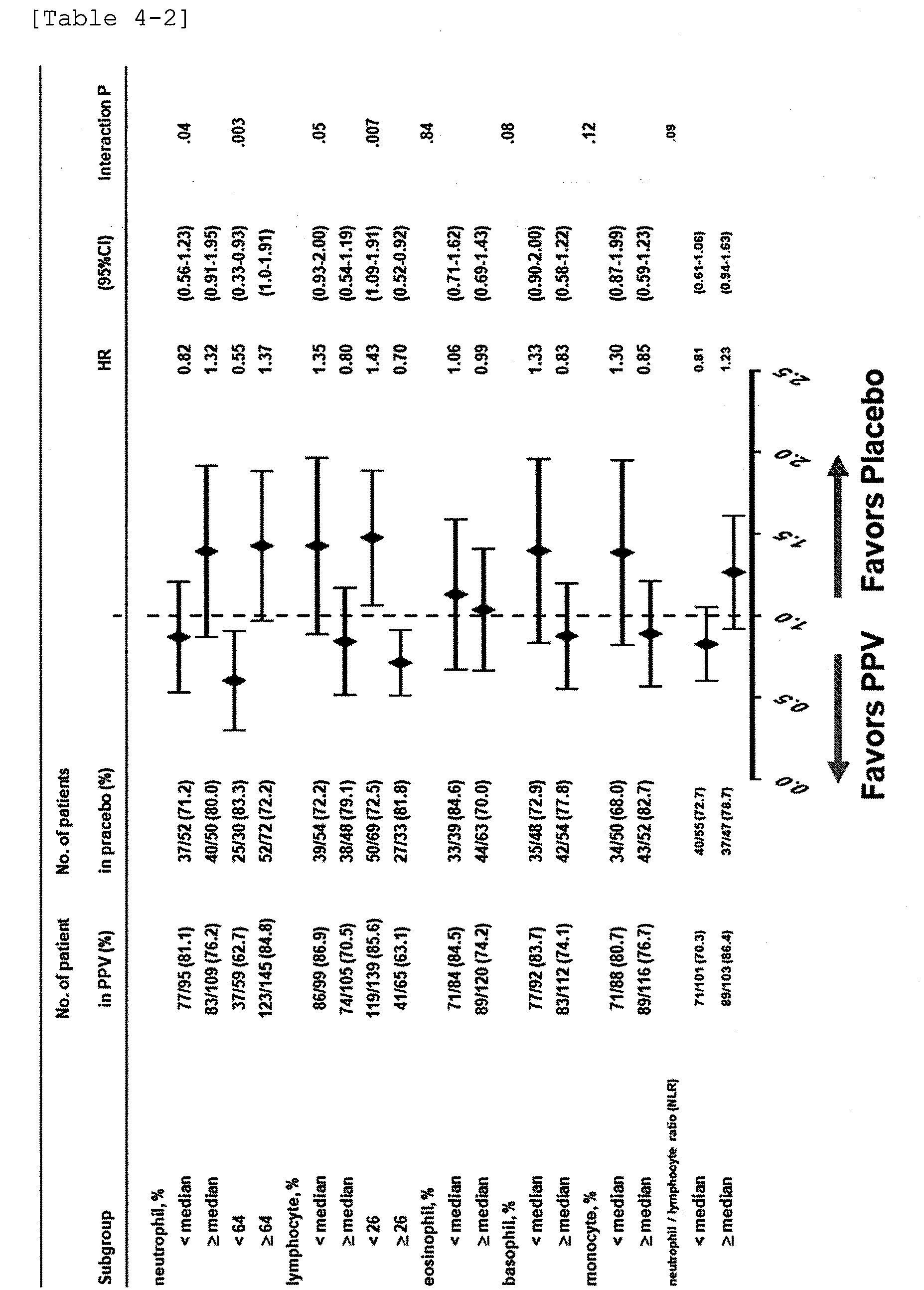

The detailed results of the correlation between each of the circulating white blood cell types and the OS of the PPV and placebo arms are shown in Table 5. The median OS of the PPV patients with percentages of neutrophils not less than median (≥700%) was significantly shorter than that of the PPV patients with lower percentages of neutrophils (HR 1.71, 95%CI: 1.3-2.3, p<0.01), whereas the median OS of the PPV patients with percentages of lymphocytes not less than median (≥21.7%) was significantly longer than that of the PPV patients with lower percentages (HR 0.63, 95%CI: 0.5-0.9, p<0.01). Similar results were obtained regarding the basophil percentages. No significant between-group difference was detected in the eosinophils or monocytes. None of these cell types affected the OS of the placebo arm. No significant between-group difference was detected in the neutrophil/lymphocyte ratio, neutrophil count, and lymphocyte count.

**[Table 5]**

| Correlation between the proportions of circulating white blood cell types and the OS of either PPV or placebo group | | | | | | | | | | | | |
|---|---|---|---|---|---|---|---|---|---|---|---|---|
| | PPV group | | | | | | placebo group | | | | | |
| proportion | median OS(M) | | 95%CI | HR | HR(95%CI) | HR(P-value) | median OS(M) | p | (95%CI) | HR | HR(95%CI) | HR(P-value) |
| neutrophil <median | 20.2 | <0.01 | 16.1-22.9 | 0.584 | 0.43-0.80 | <0.01 | 16.2 | 0.860 | 13.1-24.5 | 0.961 | 0.61-1.51 | 0.860 |
| neutrophil ≧median | 12.6 | | 10.1-15.7 | 1.712 | 1.25-2.34 | | 17.6 | | 10.3-21.8 | 1.041 | 0.66-1.63 | |
| basophil <median | 12.2 | <0.01 | 9.7-13.6 | 1.592 | 1.16-2.18 | <0.01 | 11.9 | 0.060 | 8.2-18.8 | 1.528 | 0.96-2.40 | 0.077 |
| basophil ≧median | 20.0 | | 16.6-23.7 | 0.628 | 0.46-0.86 | | 19.7 | | 15.3-24.6 | 0.654 | 0.42-1.04 | |
| eosinophil <median | 13.6 | 0.130 | 12.0-17.4 | 1.269 | 0.93-1.73 | 0.1300 | 19.9 | 0.250 | 12.0-24.7 | 0.769 | 0.49-1.21 | 0.250 |
| eosinophil ≧median | 17.3 | | 13.5-22.4 | 0.788 | 0.58-1.08 | | 15.3 | | 10.1-19.2 | 1.301 | 0.83-2.05 | |
| monocyte <median | 16.5 | 0.780 | 12.7-20.2 | 1.048 | 0.76-1.43 | 0.7700 | 21.0 | 0.080 | 15.4-24.6 | 0.668 | 0.42-1.05 | 0.080 |
| monocyte ≧median | 16.1 | | 11.3-17.8 | 0.955 | 0.70-1.31 | | 13.4 | | 10.3-18.2 | 1.496 | 0.95-2.37 | |
| lymphocyte <median | 12.7 | <0.01 | 10.1-16.0 | 1.595 | 1.17-2.18 | <0.01 | 18.8 | 0.730 | 10.6-22.6 | 0.925 | 0.59-1.45 | 0.730 |
| lymphocyte ≧median | 18.7 | | 15.7-22.9 | 0.627 | 0.46-0.86 | | 15.5 | | 12.0-22.4 | 1.081 | 0.69-1.69 | |
| Abbreviations; PPV. personalized peptide vaccination :CI. confidence interval. OS: overall survival: HR. hazard ratio | | | | | | | | | | | | |

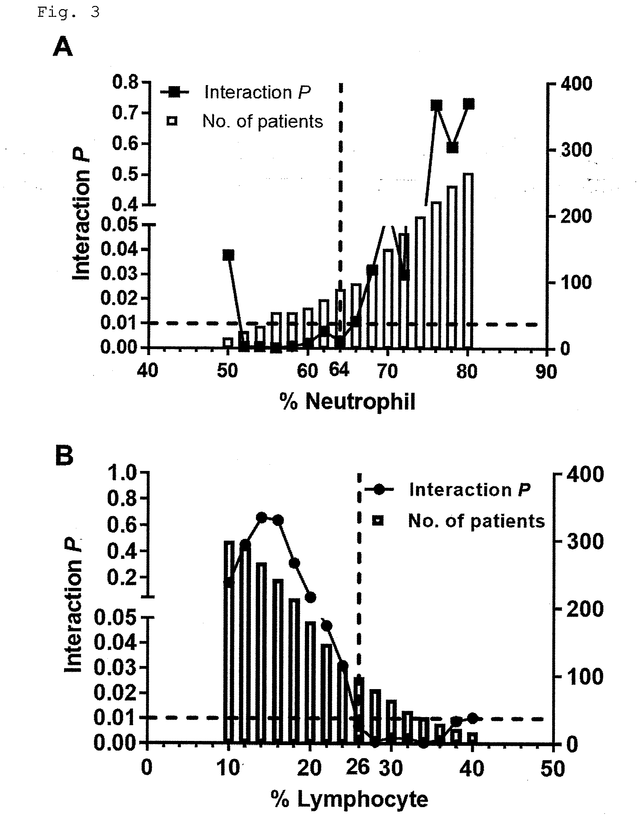

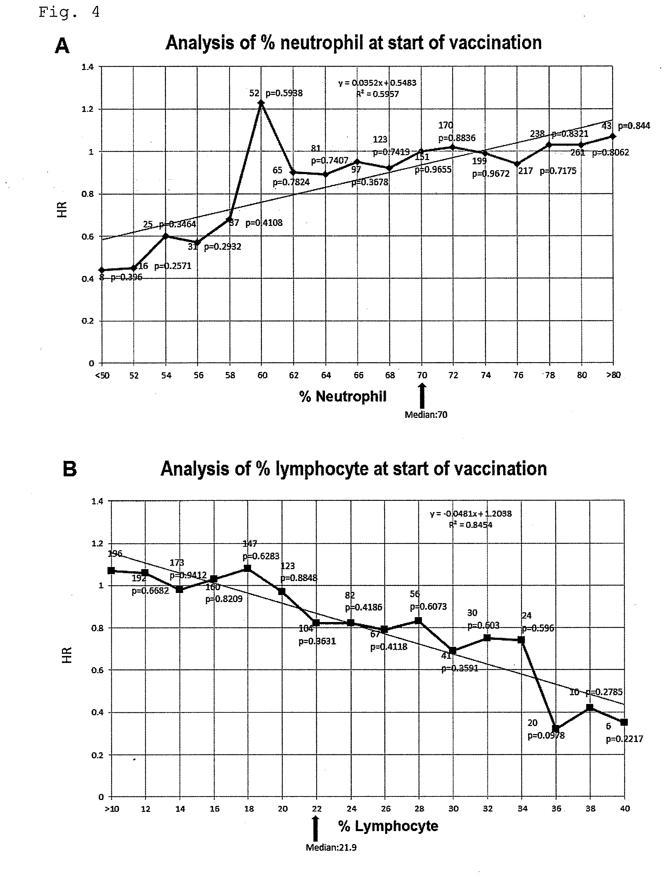

The most relevant % neutrophil or % lymphocyte cutoff level was further analyzed. Interaction p-values from 50% to 80% of the neutrophil proportion, interaction p-values from 10% to 40% of the lymphocyte proportion, and the number of target patients were plotted at each point. The most relevant % neutrophil and % lymphocyte cutoff levels were 64% and 26% (with interaction p<0.01and a larger number of patients), respectively, by the analysis of the relationship among % neutrophil (Fig. 3A) and % lymphocyte (Fig. 3B) interaction p-values and numbers of patients. On the other hand, no significant between-group difference was detected at all cutoff levels in the neutrophil proportion and lymphocyte proportion in circulation at the start of vaccination, i.e., in blood samples collected one to two hours before the treatment (Fig. 4).

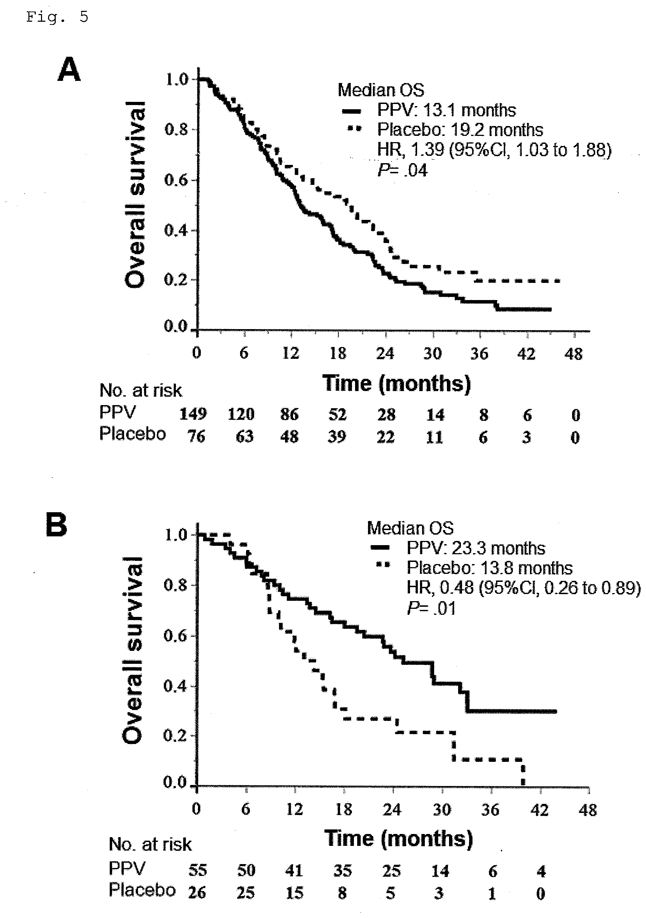

The treatment arm effects were then reanalyzed by using 64% neutrophils or 26% lymphocytes as the cutoff values. The results revealed higher HRs for OS in the PPV patients (HR 1.37, 95%CI: 1.00-1.91 for neutrophils not less than 64% and HR 1.43, 95%CI: 1.00-1.91 for lymphocytes less than 26%) with interaction p=0.003 and p=0.007, respectively (Table 4 above). The median OS for the 149 of 204 PPV patients (73.0%) who met either or both the cutoffs of neutrophils ≥64% and lymphocytes <26% was significantly shorter than that of the corresponding placebo patients (n=76, 74.3%) (median OS, PPV: 13.1 vs. placebo: 19.2 months, HR 1.39, p=0.04, Fig. 5A). In contrast, the median OS of the PPV patients with both neutrophils <64% and lymphocytes ≥26% (n=55) was significantly longer than that of the placebo patients with the same subgroups (n=26) (median OS, PPV: 25.3 vs. placebo: 13.8 months; HR 0.48, p=0.01, Fig. 5B) .

### Immune responses

Pre-treatment plasma and peripheral blood mononuclear cell (PBMC) samples were available for the study of immune responses from all 306 patients. The mean peptide-specific IgG (and CTL) levels to the selected peptides were 154 fluorescence intensity units (FIU) (0.7 pg/ml) in the PPV arm and 311 FIU (0.8 pg/ml) in the placebo arm. After the 6-dose treatment, 189 samples in the PPV and 96 samples in the placebo arm were available for the immune response evaluation. The mean value of IgG and that of CTL were significantly higher in the PPV arm compared to those in the placebo arm (PPV: 9716 vs. placebo: 284 FIU for the IgG responses, p<0.01; PPV: 80.0 vs. placebo: 0.7 pg/ml of IFN-γ for the CTL activity; p<0.01). A significant increase in peptide-specific IgG and a significant increase in CTL was observed in 27.5% and 55.6% of 189 PPV patients, respectively, but no increase was observed in the placebo patients. There was no relationship among the IgG, CTL, and OS values in the PPV arm by a linear regression model (R²=0.00012 for IgG change after the 6-dose treatment; R²=0.0026 for CTL change after the 6-dose treatment).

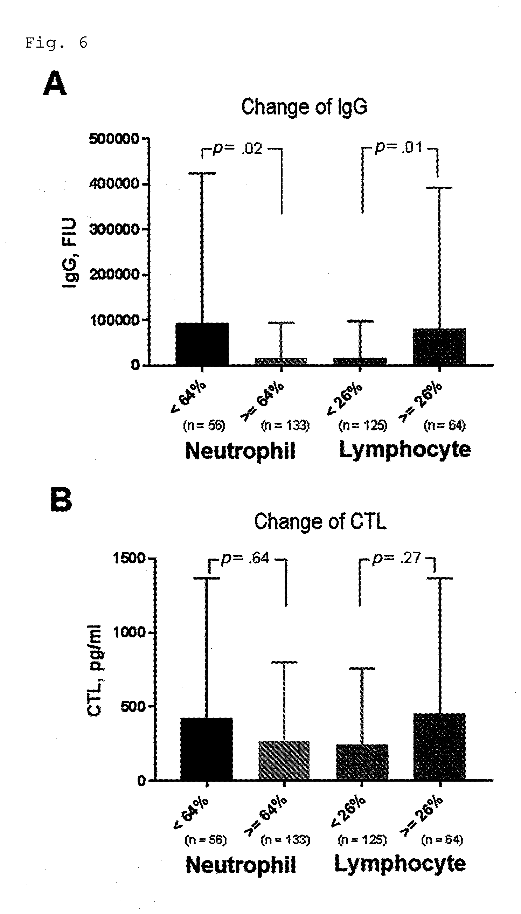

The involvement of immune responses in these factors were then analyzed. The IgG change in the PPV arm among the patients with <64% neutrophils or ≥26% lymphocytes during the first 6 doses was significantly higher than that among the patients in the subgroups with ≥64% neutrophils or <26% lymphocytes (p=0.02 or p=0.01, Fig. 6A). Similar results were obtained regarding the CTL changes, although the subgroup values were not significantly different (Fig. 6B).

### Adverse events

All AEs related to the study treatment (peptide + adjuvant or placebo + adjuvant) are shown in Table 6. Injection-site reactions and nausea were more frequent in the PPV arm than in the placebo arm (injection-site reactions: 86.8% vs. 74.5%, nausea: 9.3% vs. 2.9%). The most common AEs were injection-site reactions less than grade 3 in both arms. Grade 3 treatment-related AEs were observed in 83 patients in the PPV arm (40.7%) and 42 patients in the placebo arm (41.2%). These results indicated that the most common treatment-related AE in both arms was injection-site reaction, which was caused mainly by the use of incomplete Freund' adjuvant.

**[Table 6]**

| Any Grade or Severe AEs Recorded in ≥ 5% of Patients by Treatment Arm (CTCAE version 4.0) | | | | | | | | | | |
|---|---|---|---|---|---|---|---|---|---|---|
| AE^{a} | PPV (n = 204) | | Placebo (n = 102) | | *P-value ^{b}* | PPV (n= 204) | | Placebo (n = 102) | | *P-value*^{b} |
| | Any grade | | Any grade | | | Grade ≥ 3 | | Grade ≥ 3 | | |
| | No. | % | No. | % | | No. | % | No. | % | |
| All AEs | 178 | 87.3 | 79 | 77.5 | .02 | 83 | 40.7 | 42 | 41.2 | .52 |
| Injection site reaction | 177 | 86.8 | 76 | 74.5 | .008 | 5 | 0.0 | 1 | 0.0 | .35 |
| Cancer pain | 54 | 26.5 | 22 | 21.6 | .21 | 18 | 8.8 | 9 | 8.8 | .59 |
| Decreased appetite | 37 | 18.1 | 13 | 12.7 | .15 | 19 | 9.3 | 5 | 4.9 | .13 |
| Edema peripheral | 35 | 17.2 | 13 | 12.7 | .20 | 1 | 0.5 | 2 | 2.0 | 19 |
| Pyrexia | 31 | 15.2 | 12 | 11.8 | .26 | 2 | 1.0 | 0 | 0.0 | .45 |
| Anemia | 29 | 14.2 | 20 | 19.6 | .91 | 21 | 10.3 | 12 | 11.8 | .72 |
| Constipation | 29 | 14.2 | 14 | 13.7 | .53 | 1 | 0.5 | 1 | 1.0 | .89 |
| Nasopharyngitis | 28 | 13.7 | 9 | 8.8 | .15 | 0 | 0.0 | 0 | 0.0 | n.a. |
| Malaise | 28 | 13.7 | 9 | 8.8 | .15 | 2 | 1.0 | 1 | 1.0 | .74 |
| Weightgein | 22 | 10.8 | 12 | 11.8 | .68 | 2 | 1.0 | 1 | 1.0 | .74 |
| Nausea | 19 | 9.3 | 3 | 2.9 | .03 | 3 | 1.5 | 1 | 1.0 | .59 |
| Subcutaneous hemorrhage | 19 | 9.3 | 20 | 19.6 | 1.0 | 0 | 0.0 | 0 | 0.0 | n.a. |
| Fall | 17 | 8.3 | 5 | 4.9 | .20 | 0 | 0.0 | 1 | 1.0 | 1.0 |
| Diabetes mellitus | 16 | 7.8 | 7 | 6.9 | .48 | 4 | 2.0 | 2 | 2.0 | .68 |
| Diarrhea | 16 | 7.8 | 8 | 7.8 | .60 | 1 | 0.5 | 0 | 0.0 | .67 |
| Prostate cancer | 15 | 7.4 | 4 | 3.9 | .18 | 15 | 7.4 | 4 | 3.9 | .18 |
| Hypertension | 15 | 7.4 | 6 | 5.9 | .41 | 10 | 4.9 | 5 | 4.9 | .62 |
| Vomiting | 15 | 7.4 | 7 | 6.9 | .54 | 1 | 0.5 | 1 | 1.0 | .90 |
| Back pain | 15 | 7.4 | 6 | 5.9 | .41 | 2 | 1.0 | 1 | 1.0 | .74 |
| Insomnia | 14 | 6.9 | 5 | 4.9 | .35 | 0 | 0.0 | 0 | 0.0 | n.a. |
| Weight loss | 12 | 5.9 | 9 | 8.8 | .88 | 1 | 0.5 | 1 | 1.0 | .90 |
| Abnormal hepatic function | 11 | 5.4 | 2 | 2.0 | .13 | 4 | 2.0 | 0 | 0.0 | .20 |
| Urinary tract infection | 10 | 4.9 | 7 | 6.9 | .83 | 3 | 1.5 | 1 | 1.0 | .59 |
| Urinary retention | 9 | 4.4 | 6 | 5.9 | .92 | 1 | 0.5 | 1 | 1.0 | .90 |
| Hydronephrosis | 8 | 3.9 | 7 | 6.9 | .92 | 4 | 2.0 | 3 | 2.9 | .83 |
| Platelet count reduction | 8 | 3.9 | 9 | 8.8 | .80 | 5 | 2.5 | 2 | 2.0 | .57 |
| Hematuria | 7 | 3.4 | 6 | 5.9 | .90 | 3 | 1.5 | 2 | 2.0 | .79 |
| Dental caries | 3 | 1.5 | 6 | 5.9 | .99 | 1 | 0.5 | 0 | 0.0 | .67 |

| | | | | | | | | | | |
|---|---|---|---|---|---|---|---|---|---|---|
| Abbreviations:AE, adverse event; CTCAE, Common Terminology Criteria for Adverse Events;n.a., not available; PPV, personalized peptide vaccination. ^{a}Medical Dictionary for Regulatory Activities, version 20.0 (MedDRA) terms. ^{b}Fisher's exact test. | | | | | | | | | | |

### [Test Example 2]

### Trial

This trial, which is related to tailor-made peptide vaccines against various cancers, is phase II clinical study and was performed in four institutions, Kurume University Hospital, Kurume Cancer Vaccine Center, Sendai Kousei Hospital, and Naito Hospital. The protocol was approved by the institutional review boards or ethical committees at all of the participating institutions, and was registered in the UMIN Clinical Trials Registry (UMIN1482, 1839, 1844, 1847, 1850, 1854, 1855, 1856, 1875, 1881,1882, 1883, 1884, 2282, 3590, 5631, 6249, 6295, 6493, 7493, 8126, 8823, 8824, 8825, 8826, 8827, 8828, 10068, 19390, 2906, 2907, 2908, 2984, 2985, 2987, 3027, 3028, 3029, 3059, 3060, 3081, 3082, 3083, 5329, 10290, 11593, 14855, 19802, 19879, 6927, 11230). All patients provided their written informed consent before participating the study and after receiving a full explanation of the protocol. This clinical trial was performed in accordance with the principles of the Declaration of Helsinki.

### Patients

Eligible patients were patients aged 18 years or older and suffering from histologically confirmed malignant tumor. The enrollment period was from November 2008 to March 2017, during which 2,588 cases were enrolled in Japan. The details were as follows: 399 lung cancer patients, 354 prostate cancer patients, 344 colon cancer patients, 290 pancreatic cancer patients, 200 gastric cancer patients, 183 breast cancer patients, 139 urinary tract cancer patients, 126 biliary tract cancer patients, 118 ovarian cancer patients, 79 uterine cancer patients, 83 hepatocellular cancer patients, 50 head and neck cancer patients, 49 sarcoma patients, 35 brain tumor patients, 39 esophageal cancer patients, 30 renal cancer patients, and 60 patients with the other miscellaneous cancers. In screening test, the patients were immunoglobulin G (IgG) positive for two or more of the 31 warehouse peptides shown in Table 7. Regarding human leukocyte antigens, they were HLA-A2, -A24, or -A3 supertype (A3sup: A3, A11, A31, or A33), or HLA-A26 positive. The other inclusion criteria were as follows: an Eastern Cooperative Oncology Group (ECOG) performance status (PS) of 0 or 1; life expectancy of not less than 12 weeks; and adequate bone marrow function, hepatic function, and renal function. The exclusion criteria included acute infection, history of severe allergic reactions; pulmonary, cardiac, or other systemic diseases; and other inappropriate conditions for enrollment as judged by clinicians. Safety and immunological effects of the 31 warehouse peptides had been confirmed through previous clinical trials, and all the peptides were prepared under conditions of good manufacturing practice by Multiple Peptide System (San Diego, CA).

**[Table 7]**

| Symbol for peptide | HLA type | Origin protein | Position of peptide | Amino acid sequence |
|---|---|---|---|---|
| CypB-129 | A2, A3sup | Cyclophilin B | 129-138 | KLKHYGPGWV |
| Lck-246 | A2 | p56 lck | 246-254 | KLVERLGAA |
| Lck-422 | A2, A3sup | p56 lck | 422-430 | DVWSFGILL |
| MAP-432 | A2, A26 | ppMAPkkk | 432-440 | DLLSHAFFA |
| WHSC2-103 | A2, A3sup, A26 | WHSC2 | 103-111 | ASLDSDPWV |
| HNRPL-501 | A2, A26 | HNRPL | 501-510 | NVLHFFNAPL |
| UBE-43 | A2 | UBE2V | 43-51 | RLQEWCSVI |
| UBE-85 | A2 | UBE2V | 85-93 | LIADFLSGL |
| WHSC2-141 | A2 | WHSC2 | 141-149 | ILGELREKV |
| HNRPL-140 | A2 | HNRPL | 140-148 | ALVEFEDVL |
| SART3-302 | A2 | SART3 | 302-310 | LLQAEAPRL |
| SART3-309 | A2 | SART3 | 309-317 | RLAEYQAYI |
| SART2-93 | A24 | SART2 | 93-101 | DYSARWNEI |
| SART3-109 | A24, A3sup, A26 | SART3 | 109-118 | VYDYNCHVDL |
| Lck-208 | A24 | p56 lck | 208-216 | HYTNASDGL |
| PAP-213 | A24 | PAP | 213-221 | LYCESVHNF |
| PSA-248 | A24 | PSA | 248-257 | HYRKWIKDTI |
| EGFR-800 | A24 | EGF-R | 800-809 | DYVREHKDNI |
| MRP3-503 | A24 | MRP3 | 503-511 | LYAWEPSFL |
| MRP3-1293 | A24 | MRP3 | 1293-1302 | NYSVRYRPGL |
| SART2-161 | A24 | SART2 | 161-169 | AYDFLYNYL |
| Lck-486 | A24 | p56 lck | 486-494 | TFDYLRSVL |
| Lck-488 | A24 | p56 lck | 488-497 | DYLRSVLEDF |
| PSMA-624 | A24 | PSMA | 624-632 | TYSVSFDSL |
| EZH2-735 | A24 | EZH2 | 735-743 | KYVGIEREM |
| PTHrP-102 | A24 | PTHrP | 102-111 | RYLTQETNKV |
| SART3-511 | A3sup | SART3 | 511-519 | WLEYYNLER |
| SART3-734 | A3sup | SART3 | 734-742 | QIRPIFSNR |
| Lck-90 | A3sup | p56 lck | 90-99 | ILEQSGEWWK |
| Lck-449 | A3sup | p56 Ick | 449-458 | VIQNLERGYR |
| PAP-248 | A3sup | PAP | 248-257 | GIHKQKEKSR |

| | | | | |
|---|---|---|---|---|
| Abbreviations: HLA: human leukocyte antigen; CypB: cyclophilin B; Lck: p561ck; MAP: microtubule-associated protein; WHSC2: Wolf-Hirschhorn syndrome candidate 2; HNRPL: heterogeneous nuclear ribonucleoprotein L; UBE2V: ubiquitin-conjugating enzyme variant Kua; SART3: squamous cell carcinoma antigen 3; SART2: squamous cell carcinoma antigen 2; PAP: prostatic acid phosphatase; PSA: prostate specific antigen; EGFR: epidermal growth factor receptor; MRP3: multidrug resistance-associated protein 3; PSMA: prostate-specific membrane antigen; EZH2: enhancer of zeste homolog 2; PTHrP: parathyroid hormone-related peptide | | | | |

### Treatment

At most four peptides which were selected from the 31 warehouse peptides shown in Table 1 on the basis of each patient's human leucocyte antigen type (HLA) and pre-existing peptide-specific IgG level in a serum obtained at the time of enrollment were emulsified with Montanide ISA 51 incomplete Freund's adjuvant (Seppic, Paris, France). Each selected study drug in 1.5 ml of emulsion (3 mg/peptide) was subcutaneously administered once every week, every 2 weeks, every 4 weeks, or every 4 to 12 weeks. In one protocol, the administration was performed 6 times at 1 week interval in the first cycle, 6 times at 2 weeks intervals in the second cycle, 6 times at 4 weeks intervals in the third cycle, and at 4 to 12 weeks intervals in the following cycles. In another protocol, the administration was performed 4 times at 1 week interval in the first cycle, 8 times at 2 weeks intervals in the second cycle, 8 times at 4 weeks intervals in the third cycle, and at 4 to 12 weeks intervals in the following cycles. In another protocol, the administration was performed 4 times at 4 weeks intervals in the first cycle, 4 times at 4 to 8 weeks intervals in the second cycle, and at 4 to 12 weeks intervals in the third and following cycles. The first administration was performed 14 days after obtaining the blood for determining eligibility for the clinical trial of the patient. The trial was ended if it became difficult for the patient to visit hospital due to progression of cancer, if the patient desired, or if a primary care physician determined it difficult to continue the trial.

### Outcomes

The study's primary endpoint was overall survival (OS). OS is defined as the length of time from the date of the first administration to death of the patient by any cause. The secondary endpoints were the patients' immune responses and safety.

### Statistical Analysis

All efficacy analyses were based on the full analysis set (FAS), defined as the patients who had received at least one dose of the treatment. Survival curves were obtained by the Kaplan-Meier method, and 95% confidence intervals (CIs) were calculated. All results of the statistical tests with a p-value less than 5% were accepted as significant. All analyses were performed with JMP ver. 13 software (SAS Institute, Cary, NC) .

### Results 1

Analysis of subgroups based on the level of neutrophil percentage in circulating blood obtained prior to the clinical study (at the time of enrollment) was performed. In patients with prostate cancer, lung cancer, breast cancer, colon cancer, gastric cancer, urinary tract cancer, pancreatic cancer, biliary tract cancer, uterine cancer, ovarian cancer, and other cancers, the overall survival (median) was significantly longer in the cases in which the level of neutrophil percentage at the time of enrollment was less than 64% than in the cases in which the level was not less than 64%. In patients with brain tumor (glioblastoma) and hepatocellular cancer (liver cancer), however, there was no significant difference in the overall survival (median) between the two groups. The detailed results are shown in the following table.

**Table 8**

| **Tailor-made peptide vaccine for different cancers** | | | | | | | |
|---|---|---|---|---|---|---|---|
| | neutrophil percentage | OS(M) | p | p(95%CI) | HR | HR (95%CI) | HR (p value) |
| All | <64% | 16.8 | <0.0001 | 15.5-18.1 | 0.581 | 0.53-0.63 | <0.0001 |
| | ≥64% | 8.2 | | 7.6-8.9 | 1.722 | 1.58-1.88 | |
| Lung | <64% | 18.5 | <0.0001 | 13.0-24.3 | 0.589 | 0.47-0.74 | <0.0001 |
| | ≥64% | 9.1 | | 7.6-11.6 | 1.697 | 1.35-2.14 | |
| Colon | <64% | 16.6 | <0.0001 | 13.7-19.4 | 0.616 | 0.49-0.78 | <0.0001 |
| | ≥64% | 9.8 | | 6.9-12.5 | 1.623 | 1.29-2.04 | |
| Prostate | <64% | 30.7 | <0.0001 | 27.1-37.7 | 0.468 | 0.36-0.61 | <0.0001 |
| | ≥64% | 13.0 | | 10.9-17.1 | 2.137 | 1.65-2.79 | |
| Breast | <64% | 28.4 | 0.0002 | 22.6-39.6 | 0.522 | 0.37-0.74 | 0.0004 |
| | ≥64% | 11.3 | | 8.7-15.5 | 1.917 | 1.35-2.72 | |
| Gastric | <64% | 10.0 | 0.0019 | 8.1-12.4 | 0.627 | 0.47-0.85 | 0.0024 |
| | ≥64% | 7.7 | | 4.8-10.0 | 1.594 | 1.18-2.14 | |
| Urinary tract | <64% | 15.3 | 0.0008 | 9.9-19.0 | 0.529 | 0.36-0.77 | 0.0009 |
| | ≥64% | 4.8 | | 2.6-10.2 | 1.891 | 1.30-2.77 | |
| Pancreati c | <64% | 9.6 | <0.0001 | 7.3-11.1 | 0.505 | 0.39-0.65 | <0.0001 |
| | ≥64% | 4.4 | | 3.6-5.0 | 1.979 | 1.55-2.53 | |
| Hepatocel lular | <64% | 15.5 | 0.1522 | 9.5-21.6 | 0.712 | 0.45-1.15 | 0.1614 |
| | ≥64% | 10.8 | | 6.8-12.1 | 1.405 | 0.87-2.23 | |
| Biliary tract | <64% | 11.8 | 0.0002 | 7.4-15.4 | 0.493 | 0.34-0.72 | 0.0002 |
| | ≥64% | 4.5 | | 3.2-6.3 | 2.028 | 1.40-2.96 | |
| Uterine | <64% | 30.9 | 0.0037 | 15.2- | 0.432 | 0.23-0.76 | 0.0031 |
| | ≥64% | 8.8 | | 6.6-15.3 | 2.313 | 1.32-4.26 | |
| Ovarian | <64% | 25.0 | <0.0001 | 19.0-34.5 | 0.349 | 0.23-0.53 | <0.0001 |
| | ≥64% | 7.3 | | 4.9-15.9 | 2.862 | 1.90-4.33 | |
| Others | <64% | 18.0 | <0.0001 | 13.7-22.3 | 0.563 | 0.43-0.74 | <0.0001 |
| | ≥64% | 8.3 | | 6.3-9.7 | 1.777 | 1.36-2.34 | |
| Brain tumor | <64% | 9.5 | 0.2129 | 4.5- | 0.619 | 0.27-1.26 | 0.1961 |
| | ≥64% | 8.4 | | 5.9-10.4 | 1.615 | 0.79-3.73 | |
| KRM-20 | <64% | - | 0.0416 | 16.2- | 0.210 | 0.03-0.99 | 0.0479 |
| | ≥64% | 16.1 | | 4.0- | 4.760 | 1.01-33.47 | |

### Results 2

Analysis of subgroups based on the level of lymphocyte percentage in circulating blood obtained prior to the clinical study (at the time of enrollment) was performed. In patients with prostate cancer, lung cancer, breast cancer, colon cancer, hepatocellular cancer, urinary tract cancer, pancreatic cancer, biliary tract cancer, uterine cancer, ovarian cancer, and other cancers, the overall survival (median) was significantly longer in the cases in which the level of lymphocyte percentage at the time of enrollment was not less than 26% than in the cases in which the level was less than 26%. In patients with brain tumor and gastric cancer, however, there was no significant difference in the overall survival (median) between the two groups. The detailed results are shown in the following table.

**[Table 9]**

| **Tailor-made peptide vaccine for different cancers** | | | | | | | |
|---|---|---|---|---|---|---|---|
| | lymphocyte percentage | OS(M) | p | p(95%CI) | HR | HR (95%CI) | HR (p value) |
| All | <26% | 7.7 | <0.0001 | 7.0-8.3 | 1.896 | 1.7-2.1 | <0.0001 |
| | ≥26% | 17.8 | | 16.5-19.0 | 0.527 | 0.5-0.6 | |
| Lung | <26% | 8.6 | <0.0001 | 7.0-10.7 | 1.792 | 1.4-2.3 | <0.0001 |
| | ≥26% | 19.8 | | 13.5-25.3 | 0.558 | 0.4-0.7 | |
| Colon | <26% | 8.7 | <0.0001 | 6.6-11.0 | 2.000 | 1.6-2.5 | <0.0001 |
| | ≥26% | 18.4 | | 15.5-21.3 | 0.500 | 0.4-0.6 | |
| Prostate | <26% | 12.7 | <0.0001 | 10.8-16.6 | 2.011 | 1.6-2.6 | <0.0001 |
| | ≥26% | 29.6 | | 26.4-34.2 | 0.497 | 0.4-0.6 | |
| Breast | <26% | 11.1 | <0.0001 | 8.2-15.3 | 2.079 | 1.5-3.0 | <0.0001 |
| | ≥26% | 29.9 | | 23.5-39.6 | 0.481 | 0.3-0.7 | |
| Gastric | <26% | 8.6 | 0.1684 | 5.3-10.3 | 1.229 | 0.9-1.6 | 0.1710 |
| | ≥26% | 9.8 | | 7.4-12.1 | 0.814 | 0.6-1.1 | |
| Urinary tract | <26% | 4.2 | <0.0001 | 2.5-6.0 | 2.139 | 1.5-3.2 | <0.0001 |
| | ≥26% | 17.2 | | 10.7-19.0 | 0.467 | 0.3-0.7 | |
| Pancreati c | <26% | 4.0 | <0.0001 | 3.3-4.6 | 2.225 | 1.7-2.9 | <0.0001 |
| | ≥26% | 10.0 | | 7.9-11.1 | 0.449 | 0.4-0.6 | |
| Hepatocel lular | <26% | 8.9 | 0.0129 | 6.8-12.1 | 1.787 | 1.1-2.8 | 0.0168 |
| | ≥26% | 17.6 | | 11.2-21.7 | 0.560 | 0.4-0.9 | |
| Biliary tract | <26% | 4.5 | <0.0001 | 3.2-6.3 | 2.379 | 1.6-3.5 | <0.0001 |
| | ≥26% | 12.5 | | 7.4-17.2 | 0.420 | 0.3-0.6 | |
| Uterine | <26% | 9.2 | 0.0137 | 6.1-20.6 | 1.957 | 1.1-3.4 | 0.0136 |
| | ≥26% | 21.4 | | 14.8- | 0.511 | 0.3-0.9 | |
| Ovarian | <26% | 7.3 | <0.0001 | 4.9-13.6 | 3.172 | 2.1-4.9 | <0.0001 |
| | ≥26% | 21.8 | | 18.0-31.7 | 0.315 | 0.2-0.5 | |
| Others | <26% | 7.2 | <0.0001 | 5.5-8.9 | 2.178 | 1.7-2.9 | <0.0001 |
| | ≥26% | 20.8 | | 16.4-25.9 | 0.459 | 0.3-0.6 | |
| Brain tumor | <26% | 8.4 | 0.3777 | 5.9-10.4 | 1.433 | 0.7-3.5 | 0.3623 |
| | ≥26% | 9.5 | | 4.2- | 0.698 | 0.3-1.5 | |
| KRM-20 | <26% | 16.2 | 0.0139 | 6.7- | 9.052 | 1.5-171.4 | 0.0124 |
| | ≥26% | - | | - | 0.110 | 0.1-0.7 | |

### Results 3

Analysis of subgroups based on the levels of neutrophil and lymphocyte percentages in circulating blood obtained prior to the clinical study (at the time of enrollment) was performed. In patients with prostate cancer, lung cancer, breast cancer, colon cancer, hepatocellular cancer, urinary tract cancer, pancreatic cancer, biliary tract cancer, uterine cancer, ovarian cancer, and other cancers, the overall survival (median) was significantly longer in the cases in which the level of neutrophil percentage was less than 64% and the level of lymphocyte percentage was not less than 26% at the time of enrollment than in the other cases, which meet either or both of the cutoff values. In patients with brain tumor and gastric cancer, however, there was no significant difference in the overall survival (median) between the two groups. The detailed results are shown in the following tables.

**[Table 10-1]**

| **Tailor-made peptide vaccine for different cancers** | | | | | | | |
|---|---|---|---|---|---|---|---|
| | | OS(M ) | p | p(95%CI) | HR | HR (95%CI) | HR (p value) |
| All | neutrophil <64% lymphocyte ≥26% | 18.0 | <0.0001 | 16.8-19.6 | 0.551 | 0.5-0.6 | <0.0001 |
| | others | 8.2 | | 7.6-8.9 | 1.816 | 1.7-2.0 | |
| Lung | neutrophil <64% lymphocyte ≥26% | 20.0 | <0.0001 | 13.0-25.3 | 0.587 | 0.5-0.7 | <0.0001 |
| | others | 9.5 | | 7.6-11.9 | 1.704 | 1.4-2.2 | |
| Colon | neutrophil <64% lymphocyte ≥26% | 18.1 | <0.0001 | 14.4-21.3 | 0.578 | 0.5-0.7 | <0.0001 |
| | others | 9.9 | | 7.7-12.3 | 1.731 | 1.4-2.2 | |
| Prostate | neutrophil <64% lymphocyte ≥26% | 31.2 | <0.0001 | 27.4-38.3 | 0.472 | 0.4-0.6 | <0.0001 |
| | others | 13.3 | | 11.1-17.1 | 2.118 | 1.6-2.8 | |
| Breast | neutrophil <64% lymphocyte ≥26% | 30.8 | <0.0001 | 23.8-40.5 | 0.483 | 0.3-0.7 | <0.0001 |
| | others | 11.3 | | 8.7-15.5 | 2.072 | 1.5-2.9 | |
| Gastric | neutrophil <64% lymphocyte ≥26% | 9.8 | 0.0560 | 7.6-13.2 | 0.752 | 0.6-1.0 | 0.0571 |
| | others | 8.3 | | 5.3-10.3 | 1.330 | 1.0-1.8 | |
| Urinary tract | neutrophil <64% lymphocyte ≥26% | 17.5 | 0.0003 | 10.2-19.3 | 0.495 | 0.3-0.7 | 0.0003 |
| | others | 4.8 | | 2.6-9.3 | 2.018 | 1.4-3.0 | |
| Pancrea tic | neutrophil <64% lymphocyte ≥26% | 10.1 | <0.0001 | 8.7-11.9 | 0.446 | 0.3-0.6 | <0.0001 |
| | others | 4.4 | | 3.6-5.0 | 2.240 | 1.7-2.9 | |
| Hepatoc ellular | neutrophil <64% lymphocyte ≥26% | 17.6 | 0.0367 | 11.6-21.7 | 0.616 | 0.4-1.0 | 0.0420 |
| | others | 10.2 | | 6.9-11.9 | 1.622 | 1.0-2.6 | |

**[Table 10-2**

| **Tailor-made peptide vaccine for different cancers** | | | | | | | |
|---|---|---|---|---|---|---|---|
| | | OS(M ) | p | p(95%CI ) | HR | HR (95%CI ) | HR (p value) |
| Biliary tract | neutrophil <64% lymphocyte ≥26% | 12.5 | <0.0001 | 8.0-15.6 | 0.462 | 0.3-0.7 | <0.0001 |
| | others | 5.1 | | 3.4-6.3 | 2.165 | 1.5-3.2 | |
| Uterine | neutrophil <64% lymphocyte ≥26% | 21.4 | 0.0123 | 15.2-. | 0.483 | 0.3-0.8 | 0.0106 |
| | others | 9.2 | | 6.6-20.6 | 2.070 | 1.2-3.8 | |
| Ovarian | neutrophil <64% lymphocyte ≥26% | 23.7 | <0.0001 | 19.0-36.3 | 0.362 | 0.2-0.5 | <0.0001 |
| | others | 7.9 | | 5.2-16.6 | 2.761 | 1.8-4.2 | |
| Others | neutrophil <64% lymphocyte ≥26% | 21.3 | <0.0001 | 16.4-28.0 | 0.476 | 0.4-0.6 | <0.0001 |
| | others | 8.1 | | 6.3-9.5 | 2.101 | 1.6-2.8 | |
| Brain tumor | neutrophil <64% lymphocyte ≥26% | 9.5 | 0.3777 | 4.2-. | 0.698 | 0.29-1. 47 | 0.3623 |
| | others | 8.4 | | 5.9-10.4 | 1.433 | 0.68-3. 50 | |
| KRM-2 0 | neutrophil <64% lymphocyte ≥26% | - | 0.0328 | 14.2-. | 0.139 | 0.0-0.8 | 0.0266 |
| | others | 16.2 | | 6.7-. | 7.2 | 1.2-136 .5 | |

### [Test Example 3]

### Trial oversight

This trial was a randomized, double-blind, placebocontrolled phase II clinical study, and supported by grants of the Ministry of Health, Labor and Welfare in Japan (Project ID 2014110358). The protocol was approved by the institutional review boards or ethical committees at all of the participating institutions, and was registered in the UMIN Clinical Trials Registry (UMIN000011028). All patients provided their written informed consent before the enrollment and after receiving a full explanation of the protocol. This trial was performed in accordance with the principles of the Declaration of Helsinki.

### Patients

Patients suffering from castration-resistant prostate cancer (CRPC) not having been treated with an anti-cancer agent from 10 medical institutions in Japan were enrolled into the clinical trial. Eligible patients were patients aged 20 years or older and suffering from histologically confirmed prostate cancer. The other inclusion criteria were as follows: an Eastern Cooperative Oncology Group (ECOG) performance status (PS) of 0 or 1; life expectancy of not less than 12 weeks; serum testosterone level less than 50 ng/dl; and adequate bone marrow function, hepatic function, and renal function. The treatment of patients without a previous bilateral orchiectomy continued with luteinizing hormone-releasing agonists. The exclusion criteria included acute infection; history of severe allergic reactions; pulmonary, cardiac, or other systemic diseases; and other inappropriate conditions for enrollment as judged by clinicians.

### Treatment

Patients were randomly assigned in a 1:1 ratio to either administration of a ready-made peptide vaccine composition (KRM20) obtained by mixing 20 peptides shown in Table 11 or placebo administration. Stratification factors were the age (younger than 65 years old, or 65 years old or older) and PSA (20 ng/ml or less or 20 ng/ml or more) at each participating institution. This study was double-blinded, and all physicians, patients, and investigators giving the interventions, assessing outcomes, and analyzing data were masked to the patients' treatment assignment. KRM20 or the corresponding placebo was emulsified with Montanide ISA 51 incomplete Freund's adjuvant (Seppic, Paris, France), and 0.5 ml of an emulsion containing each study drug (20 mg of KRM20 or saline) was subcutaneously injected once every week (on days 1, 8, 15, 22, 29, and 35). The first administration was performed after 14 to 28 days from blood sampling for determining the eligibility of patients for the clinical trial. On days 1 to 36, 1 mg of dexamethasone was daily orally administered. On day 36, each test drug was subcutaneously injected after intravenous infusion of docetaxel (70 mg/m²) for 60 minutes. The treatment with the docetaxel intravenous injection and the subcutaneous administration of the study drug was performed 5 cycles with intervals of 3 weeks. During this treatment, 1 mg of dexamethasone was daily orally administered.

**[Table 11]**

| **Peptide name** | **Original protein** | **Position of peptide** | **Amino acid sequence** | **HLA type** |
|---|---|---|---|---|
| **SART3-109** | **SART3** | **109-118** | **VYDYNCHVDL** | **A24, A3 sup^{a},A26** |
| **Lck-208** | **p56 lck** | **208-216** | **HYTNASDGL** | **A24** |
| **PAP-213** | **PAP** | **213-221** | **LYCESVHNF** | **A24** |
| **PSA-248** | **PSA** | **248-257** | **HYRKWIKDTI** | **A24** |
| **EGFR-800** | **EGF-R** | **800-809** | **DYVREHKDNI** | **A24** |
| **MRP3-1293** | **MRP3** | **1293-1302** | **NYSVRYRPGL** | **A24** |
| **Lck-486** | **p56 lck** | **486-494** | **TFDYLRSVL** | **A24** |
| **Lck-488** | **p56 lck** | **488-497** | **DYLRSVLEDF** | **A24** |
| **PSMA-624** | **PSMA** | **624-632** | **TYSVSFDSL** | **A24** |
| **PTHrP-102** | **PTHrP** | **102-111** | **RYLTQETNKV** | **A24** |
| **CypB-129** | **Cyclophilin B** | **129-138** | **KLKHYGPGWV** | **A2, A3 sup^{a}** |
| **Lck-246** | **p56 lck** | **246-254** | **KLVERLGAA** | **A2** |
| **WHSC2-103** | **WHSC2** | **103-111** | **ASLDSDPWV** | **A2, A3 sup^{a}** |
| **UBE-43** | **UBE2V** | **43-51** | **RLQEWCSVI** | **A2** |
| **WHSC2-141** | **WHSC2** | **141-149** | **ILGELREKV** | **A2** |
| **HNRPL-140** | **HNRPL** | **140-148** | **ALVEFEDVL** | **A2, A3 sup^{a}, A26** |
| **SART3-302** | **SART3** | **302-310** | **LLQAEAPRL** | **A2** |
| **SART3-734** | **SART3** | **734-742** | **QIRPIFSNR** | **A3 sup^{a}** |
| **Lck-90** | **p56 lck** | **90-99** | **ILEQSGEWWK** | **A3 sup^{a}** |
| **Lck-449** | **p56 lck** | **449-458** | **VIQNLERGYR** | **A3 sup^{a}** |

| | | | | |
|---|---|---|---|---|
| ^{a}A3 sup: HLA-A3, A11, A31 or A33 Abbreviations: SART3: squamous cell carcinoma antigen 3; Lck: p561ck; PAP: prostatic acid phosphatase; PSA: prostate specific antigen; EGFR: epidermal growth factor receptor; MRP3: multidrug resistance-associated protein 3; PSMA: prostate-specific membrane antigen; PTHrP: parathyroid hormone-related peptide; CypB: cyclophilin B; WHSC2: Wolf-Hirschhorn syndrome candidate 2; UBE2V: ubiquitin-conjugating enzyme variant Kua; HNRPL: heterogeneous nuclear ribonucleoprotein L | | | | |

### Outcomes

The primary endpoint of this trial was PSA decline. The secondary endpoints were overall survival (OS), progression-free survival (PFS), overall survival, the patients' immune responses, and safety. OS is defined as the length of time from the random assignment to death of the patient by any cause.

### Statistical Analysis

All efficacy analyses were based on the full analysis set (FAS), defined as the patients who had received at least one dose of the treatment. OS data was analyzed by the Kaplan-Meier method. Survival curves were compared by log-rank test, and a hazard ratio (HR) was evaluated by Cox proportional hazard analysis. The confidence interval (CI) was 95%. Statistical analyses were performed using SAS software version 9.1 (SAS Institute, Cary, NC) with a two-sided significance level of 5%.

### Patient Characteristics

Between July 2013 and July 2014, 55 patients were screened for eligibility for this study, and 51 eligible patients were randomly assigned to either KRM20 arm (n = 25) or a placebo arm (n = 26). Since 2 patients in the KRM20 arm were not treated, 49 patients in total were analyzed as the full analysis set (KRM20: n = 23; placebo: n = 26). There was no imbalance between the randomized two arms. Most patients (72% in the KRM20 arm and 69.2% in the placebo arm) had bone disease, and all patients were refractory to previous hormone therapies. At the end of the treatment, 17 (73.9%) of the 23 patients in the KRM20 arm, and 20 (76.9%) of the 26 patients in the placebo arm had completed the treatment.

### Analysis

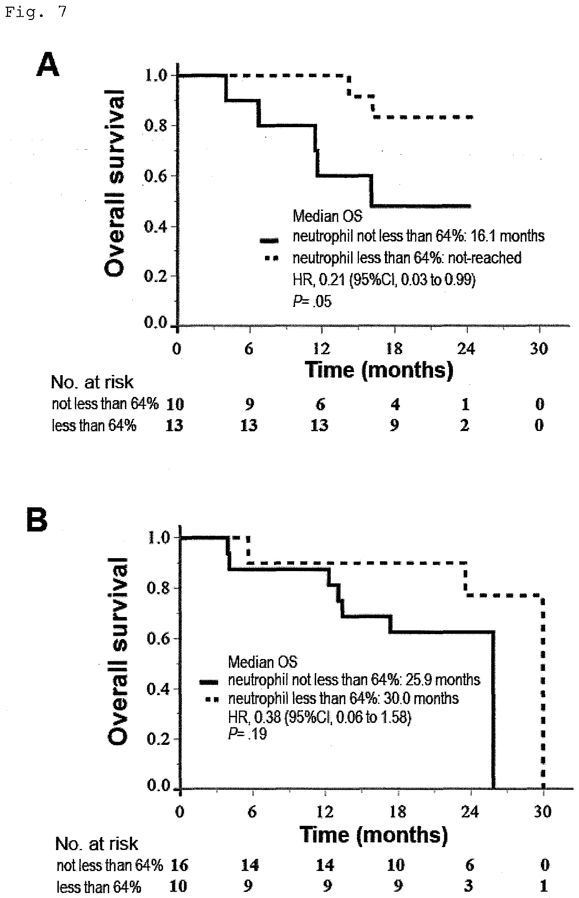

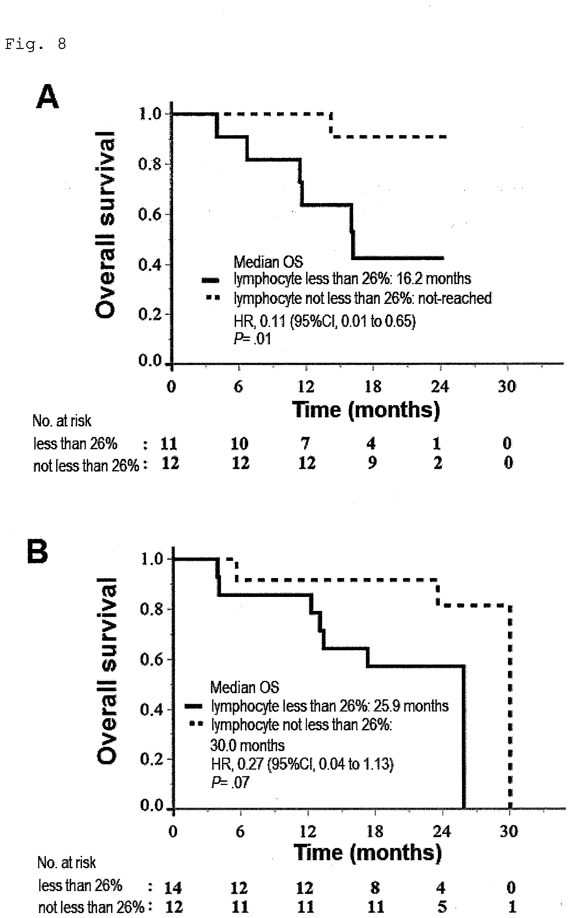

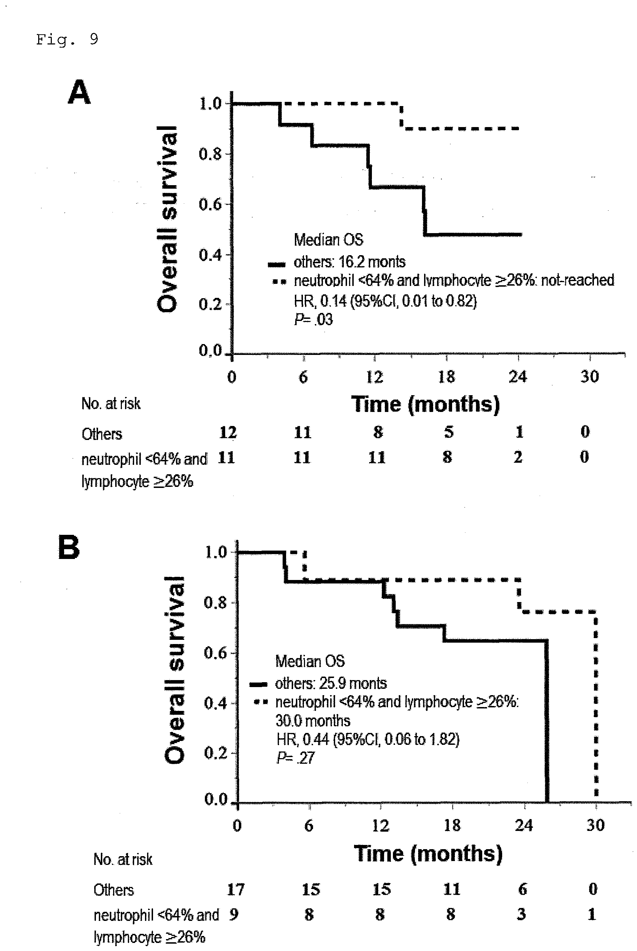

The treatment arm effects were evaluated among various patient subgroups based on the patients' baseline characteristics. Statistically significant results were obtained by analyzing subgroups based on levels of neutrophil and lymphocyte percentages in circulating blood obtained prior to the clinical trial enrollment (at the time of enrollment). Specifically, among the 23 patients in the KRM20 arm, the median OS (16.1 months) of 10 patients (43.5%) who met the cutoff level of neutrophil percentage ≥64% was significantly shorter than the median OS (not reached) of patients (n = 13) in the KRM20 arm who met the level of neutrophil percentage <64% (Fig. 7A). On the other hand, there was no significant difference in the analysis of subgroups of the placebo arm based on the level of neutrophil percentage in circulating blood obtained prior to the clinical trial (Fig. 7B). Among the 23 patients in the KRM20 arm, the median OS (16.2 months) of 11 patients (47.8%) who met the cutoff level of lymphocyte percentage <26% was significantly shorter than the median OS (not reached) of patients (n = 12) in the KRM20 arm who met the level of lymphocyte percentage ≥26% (Fig. 8A). On the other hand, there was no significant difference in the analysis of subgroups of the placebo arm based on the level of lymphocyte percentage in circulating blood obtained prior to the clinical trial (Fig. 8B). Among the 23 patients in the KRM20 arm, the median OS time (16.2 months) of 12 patients (52.2%) who met the cutoff of either or both of the cutoff levels of neutrophil percentage ≥64% and lymphocyte percentage <26% (Others) was significantly shorter than the median OS (not reached) of patients (n = 11) in the KRM20 arm who met both the level of neutrophil percentage <64% and the level of lymphocyte percentage ≥26% (Fig. 9A). On the other hand, there was no significant difference in the analysis of subgroups of the placebo arm based on the levels of neutrophil and lymphocyte percentages in circulating blood obtained prior to the clinical trial (Fig. 9B).

### [Test Example 4]

### Peptides

Thirty one peptides derived from 15 tumor associated antigens were used for personalized peptide vaccine therapy (PPV). All the 31 warehouse peptides were CTL epitopes restricted to the HLA-A2, -A24, -A3sup (-A3, -A11, -A31, or - A33), or -A26 of major histocompatibility complex (MHC) class 1A molecule (Non-Patent Literatures 4 and 5), and 12, 14, 9, and 4 peptides matched for HLA-A2, -A24, -A3sup, -A26 types of cancer patients, respectively. Details of the peptides are shown in Table 12. The peptides were prepared under conditions of good manufacturing practice by Multiple Peptide System (San Diego, CA) or American Peptide Company (Sunnyvale, CA). Two to four peptides were selected based on pre-existing immunity of the patient determined by HLA type and peptide-specific IgG levels. The selected peptides were mixed with incomplete Freund's adjuvant (Montanide ISA-51VG; Seppic, Paris, France) and subcutaneously injected into the inguinal, abdominal, neck, upper chest, or upper back area.

**[Table 12]**

| Peptide name | HLA type | Original protein | Position of peptide | Amino acid sequence |
|---|---|---|---|---|
| CypB-129 | A2,A3sup | Cyclophilin B | 129-138 | KLKHYGPGWV |
| Lck-246 | A2 | p56 lck | 246-254 | KLVERLGAA |
| Lck-422 | A2,A3sup | p56 lck | 422-430 | DVWSFGILL |
| MAP-432 | A2,A26 | ppMAPkkk | 432-440 | DLLSHAFFA |
| WHSC2-103 | A2,A3sup,A26 | WHSC2 | 103-111 | ASLDSDPWV |
| HNRPL-501 | A2,A26 | HNRPL | 501-510 | NVLHFFNAPL |
| UBE-43 | A2 | UBE2V | 43-51 | RLQEWCSVI |
| UBE-85 | A2 | UBE2V | 85-93 | LIADFLSGL |
| WHSC2-141 | A2 | WHSC2 | 141-149 | ILGELREKV |
| HNRPL -140 | A2 | HNRPL | 140-148 | ALVEFEDVL |
| SART3-302 | A2 | SART3 | 302-310 | LLQAEAPRL |
| SART3-309 | A2 | SART3 | 309-317 | RLAEYQAYI |
| SART2-93 | A24 | SART2 | 93-101 | DYSARWNEI |
| SART3-109 | A24,A3sup,A26 | SART3 | 109-118 | VYDYNCHVDL |
| Lck-208 | A24 | p56 lck | 208-216 | HYTNASDGL |
| PAP-213 | A24 | PAP | 213-221 | LYCESVHNF |
| PSA-248 | A24 | PSA | 248-257 | HYRKWIKDTI |
| EGFR-800 | A24 | EGF-R | 800-809 | DYVREHKDNI |
| MRP3-503 | A24 | MRP3 | 503-511 | LYAWEPSFL |
| MRP3-1293 | A24 | MRP3 | 1293-1302 | NYSVRYRPGL |
| SART2-161 | A24 | SART2 | 161-169 | AYDFLYNYL |
| Lck-486 | A24 | p56 lck | 486-494 | TFDYLRSVL |
| Lck-488 | A24 | p56 lck | 488-497 | DYLRSVLEDF |
| PSMA-624 | A24 | PSMA | 624-632 | TYSVSFDSL |
| EZH2-735 | A24 | EZH2 | 735-743 | KYVGIEREM |
| PTHrP-102 | A24 | PTHrP | 102-111 | RYLTQETNKV |
| SART3-511 | A3sup | SART3 | 511-519 | WLEYYNLER |
| SART3-734 | A3sup | SART3 | 734-742 | QIRPIFSNR |
| Lck-90 | A3sup | p56 lck | 90-99 | ILEQSGEWWK |
| Lck-449 | A3sup | p56 lck | 449-458 | VIQNLERGYR |
| PAP-248 | A3sup | PAP | 248-257 | GIHKQKEKSR |

Abbreviations: HLA: human leukocyte antigen; CypB: cyclophilin B; EGFR: epidermal growth factor receptor; EZH2: enhancer of zeste homolog 2; HNRPL: heterogeneous nuclear ribonucleoprotein L; Lck: p56^{lck}; MAP: microtubule-associated protein; MRP3: multidrug resistance-associated protein 3; PAP: prostatic acid phosphatase; PSA: prostate specific antigen; PSMA: prostate-specific membrane antigen; PTHrP: parathyroid hormone-related peptide; SART2: squamous cell carcinoma antigen 2; SART3: squamous cell carcinoma antigen 3; UBE2V: ubiquitin-conjugating enzyme variant Kua; WHSC2: Wolf-Hirschhorn syndrome candidate 2

### Clinical Study

Three protocols different in the vaccination intervals were used. Cancer patients who became resistant to the standard systemic therapies were vaccinated 6 times at a frequency of once a week (the first cycle), followed by 6 times at a frequency of once every 2 weeks (the second cycle). The protocol was designated as PRT1; UMIN registration Nos.: 1482, 1839, 1844, 1847, 1850, 1854, 1855, 1856, 1875, 1881, 1882, 1883, 1884, 2282, 3590, 5631, 6249, 6295, 6493, 7493, 8126, 8823, 8824, 8825, 8826, 8827, 8828, 10068, 19390. Cancer patients at any stages including early stages were vaccinated 4 times at a frequency of once every week, followed by 4 times at a frequency of once every 2 weeks (the first cycle), and subsequently 4 times at a frequency of once every 2 weeks, followed by 4 times at a frequency of once every 4 weeks (the second cycle). The protocol was designated as PRT2; UMIN registration Nos.: 2906, 2907, 2908, 2984, 2985, 2987, 3027, 3028, 3029, 3059, 3060, 3081, 3082, 3083, 5329, 10290, 11593, 14855, 19802, 19879. Alternatively, patients were vaccinated 4 times at a frequency of once every 4 weeks (the first cycle), and then vaccinated in the same schedule as the first cycle (the second cycle). The protocol was designated as PRT3; UMIN registration Nos: 6927, 11230. The patients for PRT1 or PRT2 were administered with 1.5 mL (3 mg/peptide) of an emulsion every time of the injection. The patients for PRT3 were administered with 3.0 mL (6 mg/peptide) of an emulsion every time of the injection. In all the protocols, the patients who wished to continue the PPV after the second cycle were vaccinated at two to twelve weeks intervals until withdrawal of consent or occurrence of unacceptable toxicity. This clinical trial was performed in accordance with the Declaration of Helsinki and GCP guidelines.

### Patients

From November 2008 to March 2017, this phase II clinical study was conducted at the Kurume University Cancer Vaccine Center, Kurume University Hospital, Sendai Kousei Hospital, or Naito Hospital in Japan. Patients receiving no vaccination were excluded from the analysis, and the results from the 2,588 cancer patients (399 lung cancer patients, 354 prostate cancer patients, 344 colon cancer patients, 290 pancreatic cancer patients, 200 gastric cancer patients, 183 breast cancer patients, and 818 patients with other cancers) were provided for the clinical trial. The results of the clinical trial for the 2,588 patients were partly reported previously (Non-Patent Literatures 4 to 9). Inclusion criteria were, as previously reported, the pathologically confirmed diagnosis of cancer; positive responses in pre-vaccination plasm to IgG responses for at least 2 of the 31 warehouse peptides; positive status for the HLA-A2, -A24, -A3sup, or -A26; the age ≥20; Eastern Cooperative Oncology Group (ECOG) performance status (PS) of 0 to 2 and neurological of 3 for brain tumor patients; life expectancy of not less than 12 weeks; and adequate bone marrow function, hepatic function, and renal function. Exclusion criteria were acute infection, a history of severe allergic reactions, and other systemic diseases. All the patients provided written informed consent for the enrollment in the clinical trial and data collection.

### Measurement of Immune Response

As described previously, IgG titer specific to the antigen peptides in plasma was measured by bead-based multiplexed Luminex assay (Luminex, Austin, TX) before and after vaccination, i.e., at the end of each cycle or at the end of the clinical trial (Non-Patent Literature 4). In brief, 30 mL of peripheral blood was obtained before and after vaccination. Plasma was incubated with 100 µL of peptide-coupled colorcoded beads for 2 hours at 25°C on a plate shaker. After the incubation, the resultant mixture was washed with a vacuum manifold apparatus and incubated with 100 µL of biotinylated goat anti-human IgG (gamma chain-specific; Vector Laboratories, Burlingame, CA) for 1 hour at 25°C on a plate shaker. The plate was then washed, followed by addition of 100 µL of streptavidin-PE (Life Technologies) to each well, and incubation for 30 minutes at 25°C on a plate shaker. The resultant beads were washed 4 times, followed by addition of 100 µL of Tween-PBS to each well, and detection of fluorescence intensity units (FIU) on the beads using the Luminex system. The cutoff values of anti-peptide IgG were set to 10 FIU in 100-fold diluted samples.

Each of the factors on the baseline characteristics (Table 13) and laboratory data (Table 14) at the screening time (14 days prior to the first vaccination) was also provided for the biomarker studies. The vaccinated peptides, immunological responses, overall survival (OS) during PPV treatment and follow-up study were extracted from the database of the clinical trials for the 2,588 patients.

### Estimation of clinical efficacy of individual peptides and statistical analysis

The Student's t-test and the chi-square test were used to compare quantitative and categorical variables, respectively. OS was calculated as the time in months from the date of clinical trial enrollment to death or to the date of last contact. Time-to-event endpoints were analyzed using the Kaplan-Meier method, and between-group comparison for OS was conducted using the log-rank test. Clinical efficacy of the - individual peptides for OS was evaluated by univariate and multivariate analyses with the Cox proportional hazards regression model, and HR and 95% CI were calculated. All reported p-values were two-sided, and p-values less than 0.05 were considered significant. JMP version 12 or SAS version 9.4 software (SAS Institute Inc., Cary, NC) was used to perform all analyses.

### Results

### Baseline Characteristics

Baseline characteristics and OS of the patients are shown in Table 13.

**[Table 13-1]**

| | | All | Lung | Prostate | Colon |
|---|---|---|---|---|---|
| Aqe | | 63 | 64 | 69 | 61 |
| Gender | male | 1520(10.5) | 252(9.5) | 354(18.7) | 188(11.0) |
| | female | 1068(13.7) | 147(18.0)* | 0 | 156(16.2)* |
| PS | 0 | 2072(13.7) | 279(17.7) | 291(22.3) | 308(14.8) |
| | 1 | 491(5.7) | 117(5.9) | 61(9.8) | 34(5.2) |
| | 2 | 22(3.3) | 3(2.4) | 2(3.8) | 2(2.0) |
| | 3 | 3(8.6) | 0 | 0 | 0 |
| Vaccination times | | 11 (1-76) | 10 (1-56) | 17 (1-76) | 11 (1-45) |
| Stage | I | 88(58.9) | 30(38.1) | 3(41.7) | 3(11.5) |
| | II | 104(29.5) | 19(18.5) | 15(71.3) | 6(16.6) |
| | III | 311(15.4) | 95(13.5) | 21(26.1) | 53(16.8) |
| | IV | 1753(9.9) | 246(10.0) | 315(17.6) | 259(12.3) |
| | Rec | 332(13.3) | 9(22.9) | 0 | 23(14.3) |
| Type of cancer s | lung | - | 399(11.9) | - | - |
| | prostate | - | - | 35418.7 | - |
| | colon | - | - | - | 344(13.1) |
| | pancreatic | - | - | - | - |
| | gastric | - | - | - | - |
| | breast | - | - | - | - |
| | others | - | - | - | - |
| HLA type | A24 | 1550(12.0) | 233(12.1) | 225(20.2) | 209(13.6) |
| | A2 | 1050(11.5) | 164(11.7) | 142(18.3) | 133(14.4) |
| | A3 | 1214(11.1) | 164(12.1) | 165(18.2) | 168(11.5) |
| | A26 | 552(12.4) | 107(12.6) | 72(22.4) | 71(13.8) |
| Prior systemic therapy | ope | 1574 | 155 | 86 | 317 |
| | chemotherapy | 2185 | 362 | 303 | 308 |
| | radiation | 771 | 185 | 119 | 45 |
| | localtherapy | 220 | 3 | 10 | 24 |
| Combination th erapy | all periods | | | | |
| | ope | 74 | 3 | 3 | 17 |
| | chemotherapy | 1787 | 256 | 321 | 228 |
| | radiation | 272 | 76 | 29 | 25 |
| | localtherapy | 99 | 1 | 5 | 8 |
| Study periods | | 5.4 | 5.8 | 8.9 | 5.7 |
| mOS(95%Cl) | | 11.6(11.0-1 2.3) | 11.9(10.0-1 3.5) | 18.7(16.1-22. 3) | 13.1(11.3-15. 5) |

| | | | | | |
|---|---|---|---|---|---|
| mOS:median overall survival(month). *:p<0.05 | | | | | |

**[Table 13-2]**

| | | Pancreatic | Gastric | Breast | Others |
|---|---|---|---|---|---|
| Age | | 64 | 64 | 56 | 62 |
| Gender | male | 159(5.6) | 121(9.7) | 0 | 446(9.8) |
| | female | 131(6.2) | 798.6 | 18321.2 | 372(14.8) |
| PS | 0 | 230(6.4) | 157(10.3) | 150(26.5) | 657(13.1) |
| | 1 | 58(3.7) | 42(4.1) | 33(10.9) | 146(5.2) |
| | 2 | 2(1.7) | 1(3.3) | 0 | 12(4.6) |
| | 3 | 0 | 0 | 0 | 3(8.6) |
| Vaccination times | | 9 (1-60) | 9 (1-66) | 14 (1-49) | 11 (1-57) |
| Stage | I | 3(-) | 6(24.7) | 12(-) | 31(58.9) |
| | II | 1(-) | 8(6.8) | 2332.3 | 32(32) |
| | III | 453.5 | 41(10.5) | 13(21.9) | 84(15.3) |
| | IV | 246(5.6) | 132(7.7) | 78(24.3) | 477(8.3) |
| | Rec | 36(6.1) | 13(20.0) | 57(12.2) | 194(15.9) |
| Type of cancer s | lung | - | - | - | - |
| | prostate | - | - | - | - |
| | colon | - | - | - | - |
| | pancreatic | 2905.7 | - | - | - |
| | gastric | - | 200(9.1) | - | - |
| | breast | - | - | 183(21.2) | - |
| | others | - | - | - | 818(11.3) |
| HLA type | A24 | 167(5.7) | 119(8.6) | 119(23.5) | 478(11.3) |
| | A2 | 123(5.6) | 89(8.9) | 61(18.8) | 338(11.7) |
| | A3 | 153(5.8) | 90(9.1) | 94(26.5) | 380(10.8) |
| | A26 | 56(4.7) | 36(7.2) | 32(23.7) | 178(12.3) |
| Prior systemic therapy | ope | 126 | 127 | 160 | 603 |
| | chemotherapy | 241 | 167 | 180 | 624 |
| | radiation | 49 | 9 | 102 | 262 |
| | localtherapy | 7 | 0 | 8 | 168 |
| Combination th erapy | all periods | | | | |
| | ope | 11 | 1 | 12 | 27 |
| | chemotherapy | 249 | 149 | 164 | 420 |
| | radiation | 20 | 4 | 33 | 85 |
| | localtherapy | 4 | 2 | 7 | 72 |
| Study periods | | 3.8 | 4.8 | 8.3 | 4.9 |
| mOS(95%Cl) | | 5.7(5.1-6.7) | 9.1(7.6-10.4) | 21.2(15.6-28. 4) | 11.3(9.9-12 5) |

| | | | | | |
|---|---|---|---|---|---|
| mOS:median overall survival(month). *:p<0.05 | | | | | |

In the entire 2,588 patients, the median OS was 11.6 months (M), and the 95% confidence interval (CI) was 11.0 to 12.3. For each cancer the number of patients (median OS, 95% CI) was as follows: 399 lung cancer patients (11.9 M, 95% CI: 10.0 to 13.5), 354 prostate cancer patients (18.7, 16.1 to 22.3), 344 colon cancer patients (13.1, 11.3 to 15.5), 290 pancreatic cancer patients (5.7, 5.1 to 6.7), 200 gastric cancer patients (9.1, 7.6 to 10.4), 183 breast cancer patients (21.2, 15.6 to 28.4), and 818 patients with the other cancers (Table 13). The 818 cancer patients included 139 urinary tract cancer patients (9.9, 6.2 to 12.8), 126 biliary tract cancer patients (6.3, 5.1 to 8.0), 118 ovarian cancer patients (18.0, 13.9 to 21.5), 79 uterine cancer patients (15.3, 10.0 to 21.4), 83 hepatocellular cancer patients (12.1, 10.2 to 18.0), 50 head and neck cancer patients (10.6, 6.4 to 14.4), 49 sarcoma patients (10.3, 7.2 to 17.6), 39 esophageal cancer patients (7,5, 5.4 to 9.0), 35 brain tumor patients (12.3, 7.5 to 20.8), 30 renal cancer patients (31.8, 10.4 to 59.8), and 70 patients with the other miscellaneous cancers (data not shown).

The patients included 1,520 male and 1,068 female patients with the median age of 63 years. The median OS of male and female patients was 10.5 and 13.7 M, respectively, and the OS of female patients with lung and colon cancers was longer than that of male patients. Patients with better PS had significantly longer OS in the patients with all the cancers. Patients at earlier stage had longer OS than patients at advanced stages for all cancers except for colorectal cancer. In contrast, no significant difference of the median OS was found with regard to difference in HLA-class I (A24+ patients (1,550): 12.0 M, A2+ patients (1,050): 11.5 M, A3sup+ patients (1,214): 11.1M, and A26+ patients (552): 12.4 M) (Table 13). The median OS in the patients under PRT1 (n=1,522, 10.0 M) was significantly shorter than that of those under PRT2 (n=783, 16.2M) or PRT3 (n=283, 12.5M) primarily since the majority of the patients under PRT1 entered to the study after the failure to the standard chemotherapy or targeted therapy (data not shown).

Among the 2,588 patients, the numbers of patients having received systemic therapies prior to the PPV vaccination were as follows: 1,574 cases for surgical operations, 2,185 cases for targeted therapies or chemotherapies, 771 cases for radiation therapies, and 220 cases for local therapies. The numbers of patients receiving systemic therapies combined with the PPV vaccination were as follows: 74 cases for surgical operations, 1,787 cases for targeted therapies or chemotherapies, 272 cases for radiation therapies, and 99 cases for local therapies. Systemic therapies prior to or during the PPV in each type of cancers are also given in Table 13.

The median vaccination time was 11 ranging from 1 to 76, while the median study period was 5.4 M in all 2588 cases. Those for each cancer are also shown in Table 13. The median follow-up time was 11.3M.

### Circulating Blood Cells and OS

The correlation between pre-vaccination circulating blood cell numbers or proportion and OS was examined. The results are shown in Table 14.

**[Table 14]**

| Factors(n) | Median value | OS :median< vs median≥ | HR (95%Cl) | p value |
|---|---|---|---|---|
| Pre-vaccination cell cou nts | | | | |
| White blood cells(2,588 ) | 5,600 | 15.3/8.9 | 1.47(1.35-1.60) | <0.01 |
| Red blood cells(2,588) | 384 | 8.7/16.4 | 0.61(0.56-0.66) | <0.01 |
| Platelets(2,588) | 21.4 | 13.7/10.1 | 1.29(1.19-1.40) | <0.01 |
| Neutrophils(2,587) | 3,519 | 16.1/8.3 | 1.62(1.49-1.76) | <0.01 |
| % neutrophil(2,588) | 64.8 | 16.5/8.2 | 1.70(1.56-1.85) | <0.01 |
| Lymphocytes(2,588) | 1,346 | 10.2/13.6 | 0.78(0.72-0.85) | <0.01 |
| % lymphocyte(2,588) | 25.1 | 7.6/17.5 | 0.53(0.48-0.57) | <0.01 |
| Basophiles(2,555) | 20.6 | 11.8/11.7 | 0.97(0.89-1.05) | 0.43 |
| %Basophil(2,555) | 0.4 | 10.3/12.8 | 0.82(0.75-0.89) | <0.01 |
| Eosinophils(2,562) | 101 | 11.6/11.7 | 0.98(0.90-1.07) | 0.71 |
| % Eosinophil (2,562) | 1.9 | 10.8/12.4 | 0.90(0.83-0.98) | 0.01 |
| Monocytes (2,575) | 352 | 16.5/8.5 | 1.67(1.54-1.82) | <0.01 |
| % Monocyte (2,575) | 6.2 | 13.7/10.1 | 1.28(1.18-1.40) | <0.01 |
| % Neutrophil-lymphocyt e ratio (2,588) | 2.6 | 17.2/7.6 | 1.85(1.70-2.02) | <0.01 |

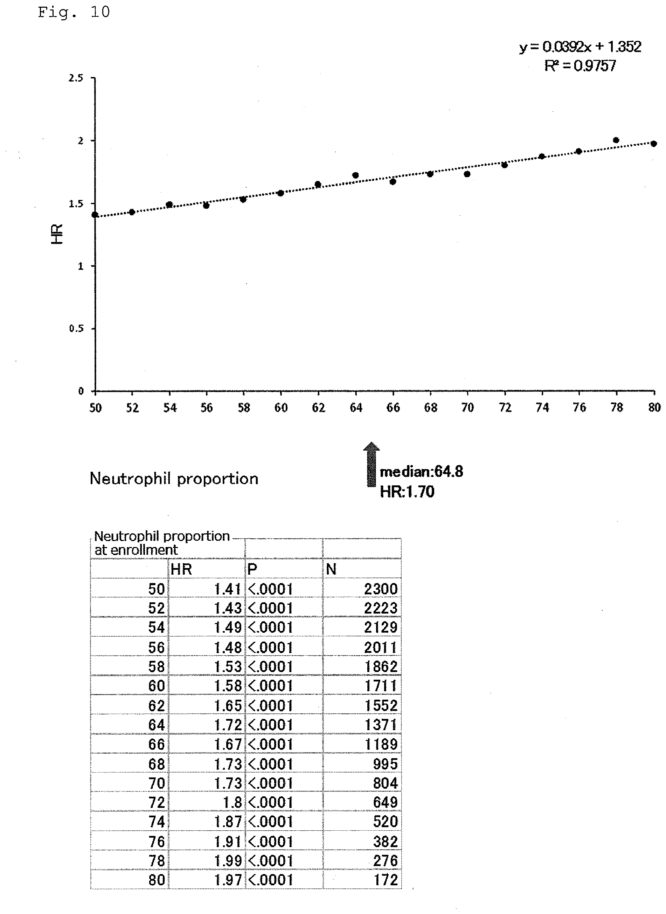

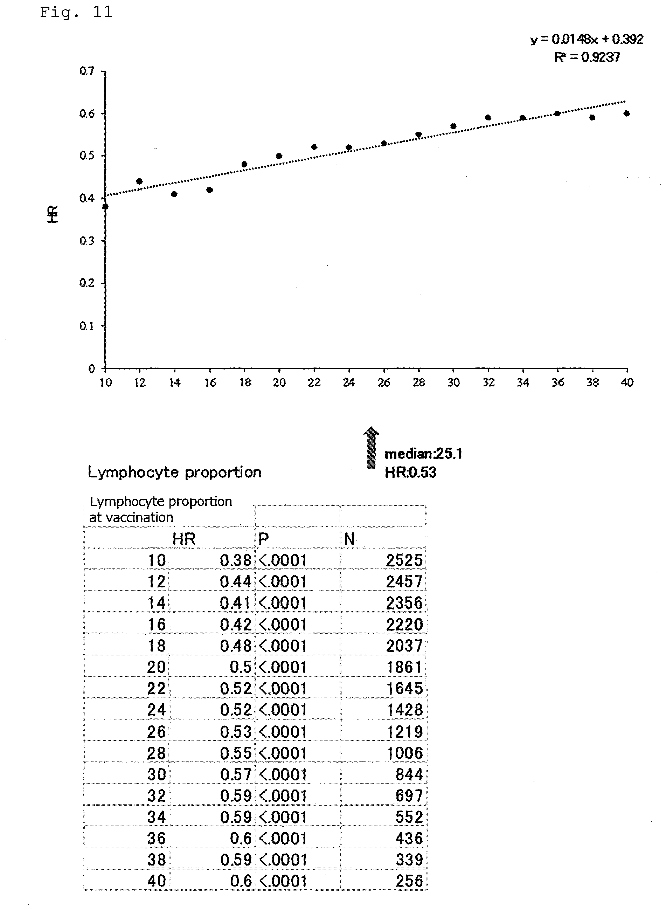

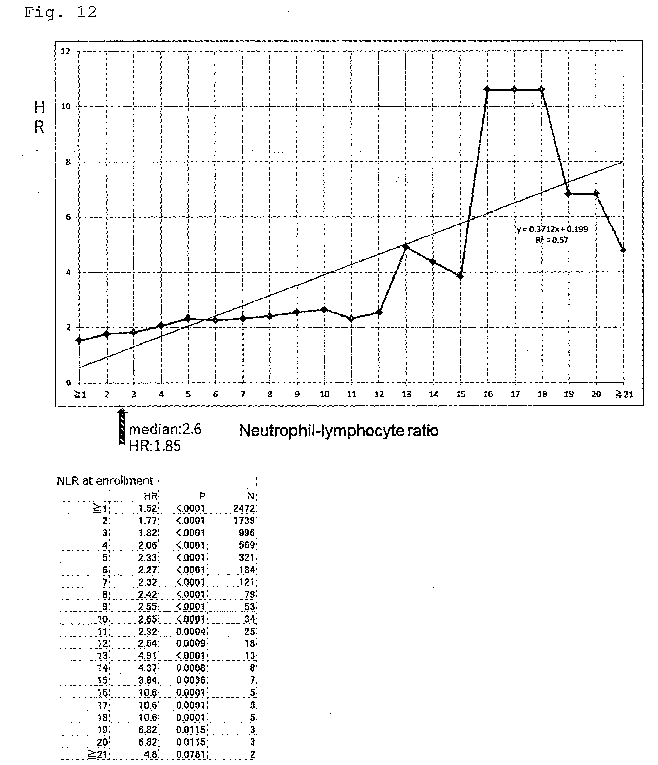

The median OS of PPV patients having the number of neutrophils, monocytes or platelets equal to or larger than the median was significantly shorter than that of PPV patients having lower numbers (p < 0.01), whereas the median OS of PPV patients having the number of lymphocytes or red blood cells equal to or larger than the median was significantly longer than that of PPV patients having lower numbers (p < 0.01). The similar trends were obtained in the cellular proportions with the more sensitive HRs as compared to those of the cellular numbers. The more potent unfavorable or favorable factors for OS was the median percentage of neutrophil (64.8%, HR:1.70) or percentage of lymphocyte (25.1%, HR:0.53) with co-efficiency (R²) of 0.97 or 0.92, respectively (Figs. 10 and 11). These percentages (64.8 and 25.1%) were mostly identical to those of the randomized, double-blind, placebo-controlled, phase III trials for advanced prostate cancer patients (unpublished; the most relevant cutoff level between the OS of PPV and that of placebo patients was 64% neutrophil or 26% lymphocyte). Accordingly, the cutoff values were set to 64% for neutrophils and 26% for lymphocytes also in this trial. The median OS of PPV patients with neutrophil-lymphocyte-ratio (NLR) equal to or higher than the median was also significantly shorter than that of PPV patients with NLR lower than the median (7.6 vs 17.2 M HR;1.85, p<0.01) with co-efficiency (R²) of 0.57 (Fig. 12) .

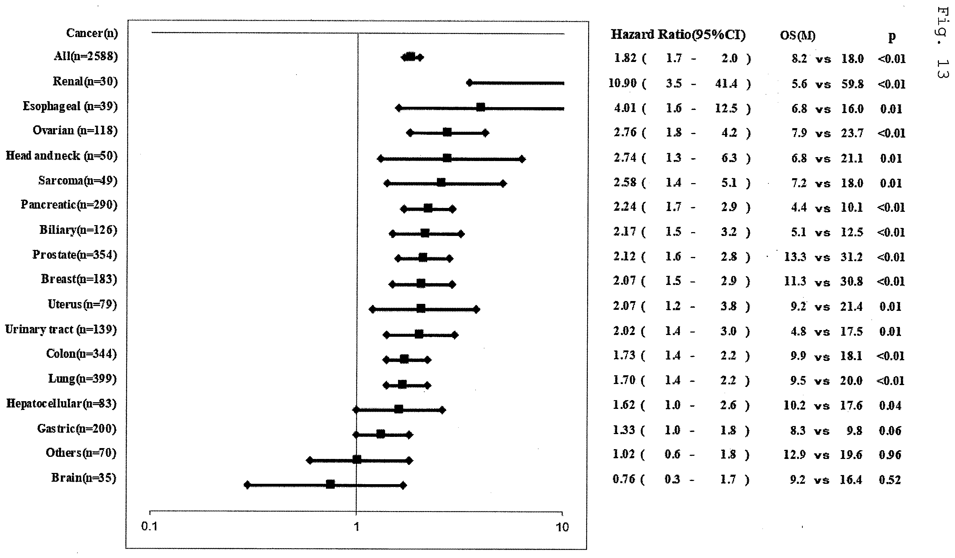

The median OS for the 1,522 of 2,588 cancer patients (58.8 %) who met either or both the cutoffs of neutrophil ≥64% and lymphocyte <26% was significantly shorter than that of the remaining 1,066 patients who showed both <64% neutrophil and ≥26% lymphocyte together (8.2 M, 95% CI: 7.6-8.9 vs 18.0, 16.8-19.6, HR: 1.8, p<0.01). The results are shown in Fig. 13. The similar results were obtained in the patients with all different cancer types tested except for gastric, brain tumor, and the other miscellaneous cancers.

The patients having the levels of neutrophil percentage at the time of enrollment less than 64% and the patients having the levels not less than 64% were compared. The results are shown in Table 15.

**[Table 15]**

| | neutrophil percentage | OS(M) | OS (95%Cl) | OS (p value) | HR | HR(95%Cl) | HR (p value) |
|---|---|---|---|---|---|---|---|
| All | <64% | 16.8 | 15.5-18.1 | <0.0001 | 0.58 | 0.53-0.63 | <0.0001 |
| | ≥64% | 8.2 | 7.6-8.9 | | 1.72 | 1.58-1.88 | |
| Lung | <64% | 18.5 | 13.0-24.3 | <0.0001 | 0.59 | 0.47-0.74 | <0.0001 |
| | ≥64% | 9.1 | 7.6-11.6 | | 1.70 | 1.35-2.14 | |
| Colon | <64% | 16.6 | 13.7-19.4 | <0.0001 | 0.62 | 0.49-0.78 | <0.0001 |
| | ≥64% | 9.8 | 6.9-12.5 | | 1.62 | 1.29-2.04 | |
| Prostate | <64% | 30.7 | 27.1-37.7 | <0.0001 | 0.47 | 0.36-0.61 | <0.0001 |
| | ≥64% | 13.0 | 10.9-17.1 | | 2.14 | 1.65-2.79 | |
| Breast | <64% | 28.4 | 22.6-39.6 | 0.0002 | 0.52 | 0.37-0.74 | 0.0004 |
| | ≥64% | 11.3 | 8.7-15.5 | | 1.92 | 1.35-2.72 | |
| Gastric | <64% | 10.0 | 8.1-12.4 | 0.0019 | 0.63 | 0.47-0.85 | 0.0024 |
| | ≥64% | 7.7 | 4.8-10.0 | | 1.59 | 1.18-2.14 | |
| Urinary tract | <64% | 15.3 | 9.9-19.0 | 0.0008 | 0.53 | 0.36-0.77 | 0.0009 |
| | ≥64% | 4.8 | 2.6-10.2 | | 1.89 | 1.30-2.77 | |
| Pancreati c | <64% | 9.6 | 7.3-11.1 | <0.0001 | 0.51 | 0.39-0.65 | <0.0001 |
| | ≥64% | 4.4 | 3.6-5.0 | | 1.98 | 1.55-2.53 | |
| Hepatocel lular | <64% | 15.5 | 9.5-21.6 | 0.1522 | 0.71 | 0.45-1.15 | 0.1614 |
| | ≥64% | 10.8 | 6.8-12.1 | | 1.40 | 0.87-2.23 | |
| Biliary tract | <64% | 11.8 | 7.4-15.4 | 0.0002 | 0.49 | 0.34-0.72 | 0.0002 |
| | ≥64% | 4.5 | 3.2-6.3 | | 2.03 | 1.40-2.96 | |
| Uterine | <64% | 30.9 | 15.2- | 0.0037 | 0.43 | 0.23-0.76 | 0.0031 |
| | ≥64% | 8.8 | 6.6-15.3 | | 2.31 | 1.32-4.26 | |
| Ovarian | <64% | 25.0 | 19.0-34.5 | <0.0001 | 0.35 | 0.23-0.53 | <0.0001 |
| | ≥64% | 7.3 | 4.9-15.9 | | 2.86 | 1.90-4.33 | |
| Brain tumor | <64% | 12.3 | 5.4-20.8 | 0.1680 | 1.88 | 0.79-4.98 | 0.1599 |
| | ≥64% | 32.8 | 4.3- | | 0.53 | 0.20-1.27 | |
| Renal | <64% | 59.8 | 29.5- | <0.0001 | 0.09 | 0.02-0.29 | <0.0001 |
| | ≥64% | 5.6 | 1.7-11.8 | | 10.92 | 3.50-41.43 | |
| Esophage al | <64% | 12.1 | 1.4-22.6 | 0.0256 | 0.40 | 0.16-0.87 | 0.0199 |
| | ≥64% | 6.6 | 4.8-8.9 | | 2.53 | 1.15-6.23 | |
| Head and neck | <64% | 20.8 | 8.1-33.8 | 0.0233 | 0.45 | 0.21-0.88 | 0.0195 |
| | ≥64% | 6.8 | 4.2-13.6 | | 2.24 | 1.13-4.77 | |
| Sarcoma | <64% | 17.6 | 8.8-22.3 | 0.0157 | 0.49 | 0.26-0.88 | 0.0176 |
| | ≥64% | 7.2 | 2.2-11.4 | | 2.06 | 1.14-3.79 | |
| Others | <64% | 17.3 | 8.4-25.9 | 0.8881 | 1.04 | 0.61-1.78 | 0.8883 |
| | ≥64% | 13.7 | 6.2-32 | | 0.96 | 0.56-1.65 | |

The median OS of the patients having the levels of neutrophil percentage less than 64% was significantly longer than that of the patients having the levels of neutrophil percentage not less than 64% in the cases of lung cancer, colon cancer, prostate cancer, breast cancer, gastric cancer, urinary tract cancer, pancreatic cancer, biliary tract cancer, uterine cancer, ovarian cancer, renal cancer, esophageal cancer, head and neck cancer, and sarcoma. There was no significant difference in the median OS between the two groups in the cases of brain tumor, hepatocellular cancer, and the other cancers.

The patients having the levels of lymphocyte percentage at the time of enrollment less than 26% and the patients having the levels not less than 26% were compared. The results are shown in Table 16.

**[Table 16]**

| | lymphocyte percentaqe | OS(M) | OS (95%Cl) | OS (p value) | HR | HR (95%Cl) | HR (p value) |
|---|---|---|---|---|---|---|---|
| All | <26% | 7.7 | 7.0-8.3 | <0.0001 | 1.90 | 1.74-2.07 | <0.0001 |
| | ≥26% | 17.8 | 16.5-19.0 | | 0.53 | 0.48-0.57 | |
| Lung | <26% | 8.6 | 7.0-10.7 | <0.0001 | 1.79 | 1.43-2.25 | <0.0001 |
| | ≥26% | 19.8 | 13.5-25.3 | | 0.56 | 0.44-0.70 | |
| Colon | <26% | 8.7 | 6.6-11.0 | <0.0001 | 2.00 | 1.59-2.52 | <0.0001 |
| | ≥26% | 18.4 | 15.5-21.3 | | 0.50 | 0.40-0.63 | |
| Prostate | <26% | 12.7 | 10.8-16.6 | <0.0001 | 2.01 | 1.57-2.59 | <0.0001 |
| | ≥26% | 29.6 | 26.4-34.2 | | 0.50 | 0.39-0.64 | |
| Breast | <26% | 11.1 | 8.2-15.3 | <0.0001 | 2.08 | 1.46-2.95 | <0.0001 |
| | ≥26% | 29.9 | 23.5-39.6 | | 0.48 | 0.34-0.69 | |
| Gastric | <26% | 8.6 | 5.3-10.3 | 0.1684 | 1.23 | 0.91-1.65 | 0.1710 |
| | ≥26% | 9.8 | 7.4-12.1 | | 0.81 | 0.61-1.09 | |
| Urinary tract | <26% | 4.2 | 2.5-6.0 | <0.0001 | 2.14 | 1.47-3.15 | <0.0001 |
| | ≥26% | 17.2 | 10.7-19.0 | | 0.47 | 0.32-0.68 | |
| Pancreati c | <26% | 4.0 | 3.3-4.6 | <0.0001 | 2.22 | 1.74-2.86 | <0.0001 |
| | ≥26% | 10.0 | 7.9-11.1 | | 0.45 | 0.35-0.58 | |
| Hepatocel lular | <26% | 8.9 | 6.8-12.1 | 0.0129 | 1.79 | 1.11-2.83 | 0.0168 |
| | ≥26% | 17.6 | 11.2-21.7 | | 0.56 | 0.35-0.90 | |
| Biliary tract | <26% | 4.5 | 3.2-6.3 | <0.0001 | 2.38 | 1.62-3.54 | <0.0001 |
| | ≥26% | 12.5 | 7.4-17.2 | | 0.42 | 0.28-0.62 | |
| Uterine | <26% | 9.2 | 6.1-20.6 | 0.0137 | 1.96 | 1.15-3.42 | 0.0136 |
| | ≥26% | 21.4 | 14.8- | | 0.51 | 0.29-0.87 | |
| Ovarian | <26% | 7.3 | 4.9-13.6 | <0.0001 | 3.17 | 2.06-4.86 | <0.0001 |
| | ≥26% | 21.8 | 18.0-31.7 | | 0.32 | 0.21-0.49 | |
| Brain tumor | <26% | 12.3 | 5.4- | 0.3471 | 0.67 | 0.27-1.54 | 0.3445 |
| | ≥26% | 14.3 | 6.3-20.8 | | 1.50 | 0.65-3.65 | |
| Renal | <26% | 4.5 | 1.7-11.8 | <0.0001 | 19.90 | 4.95-134.4 3 | <0.0001 |
| | ≥26% | 59.8 | 29.5- | | 0.05 | 0.01-0.20 | |
| Esophage al | <26% | 6.0 | 3.7-8.7 | 0.0063 | 3.12 | 1.40-7.73 | 0.0043 |
| | ≥26% | 15.1 | 2.3-22.6 | | 0.32 | 0.13-0.71 | |
| Head and neck | <26% | 6.5 | 4.2-11.6 | 0.0020 | 3.17 | 1.54-7.23 | 0.0013 |
| | ≥26% | 29.4 | 11.7-39.5 | | 0.32 | 0.14-0.65 | |
| Sarcoma | <26% | 5.2 | 2.2-11.1 | 0.0053 | 2.33 | 1.27-4.35 | 0.0059 |
| | ≥26% | 17.6 | 9.6-28.0 | | 0.43 | 0.23-0.78 | |
| Others | <26% | 9.7 | 5.2-25.6 | 0.3505 | 1.29 | 0.75-2.20 | 0.3529 |
| | ≥26% | 20.0 | 10.3-27.9 | | 0.78 | 0.45-1.33 | |

The median OS of the patients having the levels of lymphocyte percentage not less than 26% was significantly longer than that of the patients having the levels of lymphocyte percentage less than 26% in the cases of lung cancer, colon cancer, prostate cancer, breast cancer, urinary tract cancer, pancreatic cancer, hepatocellular cancer, biliary tract cancer, uterine cancer, ovarian cancer, renal cancer, esophageal cancer, head and neck cancer, and sarcoma. There was no significant difference in the median OS between the two groups in the cases of brain tumor, gastric cancer, and the other cancers.

### Immune Response to 31 Peptides

Pre-vaccination peptide-specific IgG titers to 31 peptides were measured using a Luminex system. As a detectable IgG level, 10 FIU was employed as the cut off level, as reported previously (Noguchi M, et al., Cancer Immunol Immunother. 2013 May; 62(5): 919-29; Sasada T, et al., Current Medicinal Chemistry. 21: 2332-2345, 2014; Kibe S, et al., Cancer Immunology Research 2014; 2(12): 1154-62). The positive rate of the patients showing ≥10 FIU was largely different among 31 peptides (12 to 87%) with the median positive rate of 55%. The peptides are listed in the descending order of the positive rates in Table 17. For example, SART2-93 and Lck-422 are listed on the top and bottom lines, respectively, as the positive rate of SART2-93 is 87 % (2,248 positive vs 340 negative cases) and the positive rate of Lck-422 is 12% (316 vs 2,272). In the patients showing the detectable levels, IgG titers were different among the 31 peptides and the median FIU was 40 ranging from 23 to 111.

Post-vaccination lgG titers were measured at the ends of the first cycle and the second cycle. Some patients (472 of 2,588 patients, 18.3%) were dropped from the clinical trials prior to the end of first cycle due to rapid progressive diseases. The rate of patients showing positive IgG responses in 2,116 patients who completed at least the first cycle was largely different among 31 peptides, ranging from 22 to 74 % with the median rate of 48%. EZH2-735 and Lck-422 induced the highest and lowest rate of positive immune responses (51 of 69 cases and 20 of 92 cases), respectively (Table 17). In the patients showing the positive responses, IgG titers were different among the 31 peptides and the median FIU ranged from 312 to 25,121.

**[Table 17]**

| | **Pre-vaccination IgG** | | **Post vaccination IgG** | | **Correlation between immune responses and OS** | |
|---|---|---|---|---|---|---|
| Peptides | Positive/ Negative patients | Median (FIU) | Number of vaccinated (positive) patients | Median (FIU) | Positive (mOS) / Negative (mOS) | p-value |
| SART2-93 | 2,248/340 | 66 | 1137(362) | 3,435 | 362(21.7)/568(12.3) | <0.01 |
| Lck-486 | 2,184/404 | 42 | 879(573) | 15,147 | 573(19.4)/186(7.2) | <0.01 |
| Lck-488 | 2,147/441 | 60 | 1103(635) | 11,594 | 635(20.6)/277(7.8) | <0.01 |
| Lck-90 | 2,087/501 | 44 | 502(187) | 3,350 | 187(20.9)/222(10.3) | <0.01 |
| SART3-734 | 2,041/547 | 111 | 625(225) | 4,816 | 225(22.3)/280(11.0) | <0.01 |
| PSA-248 | 2,035/553 | 48 | 283(200) | 13,165 | 200(20.2)/40(7.0) | <0.01 |
| SART3-511 | 1,967/621 | 41 | 439(145) | 921 | 145(22.6)/213(10.8) | <0.01 |
| SART3-309 | 1,921/667 | 35 | 328(158) | 3,070 | 158(21.4)/124(10.5) | <0.01 |
| WHSC2-141 | 1,908/680 | 39 | 325(173) | 13,078 | 173(19.0)/94(7.7) | <0.01 |
| CypB-129 | 1,888/700 | 31 | 483(222) | 2,483 | 222(23.8)/192(9.0) | <0.01 |
| Lck-246 | 1,815/773 | 49 | 366(169) | 10,806 | 169(19.3)/128(8.3) | <0.01 |
| WHSC2-103 | 1,673/915 | 43 | 729(222) | 827 | 222(19.6)/374(12.1) | <0.01 |
| SART3-302 | 1,640/948 | 84 | 408(271) | 14,958 | 271(18.2)/78(9.1) | <0.01 |
| MRP3-1293 | 1,558/1,030 | 27 | 324(157) | 6,003 | 157(20.6)/113(8.6) | <0.01 |
| EGFR-800 | 1,542/1,046 | 30 | 390(156) | 715 | 156(24.3)/188(12.4) | <0.01 |
| PAP-213 | 1,422/1,166 | 33 | 279(196) | 14,912 | 196(21.7)/52(7.0) | <0.01 |
| Lck-449 | 1,406/1,182 | 23 | 127(67) | 18,919 | 67(19.5)/44(6.2) | <0.01 |
| MAP-432 | 1,308/1,280 | 51 | 375(97) | 757 | 97(23.8)/203(12.0) | <0.01 |
| HNRPL-140 | 1,295/1,293 | 39 | 200(111) | 8,576 | 111(23.9)/62(9.8) | <0.01 |
| PAP-248 | 1,286/1,302 | 37 | 147(62) | 2,136 | 62(21.1)/63(9.9) | 0.01 |
| UBE2V-43 | 1,238/1,350 | 34 | 230(159) | 25,121 | 159(18.5)/40(7.0) | <0.01 |
| SART3-109 | 1,206/1,382 | 31 | 474(223) | 7,875 | 223(19.0)/191(10.3) | <0.01 |
| HNRPL-501 | 1,189/1,399 | 45 | 355(223) | 10,405 | 223(19.3)/74(6.6) | <0.01 |
| SART2-161 | 947/1,641 | 29 | 195(61) | 312 | 61(20.6)/109(12.6) | 0.05 |
| PSMA-624 | 950/1,638 | 24 | 101(50) | 7,772 | 50(21.7)/37(5.5) | <0.01 |
| PTHrP-102 | 886/1,702 | 27 | 204(99) | 1,423 | 98(20.0)/81(11.4) | 0.01 |
| Lck-208 | 560/2,028 | 30 | 134(64) | 4,597 | 64(30.5)/62(16.0) | 0.01 |
| EZH2-735 | 558/2,030 | 23 | 69(51) | 17,691 | 51(24.7)/11(5.3) | <0.01 |
| MRP3-503 | 467/2,121 | 37 | 133(85) | 6,401 | 85(20.6)/36(9.3) | 0.08 |
| UBE2V-85 | 362/2,226 | 28 | 51(26) | 3,941 | 26(16.8)/18(8.4) | 0.09 |
| Lck-422 | 316/2,272 | 33 | 92(20) | 436 | 20(18.5)/60(11.7) | 0.10 |

Correlation levels of the 31 peptides for OS were evaluated by the Cox proportional hazards regression model. In this study, when the patients under PPV containing a peptide have significantly longer survival than the patients under PPV not containing it (HR: < 1.0, 95 % CI: < 1.0, and p < 0.05), the peptide was defined correlating for OS, as reported previously (Non-Patent Literature 9). The results are shown in Table 18. In all 2,588 cases, the patients under PPV containing the following peptide showed the significantly longer survival: HNRPL-140, EGF-R-800 or Lck-486 peptide, with the median OS of vaccinated cases vs non-vaccinated cases 12.9 vs 11.1 M, 14.3 vs 11.1, or 12.3 vs 11.3.

**[Table 18]**

| | **Correlation between vaccination and OS** | | | | | |
|---|---|---|---|---|---|---|
| Peptides | Vaccinate cases (mOS) | Non-vaccinate cases (mOS) | p-value | HR | HR (95%Cl) | HR (P-value) |
| SART2-93 | 1,137(12.3) | 413(11.5) | 0.3067 | 0.9 | 0.8-1.1 | 0.31 |
| Lck-486 | 879(12.3) | 671(11.3) | 0.0077 | 0.9 | 0.8-1.0 | 0.01 |
| Lck-488 | 1,103(12.0) | 447(12.0) | 0.1662 | 0.9 | 0.8-1.0 | 0.17 |
| Lck-90 | 502(11.3) | 712(11.0) | 0.9184 | 1.0 | 0.9-1.1 | 0.92 |
| SART3-734 | 625(10.9) | 589(11.3) | 0.5228 | 1.0 | 0.9-1.2 | 0.52 |
| PSA-248 | 283(13.8) | 1,267(11.6) | 0.2389 | 0.9 | 0.8-1.1 | 0.24 |
| SART3-511 | 439(11.0) | 775(11.2) | 0.925 | 1.0 | 0.9-1.1 | 0.93 |
| SART3-309 | 328(12.1) | 722(11.1) | 0.2481 | 0.9 | 0.8-1.1 | 0.25 |
| WHSC2-141 | 325(12.1) | 725(11.1) | 0.8526 | 1.0 | 0.9-1.2 | 0.85 |
| CypB-129 | 483(12.3) | 1458(11.2) | 0.2438 | 0.9 | 0.8-1.1 | 0.24 |
| Lck-246 | 366(11.4) | 684(11.6) | 0.6799 | 1.0 | 0.9-1.2 | 0.68 |
| WHSC2-103 | 729(11.5) | 1,465(11.6) | 0.5199 | 1.0 | 0.9-1.1 | 0.52 |
| SART3-302 | 408(12.7) | 642(10.5) | 0.1038 | 0.9 | 0.8-1.0 | 0.10 |
| MRP3-1293 | 324(11.9) | 1,226(12.1) | 0.3110 | 1.1 | 0.9-1.2 | 0.31 |
| EGFR-800 | 390(14.3) | 1,160(11.1) | 0.0088 | 0.8 | 0.7-1.0 | 0.01 |
| PAP-213 | 279(16.6) | 1271(11.4) | 0.0583 | 0.9 | 0.8-1.0 | 0.06 |
| Lck-449 | 127(13.7) | 1,087(10.8) | 0.5537 | 0.9 | 0.8-1.1 | 0.55 |
| MAP-432 | 375(11.1) | 1,087(11.9) | 0.5089 | 1.0 | 0.9-1.2 | 0.51 |
| HNRPL-140 | 200(12.9) | 850(11.1) | 0.0069 | 0.8 | 0.7-0.9 | 0.01 |
| PAP-248 | 147(11.4) | 1,067(11.1) | 0.7630 | 1.0 | 0.8-1.2 | 0.76 |
| UBE2V-43 | 230(11.7) | 820(11.5) | 0.1573 | 0.9 | 0.8-1.0 | 0.15 |
| SART3-109 | 474(12.3) | 1,948(11.5) | 0.8751 | 1.0 | 0.9-1.1 | 0.88 |
| HNRPL-501 | 355(12.1) | 1,107(11.6) | 0.7499 | 1.0 | 0.9-1.1 | 0.75 |
| SART2-161 | 195(12.3) | 13,552(11.9) | 0.6856 | 1.0 | 0.8-1.1 | 0.68 |
| PSMA-624 | 101(11.7) | 1,449(12.0) | 0.7342 | 1.0 | 0.8-1.3 | 0.74 |
| PTHrP-102 | 204(13.3) | 1,346(11.9) | 0.2205 | 0.9 | 0.8-1.1 | 0.22 |
| Lck-208 | 134(19.7) | 1,416(11.4) | 0.0006 | 0.9 | 0.8-1.0 | 0.06 |
| EZH2-735 | 69(13.3) | 1,481(11.9) | 0.2494 | 0.9 | 0.7-1.1 | 0.24 |
| MRP3-503 | 133(14.1) | 1,417(11.9) | 0.5509 | 0.9 | 0.8-1.1 | 0.55 |
| UBE2V-85 | 51(9.6) | 999(11.6) | 0.3126 | 1.2 | 0.9-1.5 | 0.32 |
| Lck-422 | 92(12.0) | 1,849(11.4) | 0.2085 | 1.2 | 0.9-1.4 | 0.22 |

Similarly, in the 2,085 advanced cancer patients (stage IV + recurrence) and the 503 non-advanced cancer patients (I-III stages) correlation levels of the 31 peptides for OS were evaluated by the Cox proportional hazards regression model. In the advanced cancer patients, the median OS of patients who had the vaccination containing Lck-486, FGF-R-800, PAP-213, HNRPL-140, or Lck-208 peptide were significantly longer than the other patients who had the vaccination without them, with the median OS of vaccinated cases vs non-vaccinated cases 11.1 vs 9.6 M, 13.1 vs 9.6, 14.9 vs 10.0, 12.1 vs 10.0, or 17.1 vs 10.0 (Table 19). In contrast, no peptide showed significant correlation to OS in the non-advanced cancer patients, probably because the population was too small to evaluate the correlation levels (data not shown).

**[Table 19]**

| | **Correlation between vaccination and OS** | | | | | |
|---|---|---|---|---|---|---|
| Peptides | Vaccinate cases (mOS) | Non-vaccinate cases (mOS) | p-value | HR | HR (95%Cl) | HR (P-value) |
| SART2-93 | 906(10.7) | 340(10.2) | 0.4855 | 1.0 | 0.8-1.1 | 0.49 |
| Lck-486 | 709(11.1) | 537(9.6) | 0.0106 | 0.9 | 0.8-1.0 | 0.01 |
| Lck-488 | 872(10.7) | 374(10.1) | 0.6332 | 1.0 | 0.9-1.1 | 0.63 |
| Lck-90 | 391(10.3) | 578(9.4) | 0.8896 | 1.0 | 0.9-1.1 | 0.89 |
| SART3-734 | 502(9.5) | 467(10.2) | 0.5448 | 1.0 | 0.9-1.2 | 0.55 |
| PSA-248 | 230(13.0) | 1016(10.2) | 0.2455 | 0.9 | 0.8-1.1 | 0.24 |
| SART3-511 | 346(9.9) | 623(10.0) | 0.7381 | 1.0 | 0.9-1.2 | 0.74 |
| SART3-309 | 259(11.6) | 603(9.8) | 0.1929 | 0.9 | 0.8-1.1 | 0.19 |
| WHSC2-141 | 271(11.1) | 591(10.1) | 0.6452 | 1.0 | 0.9-1.2 | 0.65 |
| CypB-129 | 393(10.9) | 1183(10.1) | 0.3059 | 0.9 | 0.8-1.1 | 0.30 |
| Lck-246 | 300(9.8) | 562(10.4) | 0.8176 | 1.0 | 0.9-1.2 | 0.82 |
| WHSC2-103 | 581(10.2) | 1193(10.3) | 0.7439 | 1.0 | 0.9-1.1 | 0.74 |
| SART3-302 | 328(11.4) | 534(9.8) | 0.2706 | 0.9 | 0.8-1.1 | 0.27 |
| MRP3-1293 | 253(10.5) | 993(10.4) | 0.5309 | 1.0 | 0.9-1.2 | 0.53 |
| EGFR-800 | 299(13.1) | 947(9.6) | 0.0030 | 0.8 | 0.7-0.9 | 0.01 |
| PAP-213 | 228(14.9) | 1018(10.0) | 0.0071 | 0.8 | 0.7-0.9 | 0.01 |
| Lck-449 | 102(13.3) | 867(9.5) | 0.0922 | 0.8 | 0.7-1.0 | 0.09 |
| MAP-432 | 296(9.7) | 892(10.8) | 0.2602 | 1.1 | 0.9-1.2 | 0.26 |
| HNRPL-140 | 162(12.1) | 700(10.0) | 0.0246 | 0.8 | 0.7-1.0 | 0.02 |
| PAP-248 | 124(10.1) | 845(9.9) | 0.9303 | 0.8 | 0.6-1.0 | 0.10 |
| UBE2V-43 | 176(10.2) | 686(10.2) | 0.1709 | 0.9 | 0.7-1.1 | 0.17 |
| SART3-109 | 376(10.6) | 1566(10.2) | 0.6983 | 1.0 | 0.9-1.2 | 0.70 |
| HNRPL-501 | 291(10.4) | 897(10.3) | 0.7739 | 1.0 | 0.9-1.1 | 0.77 |
| SART2-161 | 156(10.7) | 1090(10.4) | 0.5224 | 0.9 | 0.8-1.1 | 0.52 |
| PSMA-624 | 78(7.9) | 1168(10.7) | 0.3944 | 1.1 | 0.9-1.4 | 0.40 |
| PTHrP-102 | 168(11.8) | 1078(10.3) | 0.7580 | 0.9 | 0.7-1.0 | 0.07 |
| Lck-208 | 103(17.1) | 1143(10.0) | 0.0016 | 0.7 | 0.6-0.9 | 0.01 |
| EZH2-735 | 53(11.9) | 1193(10.4) | 0.3887 | 0.9 | 0.7-1.2 | 0.38 |
| MRP3-503 | 108(13.4) | 1138(10.4) | 0.4385 | 0.9 | 0.7-1.1 | 0.43 |
| UBE2V-85 | 42(9.6) | 820(10.3) | 0.4454 | 1.1 | 0.8-1.5 | 0.45 |
| Lck-422 | 83(11.7) | 1493(10.2) | 0.5684 | 1.1 | 0.8-1.3 | 0.57 |

### Clinical activity of warehouse peptide set comprising a smaller number of peptides

It was further tested whether a warehouse peptide set comprising a smaller number of peptides have the possibility to induce the non-inferior clinical activities as compared to the warehouse peptide set comprising 31 peptides in view of immune boosting and clinical benefits. Candidate peptides for the new set were selected based on HLA types, cross-reactivity, biomarkers, clinical efficacy, antigenic property, and numbers of vaccinated patients. HLA-A24, -A2, or -A3sup allele is found at least >20% of populations worldwide, whereas HLA-A26 allele is found in 11% of the Japanese population, 4% of the Caucasian population in the US, and 7.5% of the African population of Cape Town (Niu Y. et al., Cancer Sci 2009; 100: 2167-74). Accordingly, the new set was designed to include four HLA-A24-macthed peptides, four HLA-A2-macthed peptides, four HLA-A3sup-macthed peptides, and two HLA-A26-macthed peptides. SART3-109 and WHSC2-103, which are capable of inducing CTL activity to three different alleles (Table 20), were also included. The cutoff levels of neutrophil percentage (64%) and lymphocyte percentage (26%) were used as biomarkers, and then the number of patients showing both <64% neutrophil and ≥26% lymphocyte together was 1,066 as reported above. Regarding the clinical efficacy, peptides with lower p-value of HR for median OS between the patients having received them and the other patients were included. When two or more peptides became candidates, preferred antigens were those encoding peptides other than common antigens expressed on all the proliferating tumor and normal cells. Peptides vaccinated for less than 60 patients of 1,066 patients (5.6%) were excluded from the set to avoid any biases.

Details of peptide selection are shown in Tables 20 and 21, in which the selected peptides are shown in bold. WHSC2-103, CypB-129, UBE-43, and HNRPL-140 peptides were selected as the four HLA-A2-matched peptides. SART3-109, Lck-208, PAP-213, and Lck-486 were selected as the four HLA-A24-matched peptides. WHSC2-103, CypB-129, and SART3-109 were selected as three of the four HLA-A3sup peptides. PAP-248 was excluded because the median OS of patients with it was shorter than that of patients without it. SART3-511 and SART3-734 were also excluded because SART3-109, derived from the common antigen, had been already selected. Lck-449 was also excluded because the number of patients with it was small. Lck-90 was then selected as the remaining one HLA-A3sup peptide. WHSC2-103 and SART3-109 were selected as the two HLA-A26 peptides. Either the pre- and post-vaccination IgG levels or clinical correlation of this new set of the nine peptides (9P: WHSC2-103, CypB-129, UBE-43, HNRPL-140, SART3-109, Lck-208, PAP-213, Lck-486, and Lck-90) was not inferior to that of the set of the remaining 22 peptides (22P) (the last two rows in Tables 20 and 21).

**[Table 20]**

| | **Pre-vaccination IgG** | | **Post vaccination IgG** | | **Correlation between immune responses and OS** | |
|---|---|---|---|---|---|---|
| Peptides | Positive/ Negative | Median (FIU) | Number of vaccinated (positive) patients | Median (FIU) | Positive (mOS) / Negative (mOS) | p-value |
| SART2-93 | 925/141 | 64 | 467(183) | 8,633 | 183(24.7)/241(17.3) | 0.01 |
| **Lck-486** | **899/167** | **40** | **356(274)** | **17,613** | **274(25.2)/56(11.4)** | **<0.01** |
| Lck-488 | 874/192 | 60 | 452(313) | 11,894 | 313(24.7)/97(9.3) | <0.01 |
| **Lck-90** | **851/215** | **43** | **210(100)** | **4,250** | **100(26.3)/91(12.0)** | **0.01** |
| SART3-734 | 835/231 | 96 | 253(116) | 7,098 | 116(29.3)/113(14.8) | <0.01 |
| PSA-248 | 835/231 | 47 | 107(81) | 18,191 | 81(27.4)/18(9.6) | <0.01 |
| SART3-511 | 802/264 | 38 | 179(82) | 1,095 | 82(29.2)/80(17.1) | 0.01 |
| SART3-309 | 813/253 | 33 | 154(99) | 6,009 | 99(28.9)/46(10.4) | <0.01 |
| WHSC2-141 | 805/261 | 38 | 134(91) | 18,171 | 91(28.2)/30(12.0) | <0.01 |
| **CypB-129** | **776/290** | **28** | **196(110)** | **3,143** | **110(29.4)/73(13.2)** | **<0.01** |
| Lck-246 | 767/299 | 48 | 161(92) | 15,377 | 92(28.2)/53(8.7) | <0.01 |
| **WHSC2-103** | **685/381** | **40** | **289(105)** | **916** | **105(27.1)/159(14.4)** | **0.01** |
| SART3-302 | 691/375 | 84 | 173(138) | 17,719 | 138(21.2)/23(10.8) | 0.01 |
| MRP3-1293 | 643/423 | 27 | 132(83) | 6,491 | 83(24.3)/39(12.6) | 0.01 |
| EGFR-800 | 635/431 | 32 | 176(79) | 746 | 79(27.5)/85(17.1) | 0.01 |
| **PAP-213** | **577/489** | **33** | **121(94)** | **15,379** | **94(28.1)/17(11.3)** | **0.01** |
| Lck-449 | 591/475 | 23 | 57(38) | 18,734 | 38(27.1)/17(10.0) | 0.01 |
| MAP-432 | 517/549 | 52 | 133(45) | 947 | 45(29.4)/73(15.5) | 0.01 |
| HNRPL-140 | 561/505 | 37 | 103(68) | 7,150 | 68(26.2)/30(10.4) | 0.01 |
| PAP-248 | 523/543 | 38 | 64(31) | 1,981 | 31(21.3)/25(13.0) | 0.14 |
| UBE2V-43 | 478/588 | 33 | 104(82) | 25,979 | 82(30.1)/15(7.7) | <0.01 |
| SART3-109 | 494/572 | 30 | 192(116) | 9,770 | 116(24.1)/63(17.1) | 0.04 |
| HNRPL-501 | 479/587 | 48 | 148(111) | 12,553 | 111(28.2)/22(10.6) | <0.01 |
| SART2-161 | 367/699 | 27 | 70(26) | 775 | 26(26.7)/41(14.4) | 0.06 |
| PSMA-624 | 386/680 | 26 | 37(26) | 10,984 | 26(24.4)/9(21.2) | 0.17 |
| PTHrP-102 | 356/710 | 27 | 91(51) | 5,336 | 51(39.0)/35(13.9) | 0.01 |
| **Lck-208** | **227/839** | **31** | **69(36)** | **9,551** | **36(34.0)/32(19.9)** | **0.03** |
| EZH2-735 | 229/837 | 23 | 31(25) | 20,673 | 25(26.7)/4(6.7) | 0.01 |
| MRP3-503 | 181/885 | 40 | 61(46) | 13,871 | 46(25.1)/13(12.6) | 0.34 |
| UBE2V-85 | 153/913 | 30 | 28(16) | 4,342 | 16(16.8)/9(7.0) | 0.19 |
| Lck-422 | 121/945 | 33 | 33(10) | 446 | 10(18.8)/22(15.6) | 0.47 |
| 22P | 1066/0 | 42 | 1058(913) | 64 | 913(20.7)/47(9.9) | 0.01 |
| **9P** | **933/6** | **37** | **939(820)** | **70** | **820(20.8)/39(9.9)** | **0.01** |

**[Table 21]**

| | **Correlation between vaccination and OS** | | | | | |
|---|---|---|---|---|---|---|
| Peptides | Vaccinate cases (mOS) | Non-vaccinate cases (mOS) | p-value | HR | HR (95%Cl) | HR (P-value) |
| SART2-93 | 467(19.0) | 158(17.6) | 0.7996 | 1.0 | 0.8-1.2 | 0.80 |
| **Lck-486** | **356(19.1)** | **269(17.6)** | **0.0962** | **0.9** | **0.7-1.0** | **0.10** |
| Lck-488 | 452(17.9) | 173(19.7) | 0.9460 | 1.0 | 0.8-1.2 | 0.95 |
| **Lck-90** | **210(17.8)** | **297(17.5)** | **0.9050** | **1.0** | **0.8-1.2** | **0.91** |
| SART3-734 | 253(17.9) | 254(17.5) | 0.5846 | 0.9 | 0.8-1.2 | 0.58 |
| PSA-248 | 107(20.4) | 518(17.9) | 0.2682 | 0.9 | 0.7-1.1 | 0.26 |
| SART3-511 | 179(19.0) | 328(17.5) | 0.1858 | 0.9 | 0.7-1.1 | 0.18 |
| SART3-309 | 154(19.0) | 280(15.6) | 0.4780 | 0.9 | 0.7-1.2 | 0.48 |
| WHSC2-141 | 134(18.0) | 300(16.6) | 0.9187 | 1.0 | 0.8-1.2 | 0.92 |
| **CypB-129** | **196(19.3)** | **612(16.9)** | **0.1768** | **0.9** | **0.7-1.1** | **0.17** |
| Lck-246 | 161(15.4) | 273(18.2) | 0.6127 | 1.1 | 0.9-1.3 | 0.61 |
| **WHSC2-103** | **289(17.6)** | **617(18.0)** | **0.5916** | **1.0** | **0.9-1.3** | **0.59** |
| SART3-302 | 173(18.1) | 261(16.3) | 0.5679 | 0.9 | 0.8-1.2 | 0.57 |
| MRP3-1293 | 132(17.5) | 493(18.4) | 0.8423 | 1.0 | 0.8-1.3 | 0.84 |
| EGFR-800 | 176(21.2) | 449(21.2) | 0.5802 | 0.9 | 0.8-1.2 | 0.58 |
| **PAP-213** | **121(23.7)** | **504(17.9)** | **0.1620** | **0.9** | **0.8-1.1** | **0.27** |
| Lck-449 | 57(18.1) | 450(17.5) | 0.1127 | 0.8 | 0.6-1.1 | 0.10 |
| MAP-432 | 133(16.1) | 460(18.5) | 0.2834 | 1.1 | 0.9-1.4 | 0.29 |
| **HNRPL-140** | **103(19.0)** | **331(15.6)** | **0.1847** | **0.8** | **0.7-1.1** | **0.18** |
| PAP-248 | 64(15.5) | 443(17.6) | 0.6097 | 1.1 | 0.8-1.4 | 0.61 |
| **UBE2V-43** | **104(19.0)** | **330(16.4)** | **0.2080** | **0.8** | **0.6-1.1** | **0.20** |
| **SART3-109** | **192(19.8)** | **796(18.0)** | **0.7663** | **1.0** | **0.9-1.2** | **0.77** |
| HNRPL-501 | 148(19.6) | 445(17.5) | 0.5923 | 0.9 | 0.8-1.2 | 0.59 |
| SART2-161 | 70(17.4) | 555(18.4) | 0.5293 | 1.1 | 0.8-1.4 | 0.53 |
| PSMA-624 | 37(21.2) | 588(18.0) | 0.6573 | 0.9 | 0.6-1.3 | 0.65 |
| PTHrP-102 | 91(21.2) | 534(18.0) | 0.4011 | 0.9 | 0.7-1.2 | 0.40 |
| **Lck-208** | **69(26.7)** | **556(17.6)** | **0.0082** | **0.7** | **0.5-0.9** | **0.01** |
| EZH2-735 | 31(26.5) | 594(18.0) | 0.1314 | 0.7 | 0.5-1.1 | 0.11 |
| MRP3-503 | 61(24.3) | 564(18.1) | 0.9002 | 1.0 | 0.8-1.3 | 0.90 |
| UBE2V-85 | 28(9.6) | 406(18.0) | 0.0776 | 1.4 | 0.9-2.1 | 0.10 |
| Lck-422 | 33(15.6) | 775(17.5) | 0.7250 | 1.1 | 0.7-1.6 | 0.73 |
| 22P | 1058(18.1) | 8(13.5) | 0.0263 | 0.5 | 0.3-1.0 | 0.05 |
| **9P** | **939(18.5)** | **127(15.5)** | **0.0551** | **0.8** | **0.7-1.0** | **0.06** |

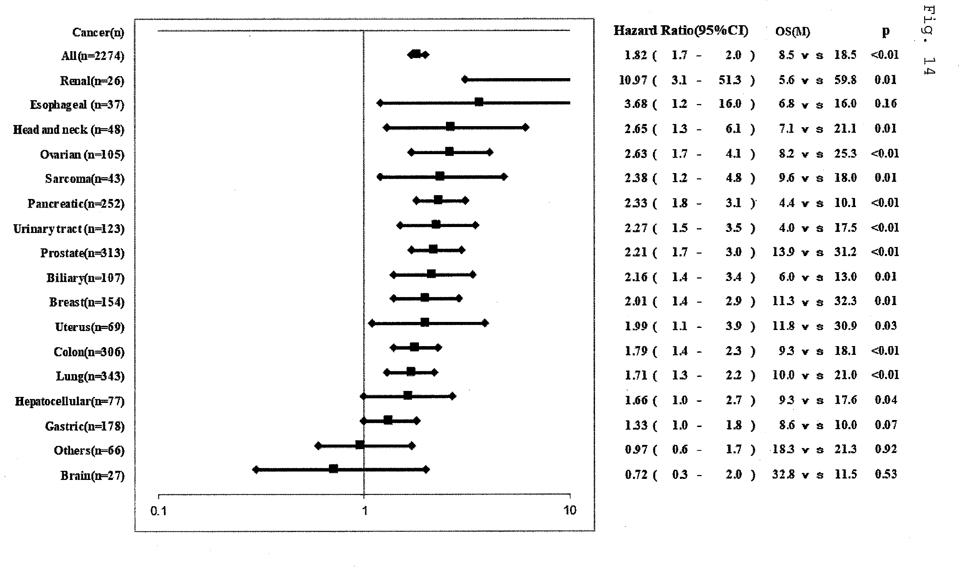

At least one of the nine peptides were vaccinated to 2,274 of the 2,588 patients (87.9%). The median OS for 1,335 of the 2,274 patients (58.7 %) who met either or both the cutoffs of neutrophil ≥64% and lymphocyte <26% was significantly shorter than that of the remaining 939 patients who showed both <64% neutrophil and ≥26% lymphocyte (8.5 M, 95% CI: 7.8-9.3 vs 18.5, 17.2-20.1, HR: 1.8, 95%CI: 1.7-2.0, p<0.01). A part of the results is shown in Fig. 14. The similar results were obtained for the patients with all tested cancers except for gastric cancer, brain tumor, and the other miscellaneous cancers. In view of clinical efficacy, the median OS of the 939 patients, who showed both <64% neutrophil and ≥26% lymphocyte (18.5 M, 95% CI: 17.2 to 20.1) (Fig. 14), was not inferior to that of 1,066 patients who showed the same property in the entire patients (18.0M, 95%CI: 16.8-19.6) (Fig. 13). The similar results were obtained for the patients with all tested cancers except for gastric cancer, brain tumor, and the other miscellaneous cancers.

Accordingly, the set of the nine peptides is not inferior to the set of the 31 warehouse peptides in view of immune response and clinical usefulness, when it is used as warehouse peptides of PPV for cancer patients who met both the neutrophil percentage <64% and the lymphocyte percentage ≥26% except for gastric cancer and brain tumor patients. The smaller number of warehouse peptides are preferred in view of cost and handleability.

### Industrial Applicability

According to the disclosure, eligibility of a cancer patient for a peptide vaccine therapy can be determined before starting the treatment. When the patient is determined as "not eligible", the patient can be subjected to another treatment, and thus, costs of preparation or administration of a peptide vaccine composition can be beneficially saved in view of the medical economy. On the other hand, when the patient is determined as "eligible", the patient can be subjected to the peptide vaccine therapy. The disclosure can also reduce the number of warehouse peptide antigens in a tailor-made peptide vaccine therapy as compared with conventional techniques.

## Claims

1. A method comprising determining whether a cancer patient is eligible for a peptide vaccine therapy on the basis of a level of neutrophil percentage and/or a level of lymphocyte percentage in a blood sample obtained from the patient about 7 to 35 days before a scheduled administration date of a peptide vaccine composition.

2. The method according to claim 1, comprising determining the level of neutrophil percentage and/or the level of lymphocyte percentage in the blood sample obtained from the patient.

3. The method according to claim 1 or 2, comprising determining whether the patient is eligible for the peptide vaccine therapy by comparing the level of neutrophil percentage and/or the level of lymphocyte percentage determined for the patient with a cutoff level of neutrophil percentage and/or a cutoff level of lymphocyte percentage.

4. The method according to claim 3, comprising determining the patient is eligible for the peptide vaccine therapy when the level of neutrophil percentage determined for the patient is less than the cutoff level of neutrophil percentage.

5. The method according to claim 3, comprising determining the patient is eligible for the peptide vaccine therapy when the level of lymphocyte percentage determined for the patient is not less than the cutoff level of lymphocyte percentage.

6. The method according to claim 3, comprising determining the patient is eligible for the peptide vaccine therapy when the level of neutrophil percentage determined for the patient is less than the cutoff level of neutrophil percentage and the level of lymphocyte percentage determined for the patient is not less than the cutoff level of lymphocyte percentage.

7. The method according to any one of claims 1 to 6, wherein the peptide vaccine therapy is a tailor-made peptide vaccine therapy.

8. The method according to claim 7, wherein the tailor-made peptide vaccine therapy uses a peptide vaccine composition comprising at least one peptide antigen selected from the group consisting of CypB-129, Lck-246, Lck-422, MAP-432, WHSC2-103, HNRPL-501, UBE-43, UBE-85, WHSC2-141, HNRPL-140, SART3-302, SART3-309, SART2-93, SART3-109, Lck-208, PAP-213, PSA-248, EGFR-800, MRP3-503, MRP3-1293, SART2-161, Lck-486, Lck-488, PSMA-624, EZH2-735, PTHrP-102, SART3-511, SART3-734, Lck-90, Lck-449, and PAP-248, wherein the at least one peptide antigen is selected on the basis of immunoreactivity of the patient for each peptide antigen.

9. The method according to claim 7 or 8, wherein the tailor-made peptide vaccine therapy uses a peptide vaccine composition comprising at least one peptide antigen selected from the group consisting of WHSC2-103, CypB-129, UBE-43, HNRPL-140, SART3-109, Lck-208, PAP-213, Lck-486, and Lck-90, wherein the at least one peptide antigen is selected on the basis of immunoreactivity of the patient for each peptide antigen.

10. The method according to any one of claims 1 to 6, wherein the peptide vaccine therapy is a ready-made peptide vaccine therapy.

11. The method according to claim 10, wherein the ready-made peptide vaccine therapy uses a peptide vaccine composition comprising at least one peptide antigen selected from the group consisting of CypB-129, Lck-246, Lck-422, MAP-432, WHSC2-103, HNRPL-501, UBE-43, UBE-85, WHSC2-141, HNRPL-140, SART3-302, SART3-309, SART2-93, SART3-109, Lck-208, PAP-213, PSA-248, EGFR-800, MRP3-503, MRP3-1293, SART2-161, Lck-486, Lck-488, PSMA-624, EZH2-735, PTHrP-102, SART3-511, SART3-734, Lck-90, Lck-449, and PAP-248.

12. The method according to any one of claims 1 to 11, wherein the patient is positive for HLA-A24, HLA-A2, HLA-A3, HLA-A11, HLA-A31, HLA-A33, or HLA-A26.

13. The method according to any one of claims 1 to 12, wherein the cancer is lung cancer, prostate cancer, colon cancer, pancreatic cancer, breast cancer, urinary tract cancer, biliary tract cancer, ovarian cancer, uterine cancer, hepatocellular cancer, head and neck cancer, sarcoma, esophageal cancer, or renal cancer.

14. A kit for determining whether a cancer patient is eligible for a peptide vaccine therapy, comprising a reagent for determining a level of neutrophil percentage and/or a level of lymphocyte percentage.

15. A peptide vaccine composition for treating a cancer in a patient, comprising at least one peptide antigen, wherein the patient has been determined eligible for a peptide vaccine therapy by the method according to any one of claims 1 to 13.

16. A kit for preparing a peptide vaccine composition for treating a cancer in a patient, comprising at least one peptide antigen, wherein the patient has been determined eligible for a peptide vaccine therapy by the method according to any one of claims 1 to 13.

17. A kit comprising WHSC2-103, CypB-129, UBE-43, HNRPL-140, SART3-109, Lck-208, PAP-213, Lck-486, and Lck-90.

18. A kit comprising one or plural polynucleotides encoding WHSC2-103, CypB-129, UBE-43, HNRPL-140, SART3-109, Lck-208, PAP-213, Lck-486, and Lck-90.


SEQUENCE LISTING

<110>  ITO, Kyogo

<120>  METHOD OF PREDICTING RESPONSIVENESS OF CANCER PATIENT TO PEPTIDE
       VACCINE THERAPY

<130>  675488

<150>  JP 2019-090530
<151>  2019-05-13

<150>  JP 2019-131830
<151>  2019-07-17

<160>  31

<170>  PatentIn version 3.5

<210>  1
<211>  10
<212>  PRT
<213>  Homo sapiens

<400>  1

Lys Leu Lys His Tyr Gly Pro Gly Trp Val
1               5                   10


<210>  2
<211>  9
<212>  PRT
<213>  Homo sapiens

<400>  2

Lys Leu Val Glu Arg Leu Gly Ala Ala
1               5


<210>  3
<211>  9
<212>  PRT
<213>  Homo sapiens

<400>  3

Asp Val Trp Ser Phe Gly Ile Leu Leu
1               5


<210>  4
<211>  9
<212>  PRT
<213>  Homo sapiens

<400>  4

Asp Leu Leu Ser His Ala Phe Phe Ala
1               5

<210>  5
<211>  9
<212>  PRT
<213>  Homo sapiens

<400>  5

Ala Ser Leu Asp Ser Asp Pro Trp Val
1               5


<210>  6
<211>  10
<212>  PRT
<213>  Homo sapiens

<400>  6

Asn Val Leu His Phe Phe Asn Ala Pro Leu
1               5                   10


<210>  7
<211>  9
<212>  PRT
<213>  Homo sapiens

<400>  7

Arg Leu Gln Glu Trp Cys Ser Val Ile
1               5


<210>  8
<211>  9
<212>  PRT
<213>  Homo sapiens

<400>  8

Leu Ile Ala Asp Phe Leu Ser Gly Leu
1               5


<210>  9
<211>  9
<212>  PRT
<213>  Homo sapiens

<400>  9

Ile Leu Gly Glu Leu Arg Glu Lys Val
1               5


<210>  10
<211>  9
<212>  PRT
<213>  Homo sapiens

<400>  10

Ala Leu Val Glu Phe Glu Asp Val Leu

1                   5


<210>  11
<211>  9
<212>  PRT
<213>  Homo sapiens

<400>  11

Leu Leu Gln Ala Glu Ala Pro Arg Leu
1                   5


<210>  12
<211>  9
<212>  PRT
<213>  Homo sapiens

<400>  12

Arg Leu Ala Glu Tyr Gln Ala Tyr Ile
1                   5


<210>  13
<211>  9
<212>  PRT
<213>  Homo sapiens

<400>  13

Asp Tyr Ser Ala Arg Trp Asn Glu Ile
1                   5


<210>  14
<211>  10
<212>  PRT
<213>  Homo sapiens

<400>  14

Val Tyr Asp Tyr Asn Cys His Val Asp Leu
1                   5                   10


<210>  15
<211>  9
<212>  PRT
<213>  Homo sapiens

<400>  15

His Tyr Thr Asn Ala Ser Asp Gly Leu
1                   5


<210>  16
<211>  9
<212>  PRT
<213>  Homo sapiens

<400>  16

Leu Tyr Cys Glu Ser Val His Asn Phe
1               5


<210>  17
<211>  10
<212>  PRT
<213>  Homo sapiens

<400>  17

His Tyr Arg Lys Trp Ile Lys Asp Thr Ile
1               5                   10


<210>  18
<211>  10
<212>  PRT
<213>  Homo sapiens

<400>  18

Asp Tyr Val Arg Glu His Lys Asp Asn Ile
1               5                   10


<210>  19
<211>  9
<212>  PRT
<213>  Homo sapiens

<400>  19

Leu Tyr Ala Trp Glu Pro Ser Phe Leu
1               5


<210>  20
<211>  10
<212>  PRT
<213>  Homo sapiens

<400>  20

Asn Tyr Ser Val Arg Tyr Arg Pro Gly Leu
1               5                   10


<210>  21
<211>  9
<212>  PRT
<213>  Homo sapiens

<400>  21

Ala Tyr Asp Phe Leu Tyr Asn Tyr Leu
1               5


<210>  22
<211>  9

<212>  PRT
<213>  Homo sapiens

<400>  22

Thr Phe Asp Tyr Leu Arg Ser Val Leu
1               5


<210>  23
<211>  10
<212>  PRT
<213>  Homo sapiens

<400>  23

Asp Tyr Leu Arg Ser Val Leu Glu Asp Phe
1               5                   10


<210>  24
<211>  9
<212>  PRT
<213>  Homo sapiens

<400>  24

Thr Tyr Ser Val Ser Phe Asp Ser Leu
1               5


<210>  25
<211>  9
<212>  PRT
<213>  Homo sapiens

<400>  25

Lys Tyr Val Gly Ile Glu Arg Glu Met
1               5


<210>  26
<211>  10
<212>  PRT
<213>  Homo sapiens

<400>  26

Arg Tyr Leu Thr Gln Glu Thr Asn Lys Val
1               5                   10


<210>  27
<211>  9
<212>  PRT
<213>  Homo sapiens

<400>  27

Trp Leu Glu Tyr Tyr Asn Leu Glu Arg
1               5

<210>  28
<211>  9
<212>  PRT
<213>  Homo sapiens

<400>  28

Gln Ile Arg Pro Ile Phe Ser Asn Arg
1               5

<210>  29
<211>  10
<212>  PRT
<213>  Homo sapiens

<400>  29

Ile Leu Glu Gln Ser Gly Glu Trp Trp Lys
1               5                   10

<210>  30
<211>  10
<212>  PRT
<213>  Homo sapiens

<400>  30

Val Ile Gln Asn Leu Glu Arg Gly Tyr Arg
1               5                   10

<210>  31
<211>  10
<212>  PRT
<213>  Homo sapiens

<400>  31

Gly Ile His Lys Gln Lys Glu Lys Ser Arg
1               5                   10